(19) Europäisches Patentamt
European Patent Office
Office européen des brevets

(11) EP 4 039 818 A1

(12) EUROPEAN PATENT APPLICATION

(43) Date of publication:
**10.08.2022 Bulletin 2022/32**

(21) Application number: **21155584.2**

(22) Date of filing: **05.02.2021**

(51) International Patent Classification (IPC):
***C12Q 1/37*** *(2006.01)* ***G01N 33/566*** *(2006.01)*
***G01N 33/68*** *(2006.01)*

(52) Cooperative Patent Classification (CPC):
**C12Q 1/37; G01N 33/566; G01N 33/6893;**
G01N 2800/26; G01N 2800/52

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
- **Max-Delbrück-Centrum für Molekulare Medizin in der Helmholtz-Gemeinschaft 13125 Berlin (DE)**
- **Charité - Universitätsmedizin Berlin 10117 Berlin (DE)**

(72) Inventors:
- **De la Rosa, Kathrin 10437 Berlin (DE)**
- **Lebedin, Mikhail 16321 Bernau (DE)**
- **Sander, Leif 14169 Berlin (DE)**
- **Kurth, Florian 10555 Berlin (DE)**

(74) Representative: **Kador & Partner PartG mbB Corneliusstraße 15 80469 München (DE)**

# (54) METHOD FOR PROGNOSING THE COURSE OF A SARS-COV-2 INFECTION AND THE RESPONSE TO A SARS-COV-2 IMMUNIZATION AND/OR NATURAL INFECTION BY MEASURING SOLUBLE ACE2

(57) The present invention relates to an *in-vitro-method* for prognosing the course of a SARS-CoV-2 (severe acute respiratory syndrome coronavirus 2) infection and/or the response to a SARS-CoV-2 immunization and/or SARS-CoV-2 natural infection of a subject by using a-sACE2 and/or sACE2 as a marker. The invention further discloses an *in-vitro-assay* (A) for determining the amount of a-sACE2 and to an *in-vitro-assay* (B) for determining the amount of sACE2 in a sample. Additionally, a kit of parts for performing the *in-vitro-assay* (A), a kit of parts for performing the *in-vitro-assay* (B) and a kit of parts (C) for performing the *in-vitro*-method of the present invention are disclosed. Moreover, the present invention relates to a-sACE2 and/or sACE2 as a marker for prognosing the course of a SARS-CoV-2-infection and/or the response to a SARS-CoV-2 immunization and/or SARS-CoV-2 natural infection.

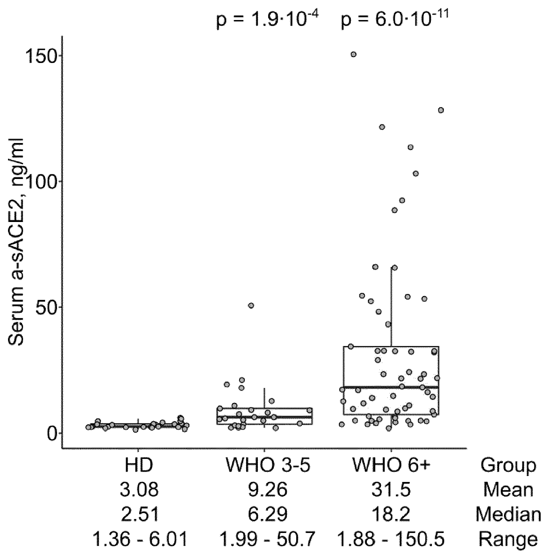


Fig. 3A

EP 4 039 818 A1

SEQUENCE LISTING

<110> Charité - Universitätsmedizin Berlin
Max-Delbrück-Centrum für Molekulare Medizin

<120> Methods for prognosing the course of a SARS-CoV-2 infection and/or the response to a SARS-CoV-2 immunization and/or natural infection

<130> MDC2016 / CH993/2020 / K 71 052

<160> 7

<170> BiSSAP 1.3.6

<210> 1
<211> 1273
<212> PRT
<213> SARS-CoV-2

<220>
<223> SARS-CoV-2 Spike protein full lenght sequence, including signal peptide and cytoplasmatic tail

<400> 1
Met Phe Val Phe Leu Val Leu Leu Pro Leu Val Ser Ser Gln Cys Val
1 5 10 15
Asn Leu Thr Thr Arg Thr Gln Leu Pro Pro Ala Tyr Thr Asn Ser Phe
20 25 30
Thr Arg Gly Val Tyr Tyr Pro Asp Lys Val Phe Arg Ser Ser Val Leu
35 40 45
His Ser Thr Gln Asp Leu Phe Leu Pro Phe Phe Ser Asn Val Thr Trp
50 55 60
Phe His Ala Ile His Val Ser Gly Thr Asn Gly Thr Lys Arg Phe Asp
65 70 75 80
Asn Pro Val Leu Pro Phe Asn Asp Gly Val Tyr Phe Ala Ser Thr Glu
85 90 95
Lys Ser Asn Ile Ile Arg Gly Trp Ile Phe Gly Thr Thr Leu Asp Ser
100 105 110
Lys Thr Gln Ser Leu Leu Ile Val Asn Asn Ala Thr Asn Val Val Ile
115 120 125
Lys Val Cys Glu Phe Gln Phe Cys Asn Asp Pro Phe Leu Gly Val Tyr
130 135 140
Tyr His Lys Asn Asn Lys Ser Trp Met Glu Ser Glu Phe Arg Val Tyr
145 150 155 160
Ser Ser Ala Asn Asn Cys Thr Phe Glu Tyr Val Ser Gln Pro Phe Leu
165 170 175
Met Asp Leu Glu Gly Lys Gln Gly Asn Phe Lys Asn Leu Arg Glu Phe
180 185 190
Val Phe Lys Asn Ile Asp Gly Tyr Phe Lys Ile Tyr Ser Lys His Thr
195 200 205
Pro Ile Asn Leu Val Arg Asp Leu Pro Gln Gly Phe Ser Ala Leu Glu
210 215 220
Pro Leu Val Asp Leu Pro Ile Gly Ile Asn Ile Thr Arg Phe Gln Thr
225 230 235 240
Leu Leu Ala Leu His Arg Ser Tyr Leu Thr Pro Gly Asp Ser Ser Ser
245 250 255
Gly Trp Thr Ala Gly Ala Ala Ala Tyr Tyr Val Gly Tyr Leu Gln Pro
260 265 270
Arg Thr Phe Leu Leu Lys Tyr Asn Glu Asn Gly Thr Ile Thr Asp Ala
275 280 285
Val Asp Cys Ala Leu Asp Pro Leu Ser Glu Thr Lys Cys Thr Leu Lys
290 295 300

```
Ser Phe Thr Val Glu Lys Gly Ile Tyr Gln Thr Ser Asn Phe Arg Val
305                 310                 315                 320
Gln Pro Thr Glu Ser Ile Val Arg Phe Pro Asn Ile Thr Asn Leu Cys
                325                 330                 335
Pro Phe Gly Glu Val Phe Asn Ala Thr Arg Phe Ala Ser Val Tyr Ala
            340                 345                 350
Trp Asn Arg Lys Arg Ile Ser Asn Cys Val Ala Asp Tyr Ser Val Leu
        355                 360                 365
Tyr Asn Ser Ala Ser Phe Ser Thr Phe Lys Cys Tyr Gly Val Ser Pro
    370                 375                 380
Thr Lys Leu Asn Asp Leu Cys Phe Thr Asn Val Tyr Ala Asp Ser Phe
385                 390                 395                 400
Val Ile Arg Gly Asp Glu Val Arg Gln Ile Ala Pro Gly Gln Thr Gly
                405                 410                 415
Lys Ile Ala Asp Tyr Asn Tyr Lys Leu Pro Asp Asp Phe Thr Gly Cys
            420                 425                 430
Val Ile Ala Trp Asn Ser Asn Asn Leu Asp Ser Lys Val Gly Gly Asn
        435                 440                 445
Tyr Asn Tyr Leu Tyr Arg Leu Phe Arg Lys Ser Asn Leu Lys Pro Phe
    450                 455                 460
Glu Arg Asp Ile Ser Thr Glu Ile Tyr Gln Ala Gly Ser Thr Pro Cys
465                 470                 475                 480
Asn Gly Val Glu Gly Phe Asn Cys Tyr Phe Pro Leu Gln Ser Tyr Gly
                485                 490                 495
Phe Gln Pro Thr Asn Gly Val Gly Tyr Gln Pro Tyr Arg Val Val Val
            500                 505                 510
Leu Ser Phe Glu Leu Leu His Ala Pro Ala Thr Val Cys Gly Pro Lys
        515                 520                 525
Lys Ser Thr Asn Leu Val Lys Asn Lys Cys Val Asn Phe Asn Phe Asn
    530                 535                 540
Gly Leu Thr Gly Thr Gly Val Leu Thr Glu Ser Asn Lys Lys Phe Leu
545                 550                 555                 560
Pro Phe Gln Gln Phe Gly Arg Asp Ile Ala Asp Thr Thr Asp Ala Val
                565                 570                 575
Arg Asp Pro Gln Thr Leu Glu Ile Leu Asp Ile Thr Pro Cys Ser Phe
            580                 585                 590
Gly Gly Val Ser Val Ile Thr Pro Gly Thr Asn Thr Ser Asn Gln Val
        595                 600                 605
Ala Val Leu Tyr Gln Asp Val Asn Cys Thr Glu Val Pro Val Ala Ile
    610                 615                 620
His Ala Asp Gln Leu Thr Pro Thr Trp Arg Val Tyr Ser Thr Gly Ser
625                 630                 635                 640
Asn Val Phe Gln Thr Arg Ala Gly Cys Leu Ile Gly Ala Glu His Val
                645                 650                 655
Asn Asn Ser Tyr Glu Cys Asp Ile Pro Ile Gly Ala Gly Ile Cys Ala
            660                 665                 670
Ser Tyr Gln Thr Gln Thr Asn Ser Pro Arg Arg Ala Arg Ser Val Ala
        675                 680                 685
Ser Gln Ser Ile Ile Ala Tyr Thr Met Ser Leu Gly Ala Glu Asn Ser
    690                 695                 700
Val Ala Tyr Ser Asn Asn Ser Ile Ala Ile Pro Thr Asn Phe Thr Ile
705                 710                 715                 720
Ser Val Thr Thr Glu Ile Leu Pro Val Ser Met Thr Lys Thr Ser Val
                725                 730                 735
Asp Cys Thr Met Tyr Ile Cys Gly Asp Ser Thr Glu Cys Ser Asn Leu
            740                 745                 750
Leu Leu Gln Tyr Gly Ser Phe Cys Thr Gln Leu Asn Arg Ala Leu Thr
        755                 760                 765
Gly Ile Ala Val Glu Gln Asp Lys Asn Thr Gln Glu Val Phe Ala Gln
    770                 775                 780
Val Lys Gln Ile Tyr Lys Thr Pro Pro Ile Lys Asp Phe Gly Gly Phe
785                 790                 795                 800
Asn Phe Ser Gln Ile Leu Pro Asp Pro Ser Lys Pro Ser Lys Arg Ser
```

```
                805                 810                 815
Phe Ile Glu Asp Leu Leu Phe Asn Lys Val Thr Leu Ala Asp Ala Gly
            820                 825                 830
Phe Ile Lys Gln Tyr Gly Asp Cys Leu Gly Asp Ile Ala Ala Arg Asp
        835                 840                 845
Leu Ile Cys Ala Gln Lys Phe Asn Gly Leu Thr Val Leu Pro Pro Leu
    850                 855                 860
Leu Thr Asp Glu Met Ile Ala Gln Tyr Thr Ser Ala Leu Leu Ala Gly
865                 870                 875                 880
Thr Ile Thr Ser Gly Trp Thr Phe Gly Ala Gly Ala Ala Leu Gln Ile
                885                 890                 895
Pro Phe Ala Met Gln Met Ala Tyr Arg Phe Asn Gly Ile Gly Val Thr
            900                 905                 910
Gln Asn Val Leu Tyr Glu Asn Gln Lys Leu Ile Ala Asn Gln Phe Asn
        915                 920                 925
Ser Ala Ile Gly Lys Ile Gln Asp Ser Leu Ser Ser Thr Ala Ser Ala
    930                 935                 940
Leu Gly Lys Leu Gln Asp Val Val Asn Gln Asn Ala Gln Ala Leu Asn
945                 950                 955                 960
Thr Leu Val Lys Gln Leu Ser Ser Asn Phe Gly Ala Ile Ser Ser Val
                965                 970                 975
Leu Asn Asp Ile Leu Ser Arg Leu Asp Lys Val Glu Ala Glu Val Gln
            980                 985                 990
Ile Asp Arg Leu Ile Thr Gly Arg Leu Gln Ser Leu Gln Thr Tyr Val
        995                 1000                1005
Thr Gln Gln Leu Ile Arg Ala Ala Glu Ile Arg Ala Ser Ala Asn Leu
    1010                1015                1020
Ala Ala Thr Lys Met Ser Glu Cys Val Leu Gly Gln Ser Lys Arg Val
1025                1030                1035                1040
Asp Phe Cys Gly Lys Gly Tyr His Leu Met Ser Phe Pro Gln Ser Ala
                1045                1050                1055
Pro His Gly Val Val Phe Leu His Val Thr Tyr Val Pro Ala Gln Glu
            1060                1065                1070
Lys Asn Phe Thr Thr Ala Pro Ala Ile Cys His Asp Gly Lys Ala His
        1075                1080                1085
Phe Pro Arg Glu Gly Val Phe Val Ser Asn Gly Thr His Trp Phe Val
    1090                1095                1100
Thr Gln Arg Asn Phe Tyr Glu Pro Gln Ile Ile Thr Thr Asp Asn Thr
1105                1110                1115                1120
Phe Val Ser Gly Asn Cys Asp Val Val Ile Gly Ile Val Asn Asn Thr
                1125                1130                1135
Val Tyr Asp Pro Leu Gln Pro Glu Leu Asp Ser Phe Lys Glu Glu Leu
            1140                1145                1150
Asp Lys Tyr Phe Lys Asn His Thr Ser Pro Asp Val Asp Leu Gly Asp
        1155                1160                1165
Ile Ser Gly Ile Asn Ala Ser Val Val Asn Ile Gln Lys Glu Ile Asp
    1170                1175                1180
Arg Leu Asn Glu Val Ala Lys Asn Leu Asn Glu Ser Leu Ile Asp Leu
1185                1190                1195                1200
Gln Glu Leu Gly Lys Tyr Glu Gln Tyr Ile Lys Trp Pro Trp Tyr Ile
                1205                1210                1215
Trp Leu Gly Phe Ile Ala Gly Leu Ile Ala Ile Val Met Val Thr Ile
            1220                1225                1230
Met Leu Cys Cys Met Thr Ser Cys Cys Ser Cys Leu Lys Gly Cys Cys
        1235                1240                1245
Ser Cys Gly Ser Cys Cys Lys Phe Asp Glu Asp Asp Ser Glu Pro Val
    1250                1255                1260
Leu Lys Gly Val Lys Leu His Tyr Thr
1265                1270
```

<210> 2
<211> 1244
<212> PRT

<213> SARS-CoV-2

<220>
<223> SARS-CoV-2 Spike without signal peptid, cytoplasmic tail is truncated

<400> 2
Val Ser Ser Gln Cys Val Asn Leu Thr Thr Arg Thr Gln Leu Pro Pro
1 5 10 15
Ala Tyr Thr Asn Ser Phe Thr Arg Gly Val Tyr Tyr Pro Asp Lys Val
20 25 30
Phe Arg Ser Ser Val Leu His Ser Thr Gln Asp Leu Phe Leu Pro Phe
35 40 45
Phe Ser Asn Val Thr Trp Phe His Ala Ile His Val Ser Gly Thr Asn
50 55 60
Gly Thr Lys Arg Phe Asp Asn Pro Val Leu Pro Phe Asn Asp Gly Val
65 70 75 80
Tyr Phe Ala Ser Thr Glu Lys Ser Asn Ile Ile Arg Gly Trp Ile Phe
85 90 95
Gly Thr Thr Leu Asp Ser Lys Thr Gln Ser Leu Leu Ile Val Asn Asn
100 105 110
Ala Thr Asn Val Val Ile Lys Val Cys Glu Phe Gln Phe Cys Asn Asp
115 120 125
Pro Phe Leu Gly Val Tyr Tyr His Lys Asn Asn Lys Ser Trp Met Glu
130 135 140
Ser Glu Phe Arg Val Tyr Ser Ser Ala Asn Asn Cys Thr Phe Glu Tyr
145 150 155 160
Val Ser Gln Pro Phe Leu Met Asp Leu Glu Gly Lys Gln Gly Asn Phe
165 170 175
Lys Asn Leu Arg Glu Phe Val Phe Lys Asn Ile Asp Gly Tyr Phe Lys
180 185 190
Ile Tyr Ser Lys His Thr Pro Ile Asn Leu Val Arg Asp Leu Pro Gln
195 200 205
Gly Phe Ser Ala Leu Glu Pro Leu Val Asp Leu Pro Ile Gly Ile Asn
210 215 220
Ile Thr Arg Phe Gln Thr Leu Leu Ala Leu His Arg Ser Tyr Leu Thr
225 230 235 240
Pro Gly Asp Ser Ser Ser Gly Trp Thr Ala Gly Ala Ala Ala Tyr Tyr
245 250 255
Val Gly Tyr Leu Gln Pro Arg Thr Phe Leu Leu Lys Tyr Asn Glu Asn
260 265 270
Gly Thr Ile Thr Asp Ala Val Asp Cys Ala Leu Asp Pro Leu Ser Glu
275 280 285
Thr Lys Cys Thr Leu Lys Ser Phe Thr Val Glu Lys Gly Ile Tyr Gln
290 295 300
Thr Ser Asn Phe Arg Val Gln Pro Thr Glu Ser Ile Val Arg Phe Pro
305 310 315 320
Asn Ile Thr Asn Leu Cys Pro Phe Gly Glu Val Phe Asn Ala Thr Arg
325 330 335
Phe Ala Ser Val Tyr Ala Trp Asn Arg Lys Arg Ile Ser Asn Cys Val
340 345 350
Ala Asp Tyr Ser Val Leu Tyr Asn Ser Ala Ser Phe Ser Thr Phe Lys
355 360 365
Cys Tyr Gly Val Ser Pro Thr Lys Leu Asn Asp Leu Cys Phe Thr Asn
370 375 380
Val Tyr Ala Asp Ser Phe Val Ile Arg Gly Asp Glu Val Arg Gln Ile
385 390 395 400
Ala Pro Gly Gln Thr Gly Lys Ile Ala Asp Tyr Asn Tyr Lys Leu Pro
405 410 415
Asp Asp Phe Thr Gly Cys Val Ile Ala Trp Asn Ser Asn Asn Leu Asp
420 425 430
Ser Lys Val Gly Gly Asn Tyr Asn Tyr Leu Tyr Arg Leu Phe Arg Lys

```
            435                 440                 445
Ser Asn Leu Lys Pro Phe Glu Arg Asp Ile Ser Thr Glu Ile Tyr Gln
        450                 455                 460
Ala Gly Ser Thr Pro Cys Asn Gly Val Glu Gly Phe Asn Cys Tyr Phe
465                 470                 475                 480
Pro Leu Gln Ser Tyr Gly Phe Gln Pro Thr Asn Gly Val Gly Tyr Gln
                485                 490                 495
Pro Tyr Arg Val Val Val Leu Ser Phe Glu Leu Leu His Ala Pro Ala
            500                 505                 510
Thr Val Cys Gly Pro Lys Lys Ser Thr Asn Leu Val Lys Asn Lys Cys
        515                 520                 525
Val Asn Phe Asn Phe Asn Gly Leu Thr Gly Thr Gly Val Leu Thr Glu
    530                 535                 540
Ser Asn Lys Lys Phe Leu Pro Phe Gln Gln Phe Gly Arg Asp Ile Ala
545                 550                 555                 560
Asp Thr Thr Asp Ala Val Arg Asp Pro Gln Thr Leu Glu Ile Leu Asp
                565                 570                 575
Ile Thr Pro Cys Ser Phe Gly Gly Val Ser Val Ile Thr Pro Gly Thr
            580                 585                 590
Asn Thr Ser Asn Gln Val Ala Val Leu Tyr Gln Asp Val Asn Cys Thr
        595                 600                 605
Glu Val Pro Val Ala Ile His Ala Asp Gln Leu Thr Pro Thr Trp Arg
    610                 615                 620
Val Tyr Ser Thr Gly Ser Asn Val Phe Gln Thr Arg Ala Gly Cys Leu
625                 630                 635                 640
Ile Gly Ala Glu His Val Asn Asn Ser Tyr Glu Cys Asp Ile Pro Ile
                645                 650                 655
Gly Ala Gly Ile Cys Ala Ser Tyr Gln Thr Gln Thr Asn Ser Pro Arg
            660                 665                 670
Arg Ala Arg Ser Val Ala Ser Gln Ser Ile Ile Ala Tyr Thr Met Ser
        675                 680                 685
Leu Gly Ala Glu Asn Ser Val Ala Tyr Ser Asn Asn Ser Ile Ala Ile
    690                 695                 700
Pro Thr Asn Phe Thr Ile Ser Val Thr Thr Glu Ile Leu Pro Val Ser
705                 710                 715                 720
Met Thr Lys Thr Ser Val Asp Cys Thr Met Tyr Ile Cys Gly Asp Ser
                725                 730                 735
Thr Glu Cys Ser Asn Leu Leu Leu Gln Tyr Gly Ser Phe Cys Thr Gln
            740                 745                 750
Leu Asn Arg Ala Leu Thr Gly Ile Ala Val Glu Gln Asp Lys Asn Thr
        755                 760                 765
Gln Glu Val Phe Ala Gln Val Lys Gln Ile Tyr Lys Thr Pro Pro Ile
    770                 775                 780
Lys Asp Phe Gly Gly Phe Asn Phe Ser Gln Ile Leu Pro Asp Pro Ser
785                 790                 795                 800
Lys Pro Ser Lys Arg Ser Phe Ile Glu Asp Leu Leu Phe Asn Lys Val
                805                 810                 815
Thr Leu Ala Asp Ala Gly Phe Ile Lys Gln Tyr Gly Asp Cys Leu Gly
            820                 825                 830
Asp Ile Ala Ala Arg Asp Leu Ile Cys Ala Gln Lys Phe Asn Gly Leu
        835                 840                 845
Thr Val Leu Pro Pro Leu Leu Thr Asp Glu Met Ile Ala Gln Tyr Thr
    850                 855                 860
Ser Ala Leu Leu Ala Gly Thr Ile Thr Ser Gly Trp Thr Phe Gly Ala
865                 870                 875                 880
Gly Ala Ala Leu Gln Ile Pro Phe Ala Met Gln Met Ala Tyr Arg Phe
                885                 890                 895
Asn Gly Ile Gly Val Thr Gln Asn Val Leu Tyr Glu Asn Gln Lys Leu
            900                 905                 910
Ile Ala Asn Gln Phe Asn Ser Ala Ile Gly Lys Ile Gln Asp Ser Leu
        915                 920                 925
Ser Ser Thr Ala Ser Ala Leu Gly Lys Leu Gln Asp Val Val Asn Gln
    930                 935                 940
```

```
Asn Ala Gln Ala Leu Asn Thr Leu Val Lys Gln Leu Ser Ser Asn Phe
945                 950                 955                 960
Gly Ala Ile Ser Ser Val Leu Asn Asp Ile Leu Ser Arg Leu Asp Lys
                965                 970                 975
Val Glu Ala Glu Val Gln Ile Asp Arg Leu Ile Thr Gly Arg Leu Gln
            980                 985                 990
Ser Leu Gln Thr Tyr Val Thr Gln Gln Leu Ile Arg Ala Ala Glu Ile
        995                 1000                1005
Arg Ala Ser Ala Asn Leu Ala Ala Thr Lys Met Ser Glu Cys Val Leu
    1010                1015                1020
Gly Gln Ser Lys Arg Val Asp Phe Cys Gly Lys Gly Tyr His Leu Met
1025                1030                1035                1040
Ser Phe Pro Gln Ser Ala Pro His Gly Val Val Phe Leu His Val Thr
                1045                1050                1055
Tyr Val Pro Ala Gln Glu Lys Asn Phe Thr Thr Ala Pro Ala Ile Cys
            1060                1065                1070
His Asp Gly Lys Ala His Phe Pro Arg Glu Gly Val Phe Val Ser Asn
        1075                1080                1085
Gly Thr His Trp Phe Val Thr Gln Arg Asn Phe Tyr Glu Pro Gln Ile
    1090                1095                1100
Ile Thr Thr Asp Asn Thr Phe Val Ser Gly Asn Cys Asp Val Val Ile
1105                1110                1115                1120
Gly Ile Val Asn Asn Thr Val Tyr Asp Pro Leu Gln Pro Glu Leu Asp
                1125                1130                1135
Ser Phe Lys Glu Glu Leu Asp Lys Tyr Phe Lys Asn His Thr Ser Pro
            1140                1145                1150
Asp Val Asp Leu Gly Asp Ile Ser Gly Ile Asn Ala Ser Val Val Asn
        1155                1160                1165
Ile Gln Lys Glu Ile Asp Arg Leu Asn Glu Val Ala Lys Asn Leu Asn
    1170                1175                1180
Glu Ser Leu Ile Asp Leu Gln Glu Leu Gly Lys Tyr Glu Gln Tyr Ile
1185                1190                1195                1200
Lys Trp Pro Trp Tyr Ile Trp Leu Gly Phe Ile Ala Gly Leu Ile Ala
                1205                1210                1215
Ile Val Met Val Thr Ile Met Leu Cys Cys Met Thr Ser Cys Cys Ser
            1220                1225                1230
Cys Leu Lys Gly Cys Cys Ser Cys Gly Ser Cys Cys
        1235                1240
```

<210> 3
<211> 223
<212> PRT
<213> SARS-CoV-2

<220>
<223> Receptor Binding Domain (RBD) of SARS-CoV-2, Wuhan variant

<400> 3

```
Arg Val Gln Pro Thr Glu Ser Ile Val Arg Phe Pro Asn Ile Thr Asn
1               5                   10                  15
Leu Cys Pro Phe Gly Glu Val Phe Asn Ala Thr Arg Phe Ala Ser Val
            20                  25                  30
Tyr Ala Trp Asn Arg Lys Arg Ile Ser Asn Cys Val Ala Asp Tyr Ser
        35                  40                  45
Val Leu Tyr Asn Ser Ala Ser Phe Ser Thr Phe Lys Cys Tyr Gly Val
    50                  55                  60
Ser Pro Thr Lys Leu Asn Asp Leu Cys Phe Thr Asn Val Tyr Ala Asp
65                  70                  75                  80
Ser Phe Val Ile Arg Gly Asp Glu Val Arg Gln Ile Ala Pro Gly Gln
                85                  90                  95
Thr Gly Lys Ile Ala Asp Tyr Asn Tyr Lys Leu Pro Asp Asp Phe Thr
            100                 105                 110
```

```
Gly Cys Val Ile Ala Trp Asn Ser Asn Asn Leu Asp Ser Lys Val Gly
        115                 120                 125
Gly Asn Tyr Asn Tyr Leu Tyr Arg Leu Phe Arg Lys Ser Asn Leu Lys
    130                 135                 140
Pro Phe Glu Arg Asp Ile Ser Thr Glu Ile Tyr Gln Ala Gly Ser Thr
145                 150                 155                 160
Pro Cys Asn Gly Val Glu Gly Phe Asn Cys Tyr Phe Pro Leu Gln Ser
                165                 170                 175
Tyr Gly Phe Gln Pro Thr Asn Gly Val Gly Tyr Gln Pro Tyr Arg Val
            180                 185                 190
Val Val Leu Ser Phe Glu Leu Leu His Ala Pro Ala Thr Val Cys Gly
        195                 200                 205
Pro Lys Lys Ser Thr Asn Leu Val Lys Asn Lys Cys Val Asn Phe
    210                 215                 220

<210> 4
<211> 222
<212> PRT
<213> SARS-CoV-2


<220>
<223> Receptor Binding Domain (RBD) of SARS-CoV-2, United Kingdom (UK)
      variant

<400> 4
Arg Val Gln Pro Thr Glu Ser Ile Val Arg Phe Pro Asn Ile Thr Asn
1               5                   10                  15
Leu Cys Pro Phe Gly Glu Val Phe Asn Ala Thr Arg Phe Ala Ser Val
            20                  25                  30
Tyr Ala Trp Asn Arg Lys Arg Ile Ser Asn Cys Val Ala Asp Tyr Ser
        35                  40                  45
Val Leu Tyr Asn Ser Ala Ser Phe Ser Thr Phe Lys Cys Tyr Gly Val
    50                  55                  60
Ser Pro Thr Lys Leu Asn Asp Leu Cys Phe Thr Asn Val Tyr Ala Asp
65                  70                  75                  80
Ser Phe Val Ile Arg Gly Asp Glu Val Arg Gln Ile Ala Pro Gly Gln
                85                  90                  95
Thr Gly Lys Ile Ala Asp Tyr Asn Tyr Lys Leu Pro Asp Asp Phe Thr
            100                 105                 110
Gly Cys Val Ile Ala Trp Asn Ser Asn Asn Leu Asp Ser Lys Val Gly
        115                 120                 125
Gly Asn Tyr Asn Tyr Leu Tyr Arg Leu Phe Arg Lys Ser Asn Leu Lys
    130                 135                 140
Pro Phe Glu Arg Asp Ile Ser Thr Glu Ile Tyr Gln Ala Gly Ser Thr
145                 150                 155                 160
Pro Cys Asn Gly Val Glu Gly Phe Asn Cys Tyr Phe Pro Leu Gln Ser
                165                 170                 175
Tyr Gly Phe Gln Pro Thr Tyr Gly Val Gly Tyr Gln Pro Tyr Arg Val
            180                 185                 190
Val Val Leu Ser Phe Glu Leu Leu His Ala Pro Ala Thr Val Cys Gly
        195                 200                 205
Pro Lys Lys Ser Thr Asn Leu Val Lys Asn Lys Cys Val Asn
    210                 215                 220

<210> 5
<211> 222
<212> PRT
<213> SARS-CoV-2


<220>
<223> Receptor Bindung Domain (RBD) of SARS-CoV-2, South Africa variant
```

<400> 5
Arg Val Gln Pro Thr Glu Ser Ile Val Arg Phe Pro Asn Ile Thr Asn
1               5                   10                  15
Leu Cys Pro Phe Gly Glu Val Phe Asn Ala Thr Arg Phe Ala Ser Val
            20                  25                  30
Tyr Ala Trp Asn Arg Lys Arg Ile Ser Asn Cys Val Ala Asp Tyr Ser
        35                  40                  45
Val Leu Tyr Asn Ser Ala Ser Phe Ser Thr Phe Lys Cys Tyr Gly Val
    50                  55                  60
Ser Pro Thr Lys Leu Asn Asp Leu Cys Phe Thr Asn Val Tyr Ala Asp
65                  70                  75                  80
Ser Phe Val Ile Arg Gly Asp Glu Val Arg Gln Ile Ala Pro Gly Gln
                85                  90                  95
Thr Gly Asn Ile Ala Asp Tyr Asn Tyr Lys Leu Pro Asp Asp Phe Thr
            100                 105                 110
Gly Cys Val Ile Ala Trp Asn Ser Asn Asn Leu Asp Ser Lys Val Gly
        115                 120                 125
Gly Asn Tyr Asn Tyr Leu Tyr Arg Leu Phe Arg Lys Ser Asn Leu Lys
    130                 135                 140
Pro Phe Glu Arg Asp Ile Ser Thr Glu Ile Tyr Gln Ala Gly Ser Thr
145                 150                 155                 160
Pro Cys Asn Gly Val Lys Gly Phe Asn Cys Tyr Phe Pro Leu Gln Ser
                165                 170                 175
Tyr Gly Phe Gln Pro Thr Tyr Gly Val Gly Tyr Gln Pro Tyr Arg Val
            180                 185                 190
Val Val Leu Ser Phe Glu Leu Leu His Ala Pro Ala Thr Val Cys Gly
        195                 200                 205
Pro Lys Lys Ser Thr Asn Leu Val Lys Asn Lys Cys Val Asn
    210                 215                 220

<210> 6
<211> 271
<212> PRT
<213> Artificial Sequence

<220>
<223> Recombinant Receptor Binding Domain (RBD) of SARS-CoV-2, Wuhan variant, comprising TwinStrep-tag

<400> 6
Met Phe Val Phe Leu Val Leu Leu Pro Leu Val Ser Ser Gln Arg Val
1               5                   10                  15
Gln Pro Thr Glu Ser Ile Val Arg Phe Pro Asn Ile Thr Asn Leu Cys
            20                  25                  30
Pro Phe Gly Glu Val Phe Asn Ala Thr Arg Phe Ala Ser Val Tyr Ala
        35                  40                  45
Trp Asn Arg Lys Arg Ile Ser Asn Cys Val Ala Asp Tyr Ser Val Leu
    50                  55                  60
Tyr Asn Ser Ala Ser Phe Ser Thr Phe Lys Cys Tyr Gly Val Ser Pro
65                  70                  75                  80
Thr Lys Leu Asn Asp Leu Cys Phe Thr Asn Val Tyr Ala Asp Ser Phe
                85                  90                  95
Val Ile Arg Gly Asp Glu Val Arg Gln Ile Ala Pro Gly Gln Thr Gly
            100                 105                 110
Lys Ile Ala Asp Tyr Asn Tyr Lys Leu Pro Asp Asp Phe Thr Gly Cys
        115                 120                 125
Val Ile Ala Trp Asn Ser Asn Asn Leu Asp Ser Lys Val Gly Gly Asn
    130                 135                 140
Tyr Asn Tyr Leu Tyr Arg Leu Phe Arg Lys Ser Asn Leu Lys Pro Phe
145                 150                 155                 160
Glu Arg Asp Ile Ser Thr Glu Ile Tyr Gln Ala Gly Ser Thr Pro Cys

```
                165                 170                 175
Asn Gly Val Glu Gly Phe Asn Cys Tyr Phe Pro Leu Gln Ser Tyr Gly
            180                 185                 190
Phe Gln Pro Thr Asn Gly Val Gly Tyr Gln Pro Tyr Arg Val Val Val
        195                 200                 205
Leu Ser Phe Glu Leu Leu His Ala Pro Ala Thr Val Cys Gly Pro Lys
    210                 215                 220
Lys Ser Thr Asn Leu Val Lys Asn Lys Cys Val Asn Phe Trp Ser His
225                 230                 235                 240
Pro Gln Phe Glu Lys Gly Gly Gly Ser Gly Gly Gly Ser Gly Gly Ser
                245                 250                 255
Ala Trp Ser His Pro Gln Phe Glu Lys His His His His His His
            260                 265                 270
```

```
<210> 7
<211> 816
<212> DNA
<213> Artificial Sequence


<220>
<223> Recombinant Receptor Binding Domain (RBD) of SARS-CoV-2, Wuhan
      variant, comprising TwinStrep-tag and which is codon-optimized
      for expression in human cells

<400> 7
atgttcgtgt ttctggtgct gctgcctctg gtgtccagcc agcgggtgca gcccaccgaa      60

tccatcgtgc ggttccccaa tatcaccaat ctgtgcccct tcggcgaggt gttcaatgcc     120

accagattcg cctctgtgta cgcctggaac cggaagcgga tcagcaattg cgtggccgac     180

tactccgtgc tgtacaactc cgccagcttc agcaccttca agtgctacgg cgtgtcccct     240

accaagctga acgacctgtg cttcacaaac gtgtacgccg acagcttcgt gatccgggga     300

gatgaagtgc ggcagattgc ccctggacag acaggcaaga tcgccgacta caactacaag     360

ctgcccgacg acttcaccgg ctgtgtgatt gcctggaaca gcaacaacct ggactccaaa     420

gtcggcggca actacaatta cctgtaccgg ctgttccgga agtccaatct gaagcccttc     480

gagcgggaca tctccaccga gatctatcag gccggcagca ccccttgtaa cggcgtggaa     540

ggcttcaact gctacttccc actgcagtcc tacggctttc agcccacaaa tggcgtgggc     600

tatcagccct acagagtggt ggtgctgagc ttcgaactgc tgcatgcccc tgccacagtg     660

tgcggcccta agaaaagcac caatctcgtg aagaacaaat gcgtgaactt ctggagtcat     720

ccacaattcg agaagggcgg aggcagcggt ggtggaagtg gaggcagtgc gtggagccat     780

ccgcagtttg aaaaacacca tcaccatcac cattga                               816
```

## Description

The present invention relates to an *in-vitro*-method for prognosing the course of a SARS-CoV-2 (severe acute respiratory syndrome coronavirus 2) infection and/or the response to a SARS-CoV-2 immunization and/or SARS-CoV-2 natural infection of a subject using a-sACE2 (enzymatically active soluble ACE2) and/or sACE2 (soluble ACE2) as a marker. The invention further relates to an *in-vitro*-assay (A) for determining the amount of a-sACE2 and to an *in-vitro-assay* (B) for determining the amount of sACE2 in a sample. Additionally, the invention relates to a kit of parts for performing the *in-vitro-assay* (A), a kit of parts for performing the *in-vitro-assay* (B) and a kit of parts (C) for performing the *in-vitro*-method of the present invention. Moreover, the present invention relates to the use of a-sACE2 and/or sACE2 as a marker for prognosing the course of a SARS-CoV-2-infection and/or the response to a SARS-CoV-2 immunization and/or SARS-CoV-2 natural infection of a SARS-CoV-2 non-infected or a SARS-CoV-2 infected subject.
The severe acute respiratory syndrome coronavirus (SARS-CoV) first emerged 17 years ago. In December 2019, a novel coronavirus (SARS-CoV-2) crossed species barriers to infect humans and was effectively transmitted from person to person, leading to a pneumonia outbreak first reported in Wuhan, China. This virus causes coronavirus disease-19 (COVID-19) with influenza-like symptoms ranging from mild disease to severe lung injury and multi-organ failure, eventually leading to death, especially in older patients with other co-morbidities. Severe acute respiratory coronavirus 2 (SARS-CoV-2) has become a worldwide pandemic but an effective therapy for COVID-19, the illness caused by the virus, is missing. The FDA approved the antiviral drug remdesivir and granted emergency use for baricitinib. Remdesivir has limited efficacy but was shown to shorten time to recovery. Further upcoming therapeutic strategies include antiinflammatory compounds. The corticosteroid dexamethasone reduced risk for death by about 30% for people on ventilators and by about 20% when supplemental oxygen was needed. Immune based COVID-19 therapies under investigation include passive immunization with convalescent plasma or recombinant neutralizing antibodies, as well as the administration of recombinant Angiotensin-converting enzyme 2 (ACE2)-receptor. The latter has been shown to intercept the virus in *in vitro* cell culture. The first vaccines were approved by the European Medicine Agency (EMA) showing more than 90% reduction in the number of symptomatic COVID-19 cases over a median of two months. Importantly, the trials showed similar efficacies for participants at risk to develop mild-moderate or even severe COVID-19 with a score of WHO3-5 and WHO6, respectively, or higher in accordance with the WHO (World Health Organization) clinical progression scale including people with chronic lung disease, asthma, high blood pressure, diabetes, and a high body mass index. Still, open questions remain with regard to unexpected safety issues when the number of vaccinations grows to millions and billions, with regard to vaccine efficacy against severe COVID-19, how long the vaccine will remain effective and if it prevents virus transmission and COVID-19 with a score of WHO1 or higher in accordance with the WHO clinical progression scale.
SARS-CoV-2, is still keeping the world in suspense. Hospitals are stretched to their limits, health systems are overburdened. The quantity and choice of vaccines is limited. Prioritization of groups to receive vaccination is becoming crucial to limit death and serious disease as much as possible. Many people are worried about how severe a SARS-CoV-2 infection will affect them and whether they belong to a risk group for a severe course of the disease.
In this respect, it would be highly desirable to have the ability to predict the severity of a possible future SARS-CoV-2 infection or the further course of an already existing SARS-CoV-2 infection.
A given person would thus be able to better assess his or her own risk to life. Persons with a particularly high risk of a mild-moderate or even severe course of COVID-19 could be specially protected. In addition, such a prediction allows health authorities and hospitals to manage their patients appropriately and thus ensure optimal care or the optimisation of care even in the event of bottlenecks. Infected persons for whom a severe course with intensive medical care is to be expected could be optimally distributed among the hospitals according to the free capacities and planned in advance.
Furthermore, it would further be highly desirable to have the ability to identify persons for whom vaccination/immunization/natural infection is not expected to provide sufficient immunity against SARS-CoV-2. Given the scarcity of vaccine resources, vaccinating such a person would be a waste of resources and of valuable time to build herd immunity as quickly as possible. There is evidence that certain vaccinations/immunizations/natural infections do not confer sufficient immunity to SARS-CoV-2 in 5-40% of individuals. Furthermore, a vaccinated person usually relies on their acquired immunity. A person who is unable to acquire immunity as a result of vaccination is under the false belief that he or she is protected against SARS-CoV-2 and, importantly, that he or she is not a carrier. This puts the person’s life at risk, but also that of those around them who need protection, since such person might become infected and infectious again. Without appropriate behaviour, there is a risk that such persons, despite being vaccinated, will inadvertently contribute to the further spread of SARS-CoV-2, even if they not show symptoms of COVID-19, and/or they may become ill with COVID-19 themselves. The ability to identify such individuals before vaccination could prevent this. It would also make it possible to choose an appropriate/different vaccine with, for example, a mode of action that would be capable to induce immunization in such individuals.
The ability to predict the course of a SARS-CoV-2 infection and/or the response to SARS-CoV-2 immunization and/or SARS-CoV-2 natural infection in a subject would thus be a highly desirable tool.

There is thus an urgent need and accordingly it is the object of the present invention to provide a method for prognosing the course of a SARS-CoV-2 infection and/or the response to SARS-CoV-2 immunization and/or SARS-CoV-2 natural infection in a subject.
The present invention is based on the finding that sACE2 and/or a-sACE2 can be used as markers for prognosing the course of a SARS-CoV-2 infection and/or the response to a SARS-CoV-2 immunization and/or SARS-CoV-2 natural infection in a subject. Particularly, the present invention is based on the surprising finding that both, the amount of a-sACE2 and that of sACE2, correlate with the severity of COVID-19 and that based thereon a prognosis of the course of a SARS-CoV-2-infection and/or the response to a SARS-CoV-2 immunization and/or SARS-CoV-2 natural infection of a subject is possible.
Consequently, it is a further object of the present invention to provide new assays for determining the amount of aACE2 and a-sACE2 with improved accuracy and sensitivity to allow for a reliable, accurate and sensitive prognosis.
Further, the present invention is based on the finding that an *in-vitro*-assay for determining the amount of a-sACE2 in a sample can be provided with high accuracy and sensitivity by the presence of a divalent cation in the sample/mixture and/or is conducted at a restricted number of times within a certain time limit.

Further, the present invention is based on the finding that an *in-vitro*-assay for determining the amount of sACE2 in a sample can be provided with high accuracy and sensitivity by conducting a purifying step and a denaturing step or a step of dissociating of non-covalent bonds before determining the amount of sACE2.
The present invention therefore provides an *in-vitro*-method for prognosing the course of a SARS-CoV-2-infection and/or the response to a SARS-CoV-2 immunization and/or SARS-CoV-2 natural infection of a SARS-CoV-2 non-infected or a SARS-CoV-2 infected subject by using a-sACE2 and/or sACE2 as a marker comprising the step of:

a) determining the amount of a-sACE2 and/or sACE2 in a sample of the subject.

The present invention further provides an *in-vitro*-assay (A) for determining the amount of a-sACE2 in a sample, comprising the steps of:

a) preparing a mixture comprising the sample and a substrate of a-sACE2; and

b) determining the amount of a-sACE2 comprising measuring the amount of a reacted and/or unreacted substrate and/or the amount of fluorescence in the mixture,

wherein the mixture further comprises a divalent cation and/or
wherein step b) is conducted in total 1 to 10 times within 45h after step a) on the mixture obtained in step a).
The present invention further provides an *in-vitro*-Assay (B) for determining an amount of sACE2 in a sample, comprising the steps of:

a) purifying the sACE2, wherein the purifying comprises performing of an affinity purification, to obtain a purified sample;
b) denaturing the purified sample obtained in step a) or dissociating of non-covalent bonds in the purified sample obtained in step a) to obtain a denatured sample or non-covalent bonds dissociated sample; and
c) determining the amount of the sACE2 in the denatured sample or non-covalent bonds dissociated sample obtained in step b).

The present invention further provides a kit of parts (A) for performing assay (A) of the invention, comprising:

a substrate of a-sACE2, preferably a fluorescent substrate of a-sACE2;
optionally a source of a divalent cation, preferably a source of $Zn^{2+}$; and
optionally a manual.

The present invention further provides a kit of parts (B) for performing the assay (B) of the invention, comprising:

a ligand, preferably a bait, optionally immobilized on a matrix;
optionally a denaturing agent or a non-covalent bonds-dissociating agent; and
optionally a manual.

The present invention further provides a kit of parts (C) for performing the *in-vitro* method of the invention, comprising:

the Kit of parts (A) of the invention and/or the Kit of parts (B) of the invention; and

optionally a manual.

More specifically, the present invention is based on the finding that the determined amount of sACE2 and/or a-sACE2 can be used for prognosing the course of a SARS-CoV-2-infection. Thereby, a specific amount of sACE2 and/or a-sACE2 is indicative for a SARS-CoV-2 non-infected subject or SARS-CoV-2 infected subject to develop a course of a SARS-CoV-2-infection with a score of e.g. WHO3 or higher or WHO6 or higher in accordance with the WHO clinical progression scale and hence, whether intensive medical care will be required. Furthermore, such specific amount of sACE2 and/or a-sACE2 is indicative for a SARS-CoV-2 non-infected subject to be a non- or poor responder to a SARS-CoV-2 immunization and/or SARS-CoV-2 natural infection. Furthermore, the new assays according to the invention allow the detection of sACE2 and a-sACE2 with high accuracy and sensitivity.
The angiotensin converting enzyme 2 (ACE2) has been identified as a key receptor for SARS-CoV-2 infections. The ACE2 enzyme is expressed as a transmembrane protein (mACE2) and can be cleaved by the ADAM17 protease inducing its shedding into the extracellular space. More particularly, the ectodomain from mACE2 is cleaved of and released into the extracellular space termed soluble ACE2 or sACE2. sACE2 exists in an enzymatically active form, a-sACE2 (enzymatically active ACE2), and in an enzymatically inactive form, i-sACE2 (enzymatically inactive ACE2). The 2002/2003 pandemic virus SARS-CoV-1 has been shown to modulate ADAM17 activity, thereby increasing receptor shedding and downregulating mACE2 expression. The SARS-coronavirus spike proteins bind with very high affinity to sACE2 and mACE2, the latter of which mediates virus uptake. The induction of antibodies that interfere with ACE2-spike interaction are crucial to develop a neutralizing immune response and confer protective immunity. However, activation of B cells and affinity maturation of potent antibodies strongly depends on the availability of the antigen, i.e. the spike proteins, because masking of protein structures by antibodies and Fab fragments was shown to abrogate efficient neutralizing antibody responses. The invention is based on the surprising finding that elevated levels of a-sACE and sACE2 correlate with a poor antibody response to the SARS-CoV-2 receptor binding domain of the spike protein. Hence, depending on the concentration of sACE2 and its affinity for the virus spike protein, shielding of the virus by its host receptor may render the most crucial virus epitopes unavailable to mount a proper antibody response.
It was now found that a-sACE2 and/or sACE2 are risk factors for a subject to be a non- or poor responder to a SARS-CoV-2 immunization and/or SARS-CoV-2 natural infection and can serve as predictive biomarkers for the efficiency of a SARS-CoV-2 immunization and/or SARS-CoV-2 natural infection.
Furthermore, it was now found that a-sACE2 and/or sACE2 are risk factors for developing severe course of a COVID-19 and can serve as predictive biomarkers for the course of a SARS-CoV-2-infection. It was evidenced that circulating a-sACE2 and/or sACE2 levels are significantly higher in patients with COVID-19-symptoms according to WHO3 or higher according to WHO ordinal scale for clinical improvement .
Accordingly, the *in-vitro*-method according to the present invention, wherein the amount of sACE2 and/or a-sACE2 is determined, provides a prognosis for the course of a SARS-CoV-2 infection and/or the response to a SARS-CoV-2 immunization and/or SARS-CoV-2 natural infection.

*Brief Description of the Drawings*

Figs. 1A-B shows that sACE2 is capable of binding to SARS-CoV-2 spike protein.

Fig. 1C shows that sACE2 efficiently prevents binding of neutralizing antibodies to the RBD of the SARS-CoV-2 spike protein.

Fig. 2A shows the optimized measurement time points and number of measurements for determining the amount of a-sACE2.

Fig. 2B shows that freezing of the sample does not affect the enzymatic activity of a-sACE2.

Fig. 2C shows that serum is the superior material for a-sACE2 activity measurements.

Fig. 2D shows a-sACE2 activity measurements in dependency of a-sACE-concentration.

Fig. 2E shows the optimized $Zn^{2+}$ concentration in Serum and plasma for a-sACE2 enzymatic activity.

Fig. 3A shows that the amount of a-sACE2 correlates with COVID-19 severity as determined by enzymatic activity assay for a-sACE2 by measuring the amount of fluorescence.

Fig. 3B-E shows the amount of a-sACE2 in sera of healthy individuals as determined by enzymatic activity assay for a-sACE2 by measuring the amount of fluorescence.

Fig. 4A shows that the cleaving activity of a-sACE2 is specific for its substrate and shows the optimized $Zn^{2+}$ concentration for a-sACE2 enzymatic activity.

Fig. 4B shows that in healthy individuals a-sACE2 levels fluctuate only poorly over a period of one day.

Fig. 4C shows that in healthy individuals a-sACE2 levels fluctuate over a period of 1.5 years.

Fig. 5A shows that a commercial ELISA cannot detect sACE2 at the same high level as the affinity assay according to the present invention and sACE2 correlates less well with the enzymatic activity assay according to the present invention than with the affinity assay according to the present invention.

Fig. 5B shows an affinity assay according to the present invention, wherein the amount of sACE2 in the blood may be considerable higher as determined via assays relying on detection of enzymatic activity and conventional ELISA.

Figs. 5C shows a comparison of ACE2 concentrations obtained with different assays.

Figs. 5D-E show the optimized SDS detergent concentration.

Fig. 5F shows Pearson correlations of ACE2 concentrations.

Figs. 6A-6D show the optimized concentrations of non-covalent bonds-dissociating agents.

Fig. 7A shows in COVID-19 patients low auto-ACE2-antibody avidity and concentration.

Fig. 7B shows that a polyclonal anti-ACE2 autoantibody interferes with enzymatic activity of a-sACE2.

Figs. 8A-B show that in patients with high sACE2 levels, the RBD specific IgG titer and the potential of antibodies to block ACE2 binding to RBD is significantly reduced.

Fig. 8C shows that sera with a-sACE2 levels above 20 ng/ml show significantly decreased blocking potential of ACE2 binding to RBD, potentially due to a limited RBD specific antibody response as shown in Fig. 8A.

Fig. 9A-D shows that in two patients with high sACE2 serum levels, RBD specific IgG titers and the potential of antibodies to block ACE2 binding to RBD are inversely correlated with a-sACE2 early during the disease.

*In-vitro-Method for prognosing the course of a SARS-CoV-2-infection and/or the response to a SARS-CoV-2 immunization and/or SARS-CoV-2 natural infection*

The present invention provides an *in-vitro*-method for prognosing the course of a SARS-CoV-2-infection and/or the response to a SARS-CoV-2 immunization and/or SARS-CoV-2 natural infection of a SARS-CoV-2 non-infected or a SARS-CoV-2 infected subject by using a-sACE2 and/or sACE2 as a marker.
Particularly, the present invention provides an *in-vitro*-method for prognosing the course of a SARS-CoV-2-infection and/or the response to a SARS-CoV-2 immunization and/or SARS-CoV-2 natural infection of a SARS-CoV-2 non-infected or a SARS-CoV-2 infected subject by using a-sACE2 and/or sACE2 as a marker comprising the step of:

a) determining the amount of a-sACE2 and/or sACE2 in a sample of the subject.

By using a-sACE2 and/or sACE2 as a marker, preferably, the amount of a-sACE2 and/or sACE2 as a marker, the method surprisingly allows for a prognosis as to the course of a SARS-CoV-2 infection and/or the response to a SARS-CoV-2 immunization and/or SARS-CoV-2 natural infection.
The term "ACE2" (angiotensin-converting enzyme 2) as used in the present invention includes sACE2 (soluble ACE2) and mACE2 (membrane-bound ACE2).
The term "sACE2" as used in the present invention comprises a-sACE2 (enzymatically inactive sACE2) and i-sACE2 (enzymatically inactive sACE2). The a-sACE2 exhibits the usual enzymatic activity and is capable of cleaving a substrate of a-sACE2. The i-sACE2 is a form of sACE2 inactivated e.g. due to being bound by a-sACE2 inhibiting agent, partial degradation, etc.

The term "amount of a-sACE2" as used herein preferably means the total amount of a-sACE2.

The term "amount of sACE2" as used herein means the amount of a-sACE2 plus i-sACE2, preferably the total amount of a-sACE2 plus i-sACE2. Furthermore, sACE2 comprises sACE2 capable of binding the SARS-CoV-2 spike protein and not capable of binding SARS-CoV-2 spike protein.

The amount of a-sACE2 in a sample of a subject correlates with the course of a SARS-CoV-2 infection as shown in Fig. 3A By using the amount a-sACE2 as marker, the method allows for a reliable, accurate and sensitive prognosis.

Furthermore, the present inventors have found that also the amount of sACE2 in a sample of a subject correlates with the course of a SARS-CoV-2 infection as shown in Figs. 7A and B. By using the amount of sACE2, including the combined amounts of a-sACE2 and i-sACE2, as marker, the method allows for an even more reliable, accurate and sensitive prognosis in comparison to the use of a-sACE2 as a marker.

It has been found that sACE2 binds with high affinity to the SARS-CoV-2 spike protein and masks the RBD (receptor binding domain) of the SARS-CoV-2 spike protein, thereby efficiently shielding spike recognition (Fig. 1A). This in turn e.g. hampers binding of neutralizing antibodies to the RBD (receptor binding domain) of the SARS-CoV-2 spike protein (Fig. 1B) and SARS-CoV-2 cannot be neutralized and eliminated by antibody effector function. Furthermore, it has been found that the amount of a-sACE2 and/or sACE2 inversely correlates with the response to a SARS-CoV-2 immunization and/or natural infection (Figs. 8A-B, Fig 9A-D).

As also already outlined above, the SARS-CoV-2 virus spike and particularly the RBD of the SARS-CoV-2 virus spike are the major recognition sites for a B-cell to elicit production of neutralizing antibodies as well as for the produced neutralizing antibody itself, or neutralizing antibodies of a passive immunization, to elicit neutralization of SARS-CoV-2. Since the SARS-CoV-2 virus spike and particularly the RBD is bound by a-sACE2, particularly sACE2, and thereby masked, the RBD is not accessible, neither for elicitation of a B-cell-based production of neutralizing antibodies against SARS-CoV-2 nor for the neutralizing antibodies itself, like neutralizing antibodies already present or neutralizing antibodies provided by a passive immunization. Hence, the determining of the amount of a-sACE2 and particularly, sACE2 in a sample of a subject, serves to measure the individual ACE2 shielding potential and can be used to prognose the course of a SARS-CoV-2 infection and/or the response to a SARS-CoV-2 immunization and/or SARS-CoV-2 natural infection.

Preferably, the *in-vitro* method according to the present invention, the SARS-CoV-2 immunization and/or SARS-CoV-2 natural infection is a SARS-CoV-2-vaccination and/or SARS-CoV-2 natural infection, most preferably a SARS-CoV-2-vaccination.

Preferably, in the method, the SARS-CoV-2 non-infected subject and/or the SARS-CoV-2 infected subject is a mammal, more preferably a human.

In the method, in case the amount of a-sACE2 is determined, the sample may preferably be any one used in the art, for example blood like whole blood, blood serum or blood plasma, sputum, feces, urine, nasopharyngeal samples like a nasopharyngeal swab, nasal swap, endonasal swab or throat swab, fibrobronchoscope brush biopsy or bronchoalveolar lavage, more preferably blood, even more preferably blood serum or blood plasma, most preferably blood serum, of the subject. The sample may be a frozen and thawed sample or a sample not subjected to freezing.

In the method, in case the amount of sACE2 is determined, the sample may preferably be any one used in the art, for example blood like whole blood, blood serum or blood plasma, sputum, feces, urine, nasopharyngeal samples like a nasopharyngeal swab, nasal swap, endonasal swab or throat swab, fibrobronchoscope brush biopsy or bronchoalveolar lavage, more preferably blood, even more preferably blood serum or blood plasma, most preferably blood serum , of the subject. The sample may be a frozen and thawed sample or a sample not subjected to freezing.

Preferably, "blood plasma" as used in the present invention refers to the fluid that remains after removal of the blood cells, namely erythrocytes and thrombocytes.

Preferably, "blood serum" as used in the present invention comprises the fluid that remains after removal of the blood cells, namely erythrocytes and thrombocytes, and the clotting factors. The blood serum thus preferably corresponds to the blood plasma minus the clotting factors. The serum may be prepared with or without anticoagulant.

Blood plasma and blood serum are obtained and/or prepared by any method known to the skilled person.

In the method in step a), the amount a-sACE2 and/or sACE2 may be determined by performing any method capable of determining the amount of a-sACE2 and/or sACE2.

Preferably, in step a) the amount of sACE2 is determined. Preferably, the amount of sACE2 determined is the amount of sACE2 capable of binding the SARS-CoV-2 spike protein, more preferably the amount of sACE2 capable of binding the RBD of the SARS-CoV-2 spike protein. More preferably, the amount of sACE2 determined is the total amount of sACE2, even more preferably the total amount of sACE2 capable of binding the SARS-CoV-2 spike protein, most preferably the total amount of sACE2 capable of binding the RBD of the SARS-CoV-2 spike protein.

Preferably, in the method in step a), the amount of a-sACE2 and/or sACE2 is determined by performing a method comprising:

electrochemoluminescence, acridinium ester based chemoluminescence, paramagnetic particles as solid phase like magnetic beads coated actively, i.e. covalent, or passively, i.e. non-covalent, with a ligand or bait of a sACE2 and/or sACE2 by using the StreptactinXT/Twin strep or Biotin/Streptavidin system;

mass spectroscopy (MS), especially liquid chromatography tandem mass spectrometry (LC-MS-MS), using an enzymatic digestion step like tryptic digestion of a sample containing sACE2 or a-sACE2 in combination with separation and quantification of sACE specific fragments or of a sACE specific fragment by mass spectroscopy and comparison to a calibrator or calibrators of known sACE2 content;

enzymatic activity measurement of a-sACE2 e.g. after affinity purification, ion exchange chromatography, semiquantitative or quantitative measurement of the amount of sACE2-mRNA in the sample, e.g. by polymerase chain reaction (PCR)-based methods such as quantitative PCR like RT-PCR, or cycle threshold, ELISA-based systems, Reverse Phase HPLC, wherein the ELISA-based system comprise the measurement of a sACE2 and/or sACE2 in a blood sample, like directly coating of the blood sample on an ELISA plate, washing with suitable buffer and detection of enzymatic activity by a measurable fragment released during cleavage, using e.g. a dye, fluorescence and/or mass spectrometry, and finally comparison with a sample running at the cutoff or in a known position to the cutoff; and/or the *in-vitro* assay (A) according to the present invention and/or the *in-vitro* assay (B) according to the present invention.

More preferably, in step a) the amount of a-sACE2 is determined by performing the *in-vitro-Assay (A)* according to the present invention and/or the amount of sACE2 is determined by performing the *in-vitro* assay (B) according to the present invention. Most preferably, in step a) the amount of sACE2 is determined by performing the *in-vitro* assay (B) according to the present invention.
In case of PCR-based methods, nasopharyngeal samples like a nasopharyngeal swab, nasal swap, endonasal swab or throat swab are preferred.
A SARS-CoV-2 infection in the subject may be determined by any method known to the skilled person, for example by detecting the virus in a sample by semiquantitative measurement of the viral burden by e.g. a polymerase chain reaction (PCR)-based method, such as quantitative PCR like RT-PCR, or cycle threshold. The sample may be any one used in the art, for example blood, like whole blood, blood serum or blood plasma, sputum, feces, urine, nasopharyngeal samples like a nasopharyngeal swab, nasal swap, endonasal swab or throat swab, fibrobronchoscope brush biopsy or bronchoalveolar lavage fluid, preferably nasopharyngeal swabs, nasal swap or endonasal swab, throat swab, fibrobronchoscope brush biopsy or bronchoalveolar lavage fluid, most preferably nasopharyngeal swab, endonasal swab or throat swab.
Preferably, as used in the present invention the term "course of a SARS-CoV-2 infection" refers to the range of possible scores of severity of a SARS-CoV-2 infection that may be caused by the SARS-CoV-2 infection in accordance with the WHO (World Health Organization) clinical progression scale shown in Table 1 below of the WHO Working Group on the Clinical Characterisation and Management of COVID-19 infection, 2020 (*A minimal common outcome measure set for COVID-19 clinical research.* Lancet Infect Dis 20, e192-e197. doi:10.1016/S1473-3099(20)30483-7). The possible scores of a SARS-CoV-2 infection in accordance with the WHO clinical progression scale range from WHO1 to WHO10. The WHO clinical progression scale provides a measure of severity of a SARS-CoV-2- infection/COVID-19 across a range from a score of WHOO (uninfected) to WHO10 (dead).

*Table 1: WHO clinical progression scale*

| ***Patient State*** | ***Descriptor/Symptoms*** | ***Score*** |
|---|---|---|
| *Uninfected* | Uninfected; no viral RNA detected | WHOO |
| *Ambulatory mild COVID-19* | Asymptomatic; viral RNA detected | WHO1 |
| | Symptomatic; independent | WHO2 |
| | Symptomatic; assistance needed | WHO3 |
| *Hospitalized: moderate COVID-19* | Hospitalised; no oxygen therapy* | WHO4 |
| | Hospitalised; oxygen by mask or nasal prongs | WHO5 |

(continued)

| ***Patient State*** | ***Descriptor/Symptoms*** | ***Score*** |
|---|---|---|
| *Hospitalized: severe COVID-19* | Hospitalised; oxygen by NIV or high flow | WHO6 |
| | Intubation and mechanical ventilation, $pO_2/FiO_2 \geq 150$ or $SpO_2/FiO_2 \geq 200$ | WHO7 |
| | Mechanical ventilation $pO_2/FIO_2$ <150 ($SpO_2/FiO_2$ <200) or vasopressors | WHO8 |
| | Mechanical ventilation $pO_2/FiO_2$ <150 and vasopressors, dialysis, or ECMO | WHO9 |
| *Dead* | Death | WHOL0 |
| *ECMO=extracorporeal membrane oxygenation.* | | |
| *$FiO_2$=fraction of inspired oxygen.* | | |
| *NIV=non-invasive ventilation.* | | |
| *$pO_2$=partial pressure of oxygen.* | | |
| *$SpO_2$=oxygen saturation.* | | |
| **If hospitalised for isolation only, record status as for ambulatory patient.* | | |

Preferably, as used in the present invention the term "prognosing" of a course of a SARS-CoV-2 infection refers in case of a SARS-CoV-2 infected subject to prognosing, which score in accordance with the WHO clinical progression scale is likely to be expected in the subject by the present SARS-CoV-2 infection; and in case of a SARS-CoV-2 non-infected subject to prognosing, which score in accordance with the WHO clinical progression scale is likely to be expected in the subject in case of a future SARS-CoV-2 infection.
The term "SARS-CoV-2 non-infected subject" as used in the present invention defines a subject, preferably a human, in which no SARS-CoV-2 infection can be determined, that is the method used for determining a SARS-CoV-2 infection gives a negative result. More preferably, the SARS-CoV-2 non-infected subject is tested negative in a nasopharyngeal swab, endonasal swab or throat swab. A SARS-CoV-2 non-infected subject as used in the present invention corresponds to WHOO in accordance with the WHO clinical progression scale.
The term "SARS-CoV-2 infected subject" as used in the present invention defines a subject, preferably a human, in which a SARS-CoV-2 infection can be determined, that is the method used for determining a SARS-CoV-2 infection gives a positive result. More, preferably, the SARS-CoV-2 infected subject is tested positive in a nasopharyngeal swab, endonasal swab or throat swab. A SARS-CoV-2 infected subject as used in the present invention corresponds to WHO1 to WHO10, like WHO1 to WHO9, in accordance with the WHO clinical progression scale.
The SARS-CoV-2 infected subject may be at an early stage of COVID-onset caused by the SARS-CoV-2 infection, or maybe already at a late stage of COVID-19-onset. An early stage of COVID-19 is defined as the first 10 days after symptom onset. A late stage of COVID-19 is defined as >10 days after symptom onset.
A score of a SARS-CoV-2-infection of less than WHO3 like WHO1 to WHO2 is also referred to as mild COVID-19, that may or may not require ambulatory medical care, but does not require ambulatory medical care with assistance, hospitalized medical care or intensive medical care. Such mild COVID-19 may be asymptomatic or the symptoms may be mild and may comprise for example fever, chills, cough, fatigue, muscle or body aches, headache, loss of taste or smell, sore throat, congestion or runny nose, nausea, vomiting.
A score of a SARS-CoV-2-infection of WHO3 to WHO5, like WHO3 to WHO4, is also referred to as mild-to-moderate COVID-19, requiring ambulatory medical care with assistance or hospitalized medical care, but does not require intensive medical care. The symptoms of such mild-to-moderate COVID-19 may be mild-moderate and may comprise for example fever, chills, cough, fatigue, muscle or body aches, headache, loss of taste or smell, sore throat, congestion or runny nose, nausea, vomiting. A score of a SARS-CoV-2-infection of WHO6 or higher (WHO6+), that is WHO6 to WHO10, like WHO6 to WHO9, is also referred to as severe COVID-19 that may include death. A course of a SARS-CoV-2 infection with a score of WHO6 or higher requires intensive medical care. The symptoms of such severe COVID-19 may be severe and may comprise for example shortness of breath or difficulty breathing, pneumonia, ARDS, inflammatory symptoms, neurological manifestations, low blood-oxygen levels, hypoxia, low oxygen saturation, increased blood clotting and/or cytokine storm.
Hence, a score of a SARS-CoV-2-infection of WHO3 or higher (WHO3+), that is WHO3 to WHO10, like WHO3 to WHO9, comprises mild-to-moderate COVID-19 and severe COVID-19 that may include death. A course of a SARS-CoV-2 infection with a score of WHO3 or higher requires at least ambulatory medical care with assistance or hospitalized medical care and may require intensive medical care.

A score of a SARS-CoV-2-infection of WHO1 or higher (WHO1+), that is WHO1 to WHO10, like WHO1 to WHO9, comprises mild COVID-19, mild-to-moderate COVID-19 and a severe COVID-19 that may include death. A course of a SARS-CoV-2 infection with a score of WHO1 or higher may or may not require ambulatory medical care up to requiring intensive medical care.

The symptoms of mild, mild-to-moderate and/or severe COVID-19 usually appear 2-14 days after exposure to or infection with SARS-CoV-2 the virus.

The term SARS-CoV-2 "immunization" as used in the present invention comprises passive immunization such as by administering neutralizing antibodies and active immunization such as by applying a vaccine. Such vaccine may be a recombinant protein, a Modified Vaccinia Virus Ankara vaccine, an inactivated pathogen, a DNA vaccine, a RNA vaccine or any other vaccine allowing for an active immunization.

The term SARS-CoV-2 "vaccination" as used in the present application is an active immunization, such as by applying a vaccine as defined above.

The term SARS-CoV-2 "natural infection" as used in the present application is a SARS-CoV-2-infection upon exposure to SARS-CoV-2 contaminated environment.

The term "prognosing" of a response to a SARS-CoV-2 immunization, SARS-CoV-2 vaccination and/or SARS-CoV-2 natural infection as used in the present invention relates to prognosing, which response level is likely to be expected in a SARS-CoV-2 non-infected subject or a SARS-CoV-2 infected subject.

Preferably, as used in the present invention, the term "response to a SARS-CoV-2 immunization", the term "response to a SARS-CoV-2 vaccination" and/or "response to a SARS-CoV-2 natural infection" refers to the quality spectrum of possible response levels that may be developed by the subject upon SARS-CoV-2 immunization, SARS-CoV-2 vaccination and/or SARS-CoV-2 natural infection, respectively. Preferably, the quality spectrum of possible response levels comprises non-responder, poor responder and responder.

A non-responder is not protected to a SARS-CoV-2 infection with a score of WHO1 or higher, like WHO3 or higher or WHO6 or higher in accordance with the WHO clinical progression scale. A non-responder may not be protected to mild, mild-moderate, COVID-19 and/or death. A non-responder transmits the SARS-CoV-2-virus. A non-responder does not generate an immune response, that is no protective antibodies against SARS-CoV-2 are generated.

A poor responder is partly protected to a SARS-CoV-2 infection with a score of WHO1 or higher, like WHO3 or higher or WHO6 or higher in accordance with the WHO clinical progression scale. A poor responder may be partly protected to mild, mild-moderate, severe COVID-19 and/or death. A poor responder does or does not transmit the virus. A poor responder generates a non-efficient immune response.

A responder is completely protected to a SARS-CoV-2-infection with a score of WHO1 or higher, like WHO3 or higher or WHO6 or higher in accordance with the WHO clinical progression scale, preferably at least up to 2-6 months post SARS-CoV-2 immunization and/or post SARS-CoV-2 natural infection. A poor responder may be protected to mild, mild-moderate and/or severe COVID-19. A responder does not transmit the virus. That is, a responder generates an effective immune response including neutralizing antibodies.

Preferably, the non- or poor responder shows at least low abundance, more preferably very low abundance or even no SARS-CoV-2 neutralizing antibodies upon SARS-CoV-2 vaccination and/or SARS-CoV-2 natural infection. More preferably, the non- or poor responder shows at least low abundance and/or low avidity/affinity of SARS-CoV-2 spike protein specific neutralizing antibodies, more preferably SARS-CoV-2 RBD protein specific neutralizing antibodies, more preferably ACE2 binding site of the spike specific neutralizing antibodies. More preferably, the SARS-CoV-2 neutralizing antibodies, the SARS-CoV-2 spike protein specific neutralizing antibodies, the SARS-CoV-2 RBD protein specific neutralizing antibodies, or the ACE2 binding site of the spike specific neutralizing antibodies are IgG and/or IgA and/or IgM. Most preferably, the SARS-CoV-2 neutralizing antibody is a SARS-CoV-2 ACE2 binding site of the spike protein specific IgG and/or IgA and/or IgM.

Since SARS-CoV-2 spike protein specific and/or RBD specific and/or ACE2 binding site of the spike specific neutralizing antibodies target the ACE2 binding site of the spike, a non- or poor responder showing low abundance or no SARS-CoV-2 spike protein and/or RBD specific and/or ACE2 binding site specific neutralizing antibodies is thus incapable of efficiently blocking spike binding to the ACE2 receptor.

The amount of specific antibodies may be determined by any method known to the skilled person, for example by lateral flow assay (LFA), ELISA, or preferably bead based multiplexed immunoassays. Antibody titers that can block the interaction between RBD and ACE2 may be determined by any method to the skilled person, for example by a blocking ELISA detection tool. The amount of neutralizing antibodies may be determined by any method known to the skilled person, for example by neutralization assays with real virus or pseudovirus. The sample may any one used in the art, preferably blood.

For human non- or poor responders medium-to-low abundance of SARS-CoV-2 neutralizing antibodies is defined as half maximum neutralizing titers below 1:500, low abundance of neutralizing antibodies is defined as half maximum neutralizing titers below 1:100 and very low abundance of neutralizing antibodies is defined as half maximum neutralizing titers below 1:50 measured at any time after seroconversion due to SARS-CoV-2 infection and/or vaccination.

For human non- or poor responders medium-to-low abundance of SARS-CoV-2 RBD specific IgG or IgA antibodies is defined as endpoint titers below 1:1000 at any day in the first 10 days after symptom onset upon SARS-CoV-2 natural infection and/or after SARS-CoV-2 vaccination; low abundance of SARS-CoV-2 RBD specific IgG or IgA antibodies is defined as endpoint titers below 1:200 at any day in the first 10 days after symptom onset upon SARS-CoV-2 natural infection and/or after SARS-CoV-2 vaccination; very low abundance of SARS-CoV-2 RBD specific IgG or IgA antibodies is defined as endpoint titers below 1:100 at any day in the first 10 days after symptom onset upon SARS-CoV-2 natural infection and/or after SARS-CoV-2 vaccination. Medium-to-low abundance of SARS-CoV-2 RBD specific IgG or IgA antibodies is defined as endpoint titers below 1:5000 at any day post 10 days of symptom onset; low abundance of SARS-CoV-2 RBD specific IgG or IgA antibodies is defined as endpoint titers below 1:1000 at any day post 10 days of symptom onset; very low abundance of SARS-CoV-2 RBD specific IgG or IgA antibodies is defined as endpoint titers below 1:200 at any day in the first 10 days after symptom onset.

For human non- or poor responders medium-to-low ACE2 binding site specific antibodies are defined as below 50% inhibition of spike binding or RBD binding to ACE2 determined at 1:10 serum dilution in presence of 0.5 μg/ml recombinant ACE2 when RBD is used as a solid carrier.

Low ACE2 binding site specific antibodies are defined as below 25% inhibition of spike binding or RBD binding to ACE2 determined at 1:10 serum dilution in presence of 0.5 μg/ml recombinant ACE2 when RBD is used as a solid carrier. Very low ACE2 binding site specific antibodies are defined as below 5% inhibition of spike binding or RBD binding to ACE2 determined at 1:10 serum dilution in presence of 0.5 μg/ml recombinant ACE2 when RBD is used as a solid carrier. Preferably, for human non- or poor responders medium-to-low ACE2 binding site specific antibodies are defined as a serum dilution that blocks 80% of maximum binding (BD80) below 20, when serial dilutions are tested in presence of 20 ng/ml RBD fused to a murine Fc tag for binding to ACE2, which is coupled to a solid carrier. Low ACE2 binding site specific antibodies are defined as a BD80 below 10, when serial dilutions are tested in presence of 20 ng/ml RBD fused to a murine Fc tag for binding to ACE2, which is coupled to a solid carrier. Very low ACE2 binding site specific antibodies are defined as a BD80 below 5, when serial dilutions are tested in presence of 20 ng/ml RBD fused to a murine Fc tag for binding to ACE2, which is coupled to a solid carrier.

Furthermore, for human non- or poor responders RBD specific antibodies capable of blocking sACE2 binding but not completely overlapping with the sACE2 receptor binding motif (RBM) of the RBD may be developed when the RBD is in a down-conformation and not bound by sACE2. The mechanism of neutralization of these antibodies is probably achieved by steric hindrance. As the overlap with the sACE2 binding site is only partial, neutralization may be in non- or poor responders less effective to block sACE2 binding and, consequently, neutralization of the virus. Non- or poor responders that developed antibodies against the RBM or sites that only partially overlap with the RBM may be discriminated from responders with ELISAs based on mutant RBDs that either have part of the RBM mutated or parts of the non-RBM target sites. Such an assay would allow to identify non- or poor responders with poor sACE2 blocking antibodies and responders with efficient sACE2 blocking antibodies.

Preferably, in the method of the invention, an amount determined in step a) of:

a-sACE2 of 2.6 ng/ml or more, determined as described in example 3a, and/or

sACE2 of 8 ng/ml or more, determined as described in example 5b

is indicative for the SARS-CoV-2 non-infected subject or SARS-CoV-2 infected subject to develop a course of a SARS-CoV-2-infection with a score of WHO3 or higher in accordance with the WHO clinical progression scale and/or being a non- or poor responder to a SARS-CoV-2 immunization and/or SARS-CoV-2 natural infection.

The indication for the development of a course of a SARS-CoV-2-infection with a score of WHO3 or higher in accordance with the WHO clinical progression scale and/or for being a non- or poor responder to a SARS-CoV-2 immunization and/or SARS-CoV-2 natural infection increases with increasing amounts of a-sACE2 and/or sACE2.

Preferably, to be indicative for a score of WHO3 or higher and/or for being a non- or poor responder, the amount of a-sACE2 is 6.1 ng/ml or more, more preferably 7 ng/ml or more, more preferably 15 ng/ml or more, even more preferably 30 ng/ml or more and most preferably 55 ng/ml or more, determined as described in examples 3a. Usually, the amount of a-sACE2 is not higher than 300 ng/ml, preferably not higher than 200 ng/ml, determined as described in example 3a. Preferably, to be indicative for a score of WHO3 or higher and/or for being a non- or poor responder, the amount of sACE2 is 10 ng/ml or more, more preferably 20 ng/ml or more, even more preferably 40 ng/ml or more, still more preferably 100 ng/ml or more, still even more preferably 500 ng/ml or more, further still even more preferably 1000 ng/ml or more and most preferably 1500 ng/ml or more, determined as described in example 5b. Usually, the amount of sACE2 is not higher than 10.000 ng/ml, determined as described in example 5b.

More preferably, in the method of the invention, an amount determined in step a) of:

a-sACE2 of 2.6 ng/ml or more and less than 55 ng/ml, determined as described example 3a, and/or

sACE2 of 8 ng/ml or more and less than 1500 ng/ml, determined as described in example 5b

is indicative for the SARS-CoV-2 non-infected subject or SARS-CoV-2 infected subject to develop a course of a SARS-CoV-2-infection with a score of WHO3 to WHO5 in accordance with the WHO clinical progression scale and/or being a non- or poor responder to a SARS-CoV-2 immunization and/or SARS-CoV-2 natural infection.
Preferably, to be indicative for a score of WHO3 to WHO5 and/or for being a non- or poor responder, the amount of a-sACE2 is for 6.1 ng/ml or more, more preferably 7 ng/ml or more and less than 30 ng/ml, most preferably less than 15 ng/ml, determined as described in examples 3a.
Preferably, to be indicative for a score of WHO3 to WHO5 and/or for being a non- or poor responder, the amount of sACE2 is 10 ng/ml or more, more preferably 20 ng/ml or more, even more preferably 40 ng/ ml or more, most preferably 100 ng/ml or more and less than 1000 ng/ml, most preferably less than 500 ng/ml, determined as described in example 5b.
Even more preferably, in the method of the invention, an amount determined in step a) of:

a-sACE2 of 15 ng/ml or more, determined as described example 3a, and/or

sACE2 of 500 ng/ml or more, determined as described in example 5b

is indicative for the SARS-CoV-2 non-infected subject or SARS-CoV-2 infected subject to develop a course of a SARS-CoV-2-infection with a score of WHO6 or higher in accordance with the WHO clinical progression scale and/or being a non- or poor responder to a SARS-CoV-2 immunization and/or SARS-CoV-2 natural infection.
The indication for the development of a course of a SARS-CoV-2-infection with a score of WHO6 or higher in accordance with the WHO clinical progression scale and/or for being a non- or poor responder to a SARS-CoV-2 immunization and/or SARS-CoV-2 natural infection increases with increasing amounts of a-sACE2 and/or sACE2.
Preferably, to be indicative for a score of WHO6 or higher and/or for being a non- or poor responder, the amount of a-sACE2 is 30 ng/ml or more and most preferably 55 ng/ml or more, determined as described in example 3a. Usually, the amount of a-sACE2 is not higher than 300 ng/ml, preferably not higher than 200 ng/ml, determined as described in example 3a.
Preferably, to be indicative for a score of WHO6 or higher and/or for being a non- or poor responder, the amount of sACE2 is 1000 ng/ ml or more and most preferably 1500 ng/ml or more, determined as described in example 5b. Usually, the amount of sACE2 is not higher than 10.000 ng/ml, determined as described in example 5b.
Preferably, in the method of the invention an amount determined in step a) of:

a-sACE2 of less than 7 ng/ml, determined as described in example 3a, and/or
sACE2 of less than 100 ng/ml, determined as described in example 5b

is indicative for the SARS-CoV-2 non-infected subject or SARS-CoV-2 infected subject to develop a course of a SARS-CoV-2-infection with a score of lower than WHO3 in accordance with the WHO clinical progression scale and/or being a responder to a SARS-CoV-2 immunization and/or SARS-CoV-2 natural infection.
More preferably, to be indicative for a score of lower than WHO3 and/or for being a responder, the amount of a-sACE2 is less than 6.1 ng/ml, even more preferably less than 2.6 ng/ml, determined as described in examples 3a.
More preferably, to be indicative for a score of lower than WHO3 and/or for being a responder, the amount of sACE2 is less than 40 ng/ml, even more preferably less than 20 ng/ml, still even more preferably less than 10 ng/ ml, most preferably less than 8 ng/ml, determined as described in example 5b.
Preferably, the method further comprises the step of:
b) comparing the amount of a-sACE2 and/or sACE2 determined in step a) with an a-sACE2 reference amount and/or a-sACE2 reference amount, respectively.
Preferably, in the method, the reference amount of a-sACE2 and/or sACE2 is the mean value of a non-infected donor cohort. A representative cohort may for example have the following criteria: 46% male and 54% female with female donors of mean age 46 years (min 23; max 83 years) and a mean BMI of 24.6 (min 17.4; max 37.9) and male donors of a mean age of 48 years (min 20; max 86 years) and a mean BMI of 25.9 (min 20.7; max 36.9). For the representative cohort a-sACE2 levels were measured as described in example 3a showing a mean at 1.96 ng/ml for female (min 0.87; max 5.86 ng/ml) and a mean of 2.67 ng/ml for male (min 1.1; max 8.6 ng/ml).
Preferably, in the method of the invention,
an amount of a-sACE2 and/or sACE2 higher than the a-sACE2 reference amount and/or the sACE2 reference amount resulting from the step of comparing b)
is indicative for the SARS-CoV-2 non-infected subject or SARS-CoV-2 infected subject to develop a course of a SARS-CoV-2-infection with a score of WHO3 or higher in accordance with the WHO clinical progression scale and/or being a non- or poor responder to a SARS-CoV-2 immunization and/or SARS-CoV-2 natural infection.
The indication for the development of a course of a SARS-CoV-2-infection with a score of WHO3 or higher in accordance with the WHO clinical progression scale and/or of being a non- or poor responder in response to a SARS-CoV-2 immunization and/or SARS-CoV-2 natural infection increases with an increasing ratio of a-sACE2 and/or sACE2 versus the a-sACE2 reference amount and/or the sACE2 reference amount, respectively.
Preferably, to be indicative for a score of WHO3 or higher and/or for being a non- or poor responder, the amount of a-sACE2 is at least 1.2 fold higher, more preferably at least 1.5 fold higher, even more preferably at least 1.8 fold higher, still more preferably at least 2.5 fold higher, still even more preferably at lest 3 fold higher and most preferably at least 7 fold higher than the a-sACE2 reference amount, when both amounts are determined with the same method. Usually, the amount of a-sACE2 is not more than 120 fold higher, like 100 fold higher than the a-sACE2 reference amount.
Preferably, to be indicative for a score of WHO3 or higher and/or for being a non- or poor responder, the amount of sACE2 is at least 1.2 fold higher, preferably at least 1.5 fold higher, more preferably at least 1.8 fold higher, even more preferably at least 2.5 fold higher, still more preferably at least 3 fold higher, still even more preferably at least 10 fold higher, further still even more preferably at least 20 fold higher and most preferably at least 30 fold higher than the sACE2 reference amount, when both amounts are determined with the same method. Usually, the amount of sACE2 is not more than 120 fold higher, like 100 fold higher than the sACE2 reference.
More preferably, in the method of the invention,

an amount of a-sACE2 higher than the a-sACE2 reference amount and less than 15 fold higher than the a-sACE2 reference amount resulting from the step of comparing b), and/or

an amount sACE2 higher than the sACE2 reference amount and less than 30 fold higher than the sACE2 reference amount resulting from the step of comparing b)

is indicative for the SARS-CoV-2 non-infected subject or SARS-CoV-2 infected subject to develop a course of a SARS-CoV-2-infection with a score of WHO3 to WHO5 in accordance with the WHO clinical progression scale and/or being a non- or poor responder to a SARS-CoV-2 immunization and/or SARS-CoV-2 natural infection.
Preferably, to be indicative for a score of WHO3 to WHO5 and/or for being a non- or poor responder, the amount of a-sACE2 is at least 1.2 fold higher, more preferably at least 1.5 fold higher, even more preferably at least 1.8 fold higher, still more preferably at least 2.5 fold higher, most preferably at least 3 fold higher and less than 10 fold higher, more preferably less than 7 fold higher, most preferably less than 5 fold higher than the a-sACE2 reference amount, when both amounts are determined with the same method.
Preferably, to be indicative for a score of WHO3 to WHO5 and/or for being a non- or poor responder, the amount of sACE2 is at least 1.2 fold higher, preferably at least 1.5 fold higher, more preferably at least 1.8 fold higher, even more preferably at least 2.5 fold higher, still more preferably at least 3 fold higher, most preferably at least 10 fold higher and less than 20 fold higher, most preferably less than 15 fold higher than the sACE2 reference amount, when both amounts are determined with the same method.
Even more preferably, in the method of the invention,
an amount of a-sACE2 and/or sACE2 higher than the a-sACE2 reference amount and/or the sACE2 reference amount resulting from the step of comparing b)
is indicative for the SARS-CoV-2 non-infected subject or SARS-CoV-2 infected subject to develop a course of a SARS-CoV-2-infection with a score of WHO6 or higher (WHO6+; severe COVID-19) in accordance with the WHO clinical progression scale and/or being a non- or poor responder to a SARS-CoV-2 immunization and/or SARS-CoV-2 natural infection.
The indication for the development of a course of a SARS-CoV-2-infection with a score of WHO6 or higher in accordance with the WHO clinical progression scale and/or for being a non- or poor responder to a SARS-CoV-2 immunization and/or SARS-CoV-2 natural infection increases with an increasing ratio of a-sACE2 and/or sACE2 versus the a-sACE2 reference amount and/or the sACE2 reference amount, respectively.
Preferably, to be indicative for a score of WHO6 or higher and/or for being a non- or poor responder, the amount of a-sACE2 is at least 5 fold higher, preferably at least 7 fold higher and most preferably at least 10 fold higher than the a-sACE2 reference amount, when both amounts are determined with the same method. Usually, the amount of a-sACE2 is not more than 120 fold higher, like 100 fold higher than the a-sACE2 reference amount.
Preferably, to be indicative for a score of WHO6 or higher and/or for being a non- or poor responder, the amount of sACE2 is at least 5 fold higher, preferably at least 7 fold higher, more preferably at least 10 fold higher, even more preferably at least 15 fold higher, still even more preferably at least 20 fold higher and most preferably at least 30 fold higher than the sACE2 reference amount, when both amounts are determined with the same method. Usually, the amount of sACE2 is not more than 100 fold higher than the sACE2 reference amount.

Preferably, in the method of the invention,

an amount of a-sACE2 of less than 3 fold higher than the a-sACE2 reference amount resulting from the step of comparing b), and/or

an amount of sACE2 of less than 10 fold higher than the sACE2 reference amount resulting from the step of comparing b)

is indicative for the SARS-CoV-2 non-infected subject or SARS-CoV-2 infected subject to develop a course of a SARS-CoV-2-infection with a score of less than WHO3 in accordance with the WHO clinical progression scale and/or being a responder to a SARS-CoV-2 immunization and/or SARS-CoV-2 natural infection.

Preferably, to be indicative for a score of lower than WHO3 and/or for being a responder, the amount of a-sACE2 is less than 2.5 fold higher, more preferably less than 1.8 fold higher, even more preferably less than 1.5 fold higher, still more preferably less than 1.2 fold higher and most preferably equal to or less than the a-sACE2 reference amount, when both amounts are determined with the same method.

Preferably, to be indicative for a score of lower than WHO3 and/or for being a responder, the amount of sACE2 is less than 3 fold higher, preferably less than 2.5 fold higher, more preferably less than 1.8 fold higher, even more preferably less than 1.5 fold higher, still more preferably less than 1.2 fold higher, most preferably equal to or less than the sACE2 reference amount, when both amounts are determined with the same method.

It was found that some subjects produce anti-ACE2 autoantibodies possibly due to an autoimmune reaction. Furthermore, the binding of such an anti-ACE2 autoantibody to a-sACE2 leads to the inactivation of its enzymatic activity. Both a-sACE2 and i-sACE2 are capable of efficiently binding to and masking the SARS-CoV-2 spike protein, specifically the RBD (receptor binding domain) of the SARS-CoV-2 spike protein. The majority of sACE in a subject may be in the form of inactive i-sACE2. In particular, it has been demonstrated that more than 96% of the total amount of sACE2 is i-sACE2.

Hence, preferably, in the method, sACE2 is used as a marker, the determined amount is the amount of sACE2, preferably determined by performing the *in-vitro* assay (B), and, if applicable, the reference amount is a sACE2 reference amount.

Furthermore, the *in-vitro* method according to the present invention for prognosing allows for classifying a SARS-CoV-2 non-infected subject or a SARS-CoV-2 infected subject as a subject of risk for developing a SARS-CoV-2-infection with a score of WHO3 or higher, a score of WHO3 to WHO5, a score of WHO6 or higher, in accordance with the WHO clinical progression scale and/or for classifying as a poor-responder or non-responder to a SARS-CoV-2 immunization and/or SARS-CoV-2 natural infection.

Vice versa, the *in-vitro* method according to the present invention for prognosing allows for classifying a SARS-CoV-2 non-infected subject or a SARS-CoV-2 infected subject as a subject of no risk for developing a SARS-CoV-2-infection with a score of WHO3 or higher in accordance with the WHO clinical progression scale and/or for classifying as a responder to a SARS-CoV-2 immunization and/or SARS-CoV-2 natural infection.

Hence, the present invention further provides a method for stratifying a SARS-CoV-2 infected or SARS-CoV-2 non-infected subject, by applying the *in-vitro*-method according to the present invention, for the need of medical care and/or for the treatment with a SARS-CoV-2 immunization comprising the step of:

a) determining an amount of a sACE2 and/or sACE2 in a sample of the subject.

Furthermore, the present invention provides a method for stratifying a SARS-CoV-2 infected or SARS-CoV-2 non-infected subject for the need of medical care and/or for the treatment with a SARS-CoV-2 immunization comprising the steps of:

a) determining an amount of a sACE2 and/or sACE2 in a sample of the subject;

b) comparing the amount of a-sACE2 and/or sACE2 determined in step a) with an a-sACE2 reference amount and/or a sACE2 reference amount, respectively,

preferably, wherein

an amount of a-sACE2 and/or sACE2 higher than the a-sACE2 reference amount and/or the sACE2 reference amount is indicative for the SARS-CoV-2 non-infected subject or SARS-CoV-2 infected subject:

- that the subject will require at least ambulatory medical care with assistance, like ambulatory medical care with assistance, intensive medical care and/or will develop mild-moderate COVID-19, severe COVID-19 that may include death; and/or

- that the subject is not suitable for the treatment with a SARS-CoV-2 immunization,

and/or
wherein an amount of a-sACE2 and/or sACE2 lower than the a-sACE2 reference amount and/or the sACE2 reference amount is indicative for the SARS-CoV-2 non-infected subject or SARS-CoV-2 infected subject:

- that the subject will not require medical assistance, like ambulatory medical care with assistance, and have mild COVID-19; and/or

- that the subject is suitable for the treatment with a SARS-CoV-2 immunization.

In the present invention, it is to be understood that any embodiment disclosed for the *in-vitro-assay* (A) and/or the *in-vitro-assay* (B) and/or the *in-vitro*-method is/are mutually applicable to each other. Furthermore, the embodiments disclosed for the *in-vitro-assay* (A) and/or the *in-vitro-assay* (B) and/or *in-vitro*-method are applicable for the methods for stratifying.

*In-vitro-Assav (A) for determining an amount of a-sACE2*

The present invention provides an *in-vitro-assay* (A) for determining an amount of a-sACE2 in a sample, comprising the steps of:

a) preparing a mixture comprising the sample and a substrate of a-sACE2; and

b) determining the amount of a-sACE2 comprising measuring an amount of a reacted and/or unreacted substrate and/or an amount of fluorescence in the mixture,

wherein the mixture further comprises a divalent cation and/or
wherein step b) is conducted in total 1 to 10 times within 45h after step a) on the mixture obtained in step a).

The assay (A) of the present invention provides an increased sensitivity and accuracy for determining the amount a-sACE2 in a sample over the prior art. In particular, the assay (A) provides an increased sensitivity and accuracy due to the divalent cation comprised in the mixture or due to the conducting of step b) 1 to 10 times within 45h after step a) on the mixture obtained in step a) or an even higher increased sensitivity and accuracy due to the cumulating effects of both. When the assay (A) is used in the *in-vitro*-method according to the present invention, it eventually allows for a precise prognosis.

However, since assay (A) is based on measuring the enzymatic activity, it is limited to a determination of the amount a-sACE2. Hence, it is limited in view of measuring the entire sACE2 masking potential of the RBD and/or SARS-CoV-2 spike protein. However, in contrast to assay (B) described further below and capable of determining the amount of sACE2 and particular the amount of sACE2 capable of binding the SARS-CoV-2 spike protein, assay (A) provides a higher sensitivity, is less time consuming when being performed, highly simplistic and extraordinary suited for large scale screenings.

The term "amount of a-sACE2" as used herein preferably means the total amount of a-sACE2.

In assay (A), the sample may be a sample of a subject. Preferably, the sample may be any one used in the art, for example blood like whole blood, blood serum or blood plasma, sputum, feces, urine, and nasal samples like nasopharyngeal swabs, nasal or endonasal swabs, throat swabs, fibrobronchoscope brush biopsy or bronchoalveolar lavage fluid, more preferably blood, even more preferably blood serum or blood plasma, most preferably blood serum, of the subject. The subject is preferably a mammal, more preferably a human. The sample may be a frozen and thawed sample or a sample not subjected to freezing.

The sACE2 inhibiting agent is any agent, which inhibits or prevents the enzymatic activity of sACE2. Preferably, for use in the present invention, the inhibiting agent may be an anti-ACE2 autoantibody produced by the subject's body.

Preferably, the preparing of the mixture in step a) is done by any methods known to the skilled person, for example by combining all components finally comprised in the mixture, particularly the sample and the substrate of a-sACE2, followed by mixing and/or vortexing.

In assay (A) the mixture comprises a divalent cation. Preferably, the divalent cation is a coenzyme of a-sACE2. Such coenzyme provides for an enhanced enzymatic activity of a-sACE2 allowing for detecting of minor amounts of a-sACE2 resulting in an increased sensitivity of the assay (A). More preferably, the divalent cation is $Zn^2$. $Zn^{2+}$ is a coenzyme

of a-sACE2. However, too high concentrations of $Zn^{2+}$ lead to at least a partial inhibition of the enzymatic activity of a-sACE2 and hence, the $Zn^{2+}$ concentration may be restricted by an upper limit and is preferably 10 mM or less, more preferably 5 mM or less, even more preferably 3.4 mM or less. Preferably, the mixture comprises $Zn^{2+}$ at a final concentration of $Zn^{2+}$ from 0.001 mM to less than 10 mM, more preferably from 0.01 mM to 5 mM, even more preferably from 0.1 mM to 3.4 mM mM, most preferably from 0.3 mM to 2 mM.

Preferably, in assay (A) the final concentration of the divalent cation is adjusted by adding an appropriate amount of a source of a divalent cation to the mixture and/or sample. Hence, in assay (A) the mixture preferably comprises a source of a divalent cation.

Preferably, the source of a divalent cation is a source of $Zn^{2+}$. More preferably, the source of $Zn^{2+}$ is selected from the group consisting of $ZnCl_2$, $ZnF_2$, $ZnBr_2$, $ZnI_2$, $(C_6H_5O_7)_2Zn_3 \cdot 2H_2O$ (zinc citrate), zinc hexafluorosilicat hydrate, $[H_2C{=}C(CH_3)CO_2]_2Zn$ (zinc methacrylate), $ZnMoO_4$ (zinc molybdate), $Zn(NO_3)_2 \cdot xH_2O$ (zinc nitrate hydrate), $ZnC_2O_4 \cdot xH_2O$ (zinc oxalate hydrate), $Zn(ClO_4)_2{\cdot}6H_2O$ (zinc perchlorate hexahydrate), $Zn_3(PO_4)_2$ (zinc phosphate) $ZnSO_4 \cdot 7H_2O$ (zinc sulfate heptahydrate), $Zn(BF_4)_2{\cdot}xH_2O$ (zinc tetrafluoroborate hydrate), $(CH_3C_6H_4SO_3)_2Zn{\cdot}xH_2O$ (zinc p-toluolsulfonate hydrate) and any combination thereof. Most preferably, the source of $Zn^{2+}$ is $ZnCl_2$.

More preferably, the final concentration of the divalent cation is adjusted by adding an appropriate amount of a divalent cation-containing buffer to the mixture and/or sample. Hence, in assay (A) the mixture preferably comprises a divalent cation containing buffer, wherein the divalent cation is defined as above. Usually, a $Zn^{2+}$-containing buffer comprises $Zn^{2+}$ in a range of 1 to 300 $\mu$M.

Preferably, the divalent cation-containing buffer comprises a source of a divalent cation. Even more preferably, the divalent cation-containing buffer is a $Zn^2$-containing buffer comprising a source of $Zn^{2+}$, wherein the source of $Zn^{2+}$ is defined as above.

Furthermore, the buffer may comprise salts, preferably in concentrations close to physiological conditions, an appropriately adjusted pH balance and protease inhibitors, wherein the preparation of such divalent cation-containing buffer can be done by any method known to skilled person and is within the skills of the skilled person.

Preferably, the mixture is free of heparin or substantially free of heparin. That means if applicable, only the amounts of heparin endogenously present in the sample may be comprised in the mixture. Preferably, no heparin is added to the sample and/or the mixture. Heparin is a chelating agent and complexes divalent cations, particularly $Zn^{2+}$, leading to a decreased availability of the divalent cations as a coenzyme for a-sACE2. Thus, heparin may act as an inhibitor for the enzymatic activity of a-sACE2 and its presence might reduce the sensitivity of assay (A).

Preferably, the mixture is free of a chelating agent like for instance EDTA (ethylenediaminetetraacetic acid), DMSA (dimercaptosuccinic acid), DMPS (2,3-Di-mercapto-1-propanesulfonic acid), Unithiol (sodium salt of DMPS), heparin, citrate and/or potassium oxalate/sodium fluoride, preferably the mixture is free of EDTA. Preferably, no chelating agent, preferably EDTA, is added to the sample and/or the mixture. Chelating agents complex divalent cations, particularly $Zn^{2+}$, leading to a decreased availability of the divalent cations as a coenzyme for a-sACE2. Thus, chelating agents may act as an inhibitor for the enzymatic activity of a-sACE2 and its presence would reduce the sensitivity of assay (A).

Preferably, in assay (A) the substrate of a-sACE2 is any substance that can be specifically converted by the enzymatic activity of a-sACE2 in a corresponding enzymatically controlled reaction. In such reaction, a-sACE2 specifically cleaves or changes the unreacted substrate into a reacted substrate, preferably by generating fluorescence. More preferably, the substrate of a-sACE2 is a non-fluorescent substrate of a-ACE2 like Ang I (angiotensin I) of human, mouse or rat origin, Ang II (angiotensin II) of human, mouse or rat origin, or $^{125}$I-AngII (1-125 radiolabeled angiotensin II), or a fluorescent substrate of a-sACE2, even more preferably a fluorescent substrate of a-sACE2, most preferably a quenched fluorescent substrate of a-sACE2. Such quenched fluorescent substrate usually comprises a fluorophore and a quencher, wherein the fluorescence of the fluorophore is quenched by the quencher, for instance since the latter is sterically arranged close-by the fluorophore. Upon cleavage and hence, release of the quencher from the fluorophore, fluorescence of the fluorophore occurs. Even more preferably, the quencher of the quenched fluorescent substrate is specifically cleaved by a-sACE2. Still even more preferably the quenched fluorescent substrate is Mca-APK(Dnp) (Mca-APK(Dnp)) or Mca-YVADAPK (Dnp), wherein Mca ((7-methoxycoumarin-4-yl)acetyl) is the fluorophore and Dnp (2,4-dinitrophenyl) is the quencher quenching the Mca fluorescence, wherein the Pro-Lys linker is specifically cleaved by a-sACE2, most preferably the substrate is Mca-Ala-Pro-Lys(Dnp).

The determining of the amount of a-sACE2 in step b) can be accomplished by any method known to the skilled person as long as the method is suitable for measuring an amount of unreacted and/or reacted substrate, preferably for measuring an amount of fluorescence. Suitable methods for measuring an amount of unreacted and/or reacted substrate are for example mass spectrometry, ELISA-based systems, Reverse Phase HPLC. The amount of the cleaved substrate may for instance be measured by mass-spectrometry as described in Elased et al., 2006, (Elased, K.M., Cunha, T.S., Gurley, S.B., Coffman, T.M., Morris, M., 2006. New mass spectrometric assay for angiotensin-converting enzyme 2 activity. Hypertension 47, 1010-1017. doi:10.1161/01.HYP.0000215588.38536.30). Suitable methods for measuring an amount of fluorescence are for example a photometrical measurement like using 340 nm excitation and 420 nm emission light filters, or a measurement of the cleavage activity of a-sACE2 by using radiolabeled peptides like $^{125}$I-AngII and

separating out the angiotensin peptides by high performance liquid chromatography (HPLC) in order to measure Ang-(1-7) levels. In case of a photometrical measurement, the samples may display a wide variation in 400-450 nm absorbance, which may influence the fluorescence reading and may prevents correct calculation. This issue can be addressed by measuring the absorbance at preferably about 450 nm and calculating its influence on the fluorescence. Most preferred for a particular accurate determination of the amount of a-sACE2 is the relation between an absorbance at about 450 nm and a fluorescence of about 340/about 420 nm.

Preferably, in assay (A) no solid carrier is used for measuring unreacted and/or reacted substrate, preferably for measuring an amount of fluorescence. Due to the high sensitivity of assay (A), an enrichment of a-sACE2 by using a solid carrier is dispensable. This has the advantage that a washing of the complex of a-sACE2/solid carrier, which is formed when using a solid carrier, is dispensable. Furthermore, less reagents like, beads, recombinant proteins and/or detection reagents are necessary and need to be handled.

Preferably, the mixture comprises the substrate, preferably the quenched fluorescent, for a-sACE2 in an excessive concentration in view of the expected enzymatic activity of a-sACE2. This avoids an exhaustion of the substrate and finally incorrect determination of the amount of a-sACE2. More preferably, in case of a quenched fluorescent substrate is used, the mixture comprises the quenched fluorescent substrate in a final concentration of 1 $\mu$M to 500 $\mu$M, more preferably of 10 $\mu$M to 200 $\mu$M, even more preferably of 20 $\mu$M to 80 $\mu$M for e.g. an amount of 10 $\mu$l to 100 $\mu$l serum.

In assay (A), step b) is conducted in total 1 to 10 times within 45h after step a) on the mixture obtained in step a). In case in step b) the fluorescence of a fluorophore is measured, the fluorophore is quenched by conducting the measurement of step b), presumably due to the energy input during measuring. Hence, with increasing number of times of the measurement, the total duration of the measurement time/the total exposure time to excitation light/the intensity of exposure to excitation light, to which the mixture is subjected, increases and the fluorophore is increasingly quenched. Finally, the measured amount of fluorescence in the mixture does not correlate anymore with the amount of a-sACE2 actually present in the mixture, leading to a falsification of the determined amount of a-sACE2. On the other hand, conducting of step b) more than once is desirable, since it allows determining of high (10-100 ng/ml), medium (1-10 ng/ml) and/or low (<1-10 ng/ml) amounts of a-sACE2. By the limitation the conducting step b) to 1 to 10 times within 45h after step a) on the mixture obtained in step a), the amount of a-sACE2 present in the sample can be determined most accurately, while concomitantly high, medium and/or low amounts of ACE2 in the sample can be precisely determined.

Preferably, step b) is conducted in total 1 to 10 times within 30h, more preferably within 25h, most preferably within 21h after step a). If step b) is conducted more than 45h after step a) as claimed, degradation of proteins, peptides etc. in the mixture occurs, falsifying the results determined in step b).

Preferably, in assay (A), step b) is conducted in total 1 to 8 times, more preferably 2 to 6 times and most preferably 3 to 4 times within 30h, more preferably within 25h, most preferably within 21h after step a).

Preferably, in assay (A), when step b) is conducted in total 1 to 10 times, preferably 1 to 8 times, more preferably 2 to 6 times and most preferably 3 to 4 times, the time interval between each conduction of step b), preferably each conduction of the measurement in step b), is 0.25h to 18h, preferably 0.5h to 12h.

Preferably, in assay (A), step b) is conducted at least once, preferably 1 to 3 times, even more preferably 1 to 2 times, even more preferably once, at each time point $t_1$=0h to <0.5h; $t_2$=0.5h to <2h; $t_3$=2h to <10h and/or $t_4$=10h to 30h after step a); more preferably at each time point $t_1$=0h to 0.25h; $t_2$=0.5h to 1.5h; $t_3$=3h to 8h and/or $t_4$=17h to 21h after step a). The time point ti is preferably immediately following step a).

Generally, high amounts of a-sACE2 can be best determined, when the time interval between step a) and step b) is short, e.g. ti, and high amounts of a-sACE2 can be best determined, when the time interval is prolonged, e.g. $t_4$.

As already mentioned above, conducting of step b) more than once allows determining of high, medium and/or low amounts of a-sACE2, wherein the above-indicated preferred number of times of performing step b), preferred time points and/or time intervals are particularly advantageous to determine high, medium and/or low amounts of a-sACE2. The fluorescence increases with an increasing time interval between step a) and step b). However, the amount of fluorescence measured is only proportional to the amount of a-sACE2 present within a certain time-frame before/until saturation of cleaved substrate. Usually, the initial enzyme velocity (enzymatic activity over time) is measured, which allows properly determining the kinetics of an enzyme. If the amount of a-sACE2 is too high, e.g. a shadowing/overlapping in the fluorescence may occur. If the amount of a-sACE2 is too low, e.g. the lower detection limit may be reached. In both cases, the measured fluorescence is not proportional to a certain amount of a-sACE2 and an accurate determination of the amount of a-sACE2 in the sample is impeded. By determining the amount of a-sACE2, only a restricted number of times, at certain time points and/or in certain time intervals, this problem may be overcome and low, medium and high amounts of a-sACE2 can be accurately measured.

Preferably, in assay (A), step a) is conducted only once. Hence, preferably, in assay (A), step b) is conducted each time on the same mixture obtained in step a). However, it is obvious that also step a) might be conducted as many times as step b) and the conduction of step b) is conducted each time on a different mixture obtained from each conducted step a), however, this is time consuming and furthermore requires many resources, e.g. a larger amount of sample and substrate, and is hence, less preferred.

*Assay (B) for determining an amount of a-sACE2*

Particularly, the present invention further provides an *in-vitro-Assay* (B) for determining an amount of sACE2 in a sample, comprising the steps of:

a) purifying the sACE2, wherein the purifying comprises performing of an affinity purification, to obtain a purified sample;

b) denaturing the purified sample obtained in step a) or dissociating of non-covalent bonds in the purified sample obtained in step a) to obtain a denatured sample or non-covalent bonds dissociated sample; and

c) determining the amount of the sACE2 in the denatured sample or non-covalent bonds dissociated sample obtained in step b).

In contrast to the assays of the prior art, the assay (B) of the present invention allows for determining the amount of sACE2, that is the combined amounts of a-sACE2 and i-sACE2, in a sample, particularly the amount of sACE capable of binding the SARS-CoV-2 spike protein. In particular, the assay (B) of the present invention provides an increased sensitivity and accuracy for determining the amount of sACE2, particularly the amount of sACE capable of binding the SARS-CoV-2 spike protein, in a sample over the prior art. Concomitantly, assay (B) can detect the entire amount of a-sACE2 and i-sACE2, allowing to measure the entire sACE2 masking potential of RBD and/or SARS-CoV-2 spike protein and, when used in the *in-vitro*-method according to the present invention, eventually allows for an even more precise prognosis. The assay (B) is thus even more suited then assay (A) for diagnostic screenings. However, the assay (B) is more time consuming and complicated when being performed than assay (A). Therefore, assay (B) allows to measure the entire ACE2 masking potential and can be used to more precisely predict the quality of B-cell responses after SARS-CoV-2 natural infection and/or SARS-CoV-2 vaccination and the response to a SARS-CoV-2 passive immunization.
The term "amount of sACE2" as used herein preferably means the total amount of sACE2.
The term "amount of sACE2" as used herein means the amount of a-sACE2 plus i-sACE2, preferably the total amount of a-sACE2 plus i-sACE2.
Preferably, the amount of sACE2 is the amount of sACE2 capable of binding the SARS-CoV-2 spike protein, more preferably the amount of sACE2 capable of binding the RBD of the SARS-CoV-2 spike protein.
More preferably, the amount of sACE2 is the total amount of sACE2, even more preferably the total amount of sACE2 capable of binding the SARS-CoV-2 spike protein, most preferably the total amount of sACE2 capable of binding the RBD of the SARS-CoV-2 spike protein.
By being capable of detecting the amount, preferably the total amount, capable of binding to SARS-CoV-2 spike protein or the RBD, the assay (B) has the advantage that only variants of sACE2 are detected that are functionally effective in epitope masking. Particularly, sACE2 may further comprise variants of i-sACE2 and/or a-sACE2 which may not be capable of binding to the SARS-CoV-2 spike protein or the RBD, due to alternative splice variants, mutations, degradation etc.
In assay (B), the sample may be a sample of a subject. Preferably, the sample of the subject may be any one used in the art, for example blood like whole blood, blood serum or blood plasma, sputum, feces, urine, nasopharyngeal samples like a nasopharyngeal swab, nasal swap, endonasal swab or throat swab, fibrobronchoscope brush biopsy or bronchoalveolar lavage fluid, more preferably urine, nasopharyngeal samples, bronchoalveolar lavage fluid or blood, even more preferably urine, nasopharyngeal samples or blood, still more preferably urine or blood, still even more preferably blood serum or blood plasma, most preferably a blood serum of the subject The subject is preferably a mammal, more preferably a human. The sample may be a frozen and thawed sample or a sample not subjected to freezing.
Preferably, in assay (B) in step a), the affinity purification is any method known to the skilled person allowing for specifically purifying sACE2, preferably purifying the total amount of sACE2, from the sample. For example, the affinity purification is a method based protein interaction partners of the type analyte-ligand like enzyme-substrate, antibody-antigen or enzyme-coenzyme, wherein the ligand may be any molecule to which sACE2, that is both a-sACE2 and i-sACE2, exhibits an affinity and the analyte is sACE2. More preferably, the affinity purification is a pull-down assay of the bait and prey type. Even more preferably the pull-down assay is of the bait and prey type, wherein the bait is any molecule to which sACE2 exhibits an affinity, preferably a SARS-CoV-2 spike protein, preferably the RBD (receptor binding domain) of the SARS-CoV-2 spike protein, and the prey is sACE2.
The term "purifying" designates a step in which the amount of proteins other than sACE2 is reduced. Thereby, the sensitivity and accuracy of the assay (B) is increased.
Preferably, the purified sample comprises sACE2 and only minor amounts of other proteins co-purified or entrained during purification, beside unavoidable contaminants, like salts and fluids. More preferably, the purified sample consists essentially of sACE2 beside unavoidable contaminants. This may be effected by further purification steps using any suitable method known in the art.

Preferably, in assay (B) the ligand, preferably the bait, is immobilized on a matrix. Preferably, the immobilisation on the matrix is accomplished by any method known to the skilled person. More preferably, the immobilisation is by covalent binding to the matrix, e.g. the matrix has an inherent covalent binding affinity to the ligand or bait, or the matrix is coated with e.g with StreptactinXT or the matrix is pre-activated by e.g. by N-hydroxysuccinimide, carboxylic groups, carboxylate groups or carboxyl groups, or by non-covalent binding to the matrix, e.g. via tags like NI-NTA, His, Streptag, anti-GST, anti-Flag, anti-c-myc, anti-HA or anti-V5. Preferably, the matrix are beads, more preferably beads allowing for an immobilization by covalent binding as stated above for the matrix, e.g. magnetic beads covalently coated with StreptactinXT and capable of binding with high affinity a Twin-Strep coupled protein, like a Twin-Strep coupled recombinant RBD.

Preferably, the SARS-CoV-2 spike protein is used as a bait. More preferably, the bait comprises any peptide sequence of the SARS-CoV-2 spike protein, i.e. any naturally occurring variant of the SARS-CoV-2 spike protein or any mutated variant of the naturally occurring variant of the SARS-CoV-2 spike protein, like the spike proteins of the naturally occurring variants Wuhan, United Kingdom (UK; B1.1.7), South Africa (B1.351) or of the mutated variants comprising mutations TR, KN, YF, EK, QM, QH, NY and/or NF of SEQ ID NO:1 or SEQ ID NO:2, preferably SEQ ID NO:2, described in Table 2 below, wherein these mutations may be present alone or two or more of them may be present in any combination, or any fragment or combination of these of these variants and mutations, wherein the peptide sequence may be recombinantly expressed, either in procaryotes or eucaryotes, isolated from the environment, an organism, a SARS-CoV2-virus or the subject, or artificially synthesized, mutated and selected for increased affinity for its host receptor, may be glycosylated or non-glycosylated, as long as sACE2 exhibits a binding affinity thereto, preferably under the conditions used in step a) of assay (B). More preferably, the peptide sequence of the SARS-CoV-2 spike protein used as a bait is a naturally occurring variant of the SARS-CoV-2 spike protein or a mutated variant of the SARS-CoV-2 spike protein or fragment of these, as long as sACE2 exhibits a binding affinity thereto.

More preferably, the RBD is used as a bait. Even more preferably, the bait comprises any peptide sequence of the RBD, i.e. any naturally occurring variant of the RBD or any mutated variant of the naturally occurring variant of the RBD, like the RDB of the naturally occurring variants Wuhan, United Kingdom (UK; B1.1.7), South Africa (B1.351) or of the mutated variants comprising mutations TR, KN, YF, EK, QM, QH, NY and/or NF of SEQ ID NO:3 described in Table 2 below, wherein these mutations may be present alone or two or more of them may be present in any combination, or any fragment or combination of these variants and mutations, wherein the peptide sequence may be recombinantly expressed, either in procaryotes or eucaryotes, isolated from the environment, an organism, a RBD or the subject, or artificially synthesized, may be glycosylated or non-glycosylated, as long as sACE2 exhibits a binding affinity thereto, preferably under the conditions used in step a) of assay (B). More preferably, the peptide sequence of the RBD used as a bait is a naturally occurring variant of the RBD or a mutated variant of the RDB or any fragment of these, as long as sACE2 exhibits a binding affinity thereto. More preferably, the RBD used as a bait has at least 95 %, more preferably 98 %, even more preferably 99 % and most preferably 100 % sequence identity with the protein sequence of SEQ ID NO: 3 (Wuhan variant), SEQ ID NO: 4 (United Kingdom variant; B1.1.7), SEQ ID NO: 5 (South Africa variant; B1.351), mutated variants comprising mutations TR, KN, YF, EK, QM, QH, NY and/or NF of SEQ ID NO:3 described in Table 2 below, wherein these mutations may be present alone or two or more of them may be present in any combination, even more preferably with SEQ ID NO: 3, most preferably the RBD has 100 % sequence identity with the protein sequence of SEQ ID NO: 3.

Table 2: *Description of mutations in relation to SEQ ID NO:1, SEQ ID NO:2 and SEQ ID NO:3*

| Mutation name | SEQ ID NO:1 (Position of mutated AA*) | SEQ ID NO:2 (Position of mutated AA) | SEQ ID NO 3: (Position of mutated AA) | wild-type AA | mutated AA |
|---|---|---|---|---|---|
| TR | 385 | 375 | 67 | T | R |
| KN | 417 | 407 | 99 | K | N |
| YF | 453 | 443 | 135 | Y | F |
| EK | 484 | 474 | 166 | E | K |
| QM | 493 | 483 | 175 | Q | M |
| QH | 498 | 488 | 180 | Q | H |
| NY | 501 | 491 | 183 | N | Y |

(continued)

| Mutation name | SEQ ID NO:1 (Position of mutated AA*) | SEQ ID NO:2 (Position of mutated AA) | SEQ ID NO 3: (Position of mutated AA) | wild-type AA | mutated AA |
|---|---|---|---|---|---|
| NF | 501 | 491 | 183 | N | F |
| **AA: amino acid; the wild-type AA is replaced/mutated with the AA as indicated under mutated AA at the respective position indicated for SEQ ID NOs:1, 2 and 3, respectively.* | | | | | |

The RBD is preferred over the SARS-CoV-2 spike protein as the bait. Due to its shorter sequence, it may be easier to produce and provides fewer sequences parts to which proteins other than sACE2, like antibodies specific for such sequence parts, may have an affinity and may sterically prevent sACE2 binding to a certain extent or compete with sACE2 for binding. For instance, the RBD still has areas that do not bind to sACE2 like the RBM (receptor binding motif), which is distinguished from the entire RBD. Such other proteins other than sACE2 may then be co-purified in the pull-down assay, the sACE2 in the purified sample obtained in step b) may thereby contaminated and the accuracy of the assays deteriorate. Hence, it is advantageous to take an RBD variant that has a particularly high affinity, as this allows a better pull-down despite the presence of proteins other than sACE2.

As outlined in detail above, sACE2 masks the RBD of the spike of the SARS-CoV-2 virus preventing an efficient stimulation for a production of neutralizing antibodies against SARS-CoV-2. By using the SARS-CoV-2 spike protein or the RBD for the pull-down assay, all sACE2 capable of masking the RBD can be isolated specifically. This allows for an accurate determination of the total amount of sACE2 capable of binding the RBD and only variants of sACE2 are detected that are functionally effective in epitope masking and which are eventually crucial for prognosing the course of a SARS-CoV-2 infection and/or the response to a SARS-CoV-2 immunization, preferably SARS-CoV-2 vaccination, and/or SARS-CoV-2 natural infection. In case a protein other than the SARS-CoV-2 spike protein or RBD is used as ligand or bait, e.g. an antibody or a substrate for the enzymatic activity of sACE2, in the affinity purification, the significance of the assay may be reduced, as sACE2-variants may be isolated not capable of binding and masking the RBD, except e.g. monoclonal antibodies are used that overlap with the sACE2 epitopes and bind with even higher affinity than sACE2.

Preferably, in assay (B) in step b) the denaturing of the purified sample obtained in step a) to obtain a denatured sample is by adding a denaturing agent like SDS (Sodium dodecyl sulphate). A denaturing agent is capable of dissociating non-covalent bonds and denaturing of proteins.

Preferably, the denaturing is by adding SDS to a final concentration in the denatured sample of 1 % w/v or less, more preferably of 0.5 % w/v or less, even more preferably of 0.1 % w/v or less and a lower limit is preferably 0.001 % w/v, more preferably 0.01 % w/v. Hence, the concentration of the denaturing agent in the denatured sample may be optimized to concomitantly allow for a good elution of sACE2 and good detection of sACE2 in step c) by e.g. sACE2 specific antibodies. The denaturation with SDS is less preferred, since SDS is very corrosive to the technical equipment.

Preferably, in assay (B) in step b) the dissociating of non-covalent bonds in the purified sample obtained in step a) to obtain a non-covalent bonds-dissociated sample is by adding a non-covalent bonds-dissociating agent like a chaotropic agents known to the skilled person like sodium thiocyanate (NaSCN), glycine, diethylamine, guanidine or urea. A non-covalent bonds-dissociating agent is capable of dissociating non-covalent bonds but does not or essentially does not denature proteins. Preferably, the non-covalent bonds-dissociating agent is NaSCN or urea, most preferably NaSCN, since these agents are less corrosive for the technical.

Preferably, the dissociating of non-covalent bonds is by adding the non-covalent bonds-dissociating agent to a final concentration in the non-covalent bonds-dissociated sample of 3 M or less, more preferably 2.5 M or less, even more preferably 1.5 M or less, still even more preferably 1 M or less and most preferably 0.8 M or less. A lower limit for the final concentration of the non-covalent bonds-dissociating agent is preferably 0.01 M, more preferably 0.1 M.

In case of urea the final concentration is preferably 3 M or less, more preferably 2.5 M or less, even more preferably 1.5 M or less and a lower limit is preferably 0.01 M, more preferably 0.1 M.

In case of NaSCN the final concentration is preferably 1.5 M or less, more preferably 1 M or less, even more preferably 0.8 M or less and a lower limit is preferably 0.01 M, more preferably 0.1 M.

The interaction of sACE2 with RBD is stable in the indicated ranges for the non-covalent bonds-dissociating agent e.g. urea and NaSCN (sodium thiocyanate). Hence, sACE2 stays bound to e.g. the RBD, while low avidity interactions that can mask the recognition of sACE2 in e.g. serum, are dissolve in step a) of assay (B). Hence, the concentration of the non-covalent bonds-dissociating agent in the non-covalent bonds-dissociated sample may be optimized to concomitantly allow for a good binding of sACE2 to e.g. the RDB and a good detection of sACE2 in step c) by e.g. immunodetection using sACE2 specific antibodies, or e.g. mass spectrometry.

The dissociation of non-covalent bonds or denaturation may make the epitopes of both a-sACE2 and i-sACE2 accessible for a specific detection of sACE2 in step c) and eventually provides for an accurate determination of the amount of sACE2. Conventional assays do not take into account that not all sACE2 is freely available in the blood, e.g. due to being bound/inhibited by e.g. an anti-ACE2 autoantibody like in the case of i-sACE2. Hence, without dissociation of non-covalent bonds or denaturation, i-sACE2 will be detected poorly or not at all (c.f. Figs.5B-C). Proteins may be unfolded, non-covalent bonds are dissolved and any non-covalently bound proteins that may be present, e.g. anti-ACE2 autoantibody, dissociate making i-sACE2 accessible for detection, particularly making the epitope of i-sACE2 accessible for specific immunodetection by using e.g. an immunoassay. Hence, also from this point of view, the dissociation of non-covalent bonds is preferred over a denaturation as it may not fully denature proteins and thus better allows subsequent immunodetection by antibodies as the epitopes may maintain at least partially their natural conformational folding structure.

More preferably, in assay (B), the step b) of denaturing the purified sample obtained in step a) or dissociating of non-covalent bonds in the purified sample obtained in step a) to obtain a denatured sample or non-covalent bonds dissociated sample is a step b) of dissociating of non-covalent bonds in the purified sample obtained in step a) to obtain a non-covalent bonds dissociated sample.

Furthermore, it is pointed out that the dissociation of non-covalent bonds does not elute bound sACE2 from the ligand, preferably from the RBD, into the solution. Hence, sACE2 stays bound to e.g. the beads, and in step c) the amount of sACE2, while still being bound to e.g. the beads via the RDB, is determined in the non-covalent bonds-dissociated sample, by e.g. immunodetection using sACE2 specific antibodies, or e.g. mass spectrometry.

Hence, in case step b) is a step of dissociating of non-covalent bonds, preferably, step b) is performed after step a) and before step c). This allows to reveal high-affinity binding of RBD-to-sACE2 but dissolves any bound proteins that leave sACE2 inaccessible for subsequent detection.

In case of step b) is a step of denaturing, the bound sACE2 is eluted from the ligand, preferably from the RBD, into the solution. Hence, in step c) the amount of sACE2, which is the eluted sACE2, is determined in the denatured sample, by e.g. immunodetection using sACE2 specific antibodies.

In assay (B), step c) further enhances the accuracy of the assay (B). As already stated above, the affinity purification or the pull-down assay may co-purify proteins other than sACE2. For example, i-sACE2 is bound to the inhibiting anti-ACE2 autoantibody, constituting a considerable amount of protein.

Preferably, in assay (B) in step c) the determining of the amount of sACE2 is specific for sACE2, preferably by any method capable of specifically detecting and quantifying the amount of sACE2. Such method is preferably an immunoassay using sACE2 specific antibodies, preferably an ELISA, mass spectrometry, or Western blot, more preferably an ELISA.

In view of the fact that more than 97 % of the total sACE2 may be inactivated i-sACE2, an unspecific detection of the total amount of proteins like a coomassie-staining or adsorption measurement would also detect co-purified proteins like anti-ACE2 autoantibodies, leading to a poor significance of the assay as to the actual amount of sACE2 present. Hence, unspecific staining methods are less preferred.

Preferably, in assay (B) in step c), the denatured sample, comprises eluted sACE2, that is sACE2 not bound to a matrix. More preferably, in assay (B) step c) of determining the amount of the sACE2 in the denatured sample obtained in step b) is a step c) of determining the amount of the sACE2 in the non-covalent bonds-dissociated sample obtained in step b), and the non-covalent bonds-dissociated sample comprises sACE2 bound to a matrix, preferably, sACE2 bound to beads via the RBD immobilized on said beads.

Preferably, in assay (B) the amount of sACE2 determined in step c) is the amount of sACE2 capable of binding the SARS-CoV-2 spike protein, more preferably the amount of sACE2 capable of the RBD of the SARS-CoV-2 spike protein, in the non-covalent bonds-dissociated sample.

More preferably, in assay (B) the amount of sACE2 determined in step c) is the total amount of sACE2, even more preferably, the total amount of sACE2 capable of binding the SARS-CoV-2 spike protein, most preferably the total amount of sACE2 capable of binding the RBD of the SARS-CoV-2 spike protein, in the non-covalent bonds-dissociated sample.

The present invention further provides a kit of parts (A) for performing the assay (A) according to the invention, comprising:

- a substrate of a-sACE2, preferably a fluorescent substrate of a-sACE2;
- optionally a source of a divalent cation, preferably a source of $Zn^{2+}$, more preferably a divalent cation containing buffer, even more preferably a $Zn^{2+}$-containing buffer; and
- optionally a manual.

The present invention further provides a kit of parts (B) for performing the assay (B) according to the invention, comprising:

- a ligand, preferably a bait, according to the present invention, optionally immobilized on a matrix, preferably beads;
- optionally a denaturing agent, preferably a non-covalent bonds-dissociating agent; and

- optionally a manual.

The present invention further provides a kit of parts (C) for performing the *in-vitro* method according to the present invention, comprising:

- the Kit of parts (A) according to the present invention and/or the Kit of parts (B) according to the present invention; and
- optionally a manual.

The present invention further provides the use of the Kit of parts (C) for prognosing the course of a SARS-CoV-2 infection and/or the response to a SARS-CoV-2 immunization and/or natural infection of a SARS-CoV-2 infected or SARS-CoV-2 non-infected subject
The present invention further provides the use of the Kit of parts (C) for stratifying a SARS-CoV-2 infected or SARS-CoV-2 non-infected subject for the need of medical care and/or for the treatment with a SARS-CoV-2 immunization by using a-sACE2 and/or sACE2 as a marker.
The present invention further provides the use of a Kit of parts for prognosing the course of a SARS-CoV-2 infection and/or the response to a SARS-CoV-2 immunization and/or natural infection of a SARS-CoV-2 infected or SARS-CoV-2 non-infected subject by using a-sACE2 and/or sACE2 as a marker, comprising:

- means for determining an amount of a-sACE2 and/or sACE2 in a sample of the subject.

The present invention further provides the use of a Kit of parts for stratifying a SARS-CoV-2 infected or SARS-CoV-2 non-infected subject for the need of medical care and/or for the treatment with a SARS-CoV-2 immunization by using a-sACE2 and/or sACE2 as a marker, comprising

- means for determining an amount of a-sACE2 and/or sACE2 in a sample of the subject.

The present invention further provides the use of a-sACE2 and/or sACE2, preferably sACE2, as a marker for prognosing the course of a SARS-CoV-2-infection and/or the response to a SARS-CoV-2 immunization of a SARS-CoV-2 non-infected or a SARS-CoV-2 infected subject.
The present invention further provides a-sACE2 and/or sACE2, preferably sACE2, as a marker for prognosing the course of a SARS-CoV-2-infection and/or the response to a SARS-CoV-2 immunization of a SARS-CoV-2 non-infected or a SARS-CoV-2 infected subject.
In the present invention, it is to be understood that any embodiment disclosed for the *in-vitro-assay* (A) and/or the *in-vitro-assay* (B) and/or the *in-vitro*-method is/are mutually applicable to each other. Furthermore, the embodiments disclosed for the *in-vitro-assay* (A) and/or the *in-vitro-assay* (B) and/or the *in-vitro*-method are applicable for the kit of parts (A) for performing the *in-vitro-assay* (A) and/or for the kit of parts (B) for performing the *in-vitro-assay* (B) and/or for the kit of parts (C) for performing the *in-vitro* method and/or for the kit of parts for prognosing and/or for the kit of parts for stratifying.
The invention is further illustrated by the following figures and examples.

## Examples

### *Example 1:*

*a) sACE2 is capable of binding to SARS-CoV-2 spike protein*

SARS-CoV-2 RBD was immobilized on an ELISA plate by incubating 25 μl of 4 μg/ml in PBS solution in wells of a high-binding 96 well plate (Corning, CLS3690) for at least 8 hours at +4°C. After washing 3x with 100 μl/well PBS containing 0.05% of Tween-20 (PBST buffer) plates were blocked for 1h with 25 μl/well 1% BSA in PBS (w/v), followed by 3x washing with 100 μl/well PBST. Distinct Antibodies (FLU1, S309, HYA, HYB) and recombinant proteins (ACE2-hFcg1, sACE2) were added at indicated concentrations starting from 50 μg/ml in 1:3 dilutions and incubated for 1h. Without prior washing of the plates, biotinylated ACE2 (ACE2-bio) (**Fig**. **1A**) or biotinylated S309 (S309-bio) (**Fig**. **1B**) were added in 25ul of PBS 1% BSA at a concentration to achieve 70% of their maximum binding (EC70). During one hour of incubation at room temperature, the biotinylated proteins competed for binding to RBD with previously added antibodies and recombinant proteins. Afterwards, plates were washed 3x with 100 μl/well PBST and incubated with an alkaline phosphatase (AP)-coupled streptav-idin (Southern Biotech, SBA-7100-04) at a 1:500 dilution in PBS 1% BSA (w/v) to detect ACE2-bio and S309-bio molecules that were not prevented from binding to the RBD. Bicarbonate buffer with 4-Nitrophenyl phosphate disodium salt hexahydrate substrate (SIGMA, Cat No S0942-50TAB; 1 tablet per 20 ml buffer) was added (50 μl/well) and absorbance was measured at 405 nm after 30 min in a Cytation 5 device (BioTek).

ACE2-hFcg1: recombinant ACE2 fused to human IgG1-Fc portion; FLU1: human recombinant IgG antibody specific for influenza virus hemagglutinin; HYB: mouse hybridoma antibody specific for the SARS-CoV-2 spike protein; HYA: mouse hybridoma antibody specific for the ACE2 binding site of the SARS-CoV-2 spike-RBD. The hybridoma antibodies were generated following classical hybridoma protocols after immunizing mice with SARS-CoV-2 full spike proteins. The monoclonal hybrid cell lines were screened and selected for their reactivity to RBD applying the assay described in example 1b with the exception of using a secondary AP coupled antibody specific for mouse IgG (Southern Biotech, cat #SBA-1033-04). S309 is a human recombinant antibody recognizing an epitope of the SARS-CoV-2 RBD distinct from ACE2 and was produced as described in the original publications Piccoli et al. 2020 and Pinto et al. 2020 (Piccoli, L., Park, Y.-J., Tortorici, M.A., Czudnochowski, N., Walls, A.C., Beltramello, M., Silacci-Fregni, C., Pinto, D., Rosen, L.E., Bowen, J.E., Acton, O.J., Jaconi, S., Guarino, B., Minola, A., Zatta, F., Sprugasci, N., Bassi, J., Peter, A., De Marco, A., Nix, J.C., Mele, F., Jovic, S., Rodriguez, B.F., Gupta, S.V., Jin, F., Piumatti, G., Presti, Lo, G., Pellanda, A.F., Biggiogero, M., Tarkowski, M., Pizzuto, M.S., Cameroni, E., Havenar-Daughton, C., Smithey, M., Hong, D., Lepori, V., Albanese, E., Ceschi, A., Bernasconi, E., Elzi, L., Ferrari, P., Garzoni, C., Riva, A., Snell, G., Sallusto, F., Fink, K., Virgin, H.W., Lanzavecchia, A., Corti, D., Veesler, D., 2020. Mapping Neutralizing and Immunodominant Sites on the SARS-CoV-2 Spike Receptor-Binding Domain by Structure-Guided High-Resolution Serology. Cell 183, 1024-1042.e21. doi:10.1016/j.cell.2020.09.037; Pinto, D., Park, Y.-J., Beltramello, M., Walls, A.C., Tortorici, M.A., Bianchi, S., Jaconi, S., Culap, K., Zatta, F., De Marco, A., Peter, A., Guarino, B., Spreafico, R., Cameroni, E., Case, J.B., Chen, R.E., Havenar-Daughton, C., Snell, G., Telenti, A., Virgin, H.W., Lanzavecchia, A., Diamond, M.S., Fink, K., Veesler, D., Corti, D., 2020. Cross-neutralization of SARS-CoV-2 by a human monoclonal SARS-CoV antibody. Nature 583, 290-295. doi:10.1038/s41586-020-2349-y). ACE2-Fcg1 and FLU1 were produced following the same protocol. ACE2-bio and S309-bio were generated applying the EZ-link NHS-PEG Solid-Phase Biotinylation Kit (Thermo-Scientific #21450). The EC70 of ACE2-bio and S309-bio were determined by performing the RBD-IgG ELISA as described here without competing agents. The concentration of biotinylated protein at which 70% of maximum binding to RBD is achieved was assessed.

b) *sACE2 efficiently prevents binding of neutralizing antibodies to the RBD of the SARS-CoV-2 spike protein*

In **Fig. 1C,** a SARS-CoV-2 RBD ELISA was performed as described in example 1a with the following changes: after coating of RBD and washing, indicated serum dilutions of 3 COVID-19 patients (COVID-1, -2, -4) in PBS 1% BSA were added in the presence or absence of 100 ng/ml soluble recombinant ACE2 (rACE2-N'His). After washing, plates were developed with 25 μl/well 1:500 dilution of an anti-human IgG-alkaline phosphatase coupled agent (SouthernBiotech cat#2040-04) in PBS 1% BSA to detect human IgG that was not prevented by ACE2 from binding to the RBD. Plates were developed as described. The RBD-reactive antibody S309 (Example 1a) served as a control and is not to scale in the plot as a starting concentration of 1 μg/ml was diluted 1:5.

Collectively, the data of Fig. 1 show that sACE2 blocks human and murine neutralizing antibodies from binding to the RBD. Although distinct RBD epitopes are recognized by ACE2 and S309, more than 200 ng/ml sACE2 can partially block the binding of S309 at EC70 to the RBD, likely due to steric hindrance (**Fig. 1B**). Thus, sACE can prevent distinct neutralizing antibodies from binding to the RBD and would consequently shield recognition of the RBD by antibodies expressed on the surface of B cells.

***Example 2:*** *Optimization of a-sACE2 activity measurement*

Various factors were optimized to measure a-sACE2 activity by cleavage of the fluorescent substrate Mca-Ala-Pro-Lys(Dnp) (Mca = (7-methoxycoumarin-4-yl)acetyl; Dnp = (2,4-dinitrophenyl), Carbosynth Limited, #FM111000). The substrate quenches the Mca fluorescence until the Pro-Lys linker is cleaved specifically by a-sACE2.

*a) Determination of optimal measurement time points and number of measurements for determining the amount of a-sACE2*

The enzymatic activity of a-sACE2 was measured for 0.7 and 70 ng of a-sACE2 in PBS and a healthy donor plasma sample at multiple time points over more than 3h (**Fig. 2A**). The graph shows relative fluorescence units (RFU), determined as described in example 3a with the following variations: the measurements were performed at 37°C and the plate was kept inside the device for the entire time. The data show that RFUs decrease with each measurement. Thus, multiple readings hamper detection likely due to photobleaching, which is especially of high relevance when measuring a-sACE2 in samples with low a-sACE2 concentrations.

It was concluded that the method can be optimized through reduction of measurements to a maximum of 4 time points. 0h, 1h, 4h or 7h, and >18h, e.g. 20h were identified as optimal measurement time points to determine enzyme velocity and concentration to cover high (10-100 ng/ml; 1h), medium (4-10 ng/ml; 7h) or low (<1-4 ng/ml; >18h) a-sACE2 concentrations in the blood.

*b) Freezing of the sample does not affect the enzymatic activity of a-sACE2*

Plasma samples were either frozen at -80°C or stored at 4°C and a-sACE2 enzyme cleavage was measured as described in example 3a with the following variations: The plate was kept inside the device for 24 hours at 37°C, first two hours the F340/420 value was measured each 30 minutes, then the value was measured each hour. (**Fig. 2B**). Storage conditions did not affect the fluorescence measurement.

*c) Serum is the superior material for a-sACE2 activity measurements*

Serum and Plasma samples of COVID-19 patients donated at the same time point (COVID-1, -2, -3, -4) were analyzed for a-sACE2 substrate cleavage over time in technical duplicates as described in example 3a with the following variations: The plate was kept inside the device for 24 hours at 37°C, first two hours the F340/420 value was measured each 30 minutes, then the value was measured each hour (**Fig. 2C**).

Serum was identified to be the superior material as it shows higher activity and lower technical variability. Reaction velocity is calculated as the difference between the fluorescence at a given point, a zero point value divided by time passed (in hours). Furthermore, for reaction velocity calculations it was observed that a correction for intrinsic fluorescence measured at 0h is needed, likely because certain medications affect the fluorescence of blood samples.

*d) a-sACE2 activity measurements in dependency of a-sACE- concentration*

Substrate cleavage measured for distinct concentrations of recombinant a-sACE2 (rACE-N'His) in PBS over time in technical duplicates (**Fig. 2D**). The kinetics over 24h was performed in parallel with the frozen versus fresh experiment as described in example 2b and example 2c.

*e) Determination of optimal $Zn^{2+}$ concentration in serum and plasma for a-sACE2 enzymatic activity*

Serum samples (**Fig**. **2E, left panel**) and plasma samples (**Fig**. **2E, right panel**) of COVID-19 patients (COVID-1, -2, -3) and healthy donors (HD-A, -B, -C) donated at the same time point were analyzed for ACE2 enzymatic activity by substrate cleavage. Enzyme velocity (RFU/time) was determined as described in example 3a and is displayed in presence of different $Zn^{2+}$ concentrations. 10 ng recombinant ACE2 (recACE2) in PBS was used as a control.

It is concluded that ACE2 activity is low or absent in plasma of certain individuals while detectable in serum. Furthermore, in serum, ACE2-mediated cleavage was observed at a wider range of $Zn^{2+}$ concentrations between 0 - 100 μM, while in plasma enzymatic activity shows a narrow optimum around 100 μM $ZnCl_2$ that also varies between donors. Of note, previous plasma-based protocols used more than 3.5mM $ZnCl_2$ for which an inhibitory effect was observed.

***Example 3:***

*a) Enzymatic activity assays according to the present invention: the amount of a-sACE2 correlates with COVID-19 severity as determined by enzymatic activity assay for a-sACE2 by measuring the amount of fluorescence*

To address if a-sACE2 levels correlate with COVID-19 severity, sera of a prospective observational cohort of patients were studied with PCR confirmed SARS-CoV-2 infections. Severity ranged from a score in accordance with the WHO clinical progression scale of WHO3-5 to a score of WHO6+ (intensive medical care necessary) (**Fig. 3A**). Patient sera of each severity class and controls were analyzed by the enzymatic activity assays for a-sACE2 as described below. It was found that levels of a-sACE2 highly correlated with disease severity. The data in Figure 2E and F show that our methodology is suited to successfully measure a-sACE2 in plasma and serum, respectively.

Patient cohort description: The COVID-19 cohort had the following criteria: 66% male and 34% female with female donors of a mean age of 56.9 years (min 22; max 80 years) and a mean BMI of 29.9 (min 22; max 56) and male donors of a mean age of 64.5 years (min 22; max 81 years) and a mean BMI of 27.4 (min 16; max 36). Longitudinal blood samples were collected at distinct time points over a period of up to 250 days post symptom onset. A total of 50 donors is included in the analysis.

The healthy donor cohort is described in example 3b.

Measurements of a-sACE2 concentration in sera and plasma after full optimization according to the results of Examples 2A-F:

the reaction is performed by mixing 7.5 μl serum with 7.5 μl PBS-1% BSA or by using 15 μl plasma that was prepared by diluting blood with an equal volume of PBS 2mM EDTA to achieve 1:2 dilution. 15 μl sample is then mixed with 85 μl of the substrate solution that contains: the substrate (Mca-Ala-Pro-Lys(Dnp), Max Absorption/Emission upon cleavage = 325/393 nm) at 8 μM, 75 mM trisamino-methane adjusted to pH 6.5 with hydrochloride, 1 M sodium chloride, 100 μM zinc chloride, and freshly added protease inhibitors: 10 μM captopril, 10 μM bestatin hydrochloride, 10 μM Z-Pro-prolinal. All fluorescence measurements are performed using 340 nm excitation and 420 nm emission light filters (Cytation 5, BioTek). The first fluorescence measurement is performed immediately after the substrate addition. This initial measurement is a prerequisite to correct for the sample baseline fluorescence. The samples display wide variation in 400-450 nm absorbance, which influences the fluorescence reading and prevents correct calculation. This issue is addressed in the current protocol by measuring the 450 nm absorbance and calculating its influence on the fluorescence. The relation between 450 nm absorbance and 340/420 nm fluorescence was established by mixing different amount of the low-density fraction of human serum (lipids fraction) with PBS-BSA buffer containing various amount of the cleaved substrate. The RFU/[Mca] linear fit slope depends on the 450 nm absorbance logarithmically and was determined to be equal to 202.94 - 123.3 * log10(A450) by linear regression. The reaction is incubated at 37°C and can also be performed at room temperature. The second fluorescence reading is performed in 1 hour after the substrate addition. This measurement allows to calculate the a-sACE2 concentration in the samples with high level of the a-sACE2 (10->100 ng/ml). The third fluorescence measurement is performed in 7 hours after the substrate addition and is intended to assess the concentration of a-sACE2 in the samples with low level of a-sACE2 (4-10 ng/ml). A >20 hours measurement is performed for very low a-sACE2 concentrations (0.1-4 ng/ml). The number of fluorescence readings is minimized in the current protocol to avoid the bleaching effect - the decrease in fluorescence upon excitation with 340 nm light.

For data analysis, the amount of the substrate cleaved is calculated using the formula:

$$Substrate\ cleaved, pmol = \frac{F340/420\ (\text{second or following measurement}) - F340/420_0}{202.94 - 123.3 \times lg(A450)}$$

F340/420 fluorescence is measured at second or consecutive time points (340 nm excitation and 420 emission). $F340/420_0$ fluorescence is measured at the very first time point right after substrate addition. A450 is the absorbance of the sample at 450 nm. The a-sACE2 level is determined by interpolating the enzyme velocity (RFU/time) of the calibration curve in the linear range (initial enzyme velocity before fluorescence saturation) with recombinant ACE2 protein. To standardize the reaction rACE2-N'His was produced in our laboratory as described below and was standardized by comparing to the commercially available recombinant human ACE2 (Abcam, #ab151852) and another ACE2-Fcg1 that was generated by fusing the ACE2 ectodomain to the human Fc portion of IgG1 (rACE2-Fcg1).

As different standards can lead to distinct outcomes, standards need to be calibrated to the average a-sACE2 concentration of a healthy donor cohort of mean age of 30-50 years without comorbidities associated with COVID-19 as described in example 3b. The calibration samples contain recombinant ACE2 diluted to various levels from 0.5 to 100 ng/ml. In the conditions described above, the velocity of the reaction is constant for 0.5-100 ng/ml calibration samples over one hour, allowing to interpolate the calibration curve via linear regression with a zero point intercept. The slope coefficient of the fitted line is used to calculate the a-sACE2 activity in the sample using the following formula:

$$\text{ACE2, ng/ml} = \frac{\text{Substrate cleaved, pmol}}{\text{Linear Fit Slope, pmol} * \text{ml/ng}}$$

The calculated values are averaged between the replicates. The samples can be stored at room temperature for two weeks or at +4°C for several months without loss of activity. The buffer is stored at +4°C. Captopril (Sigma-Aldrich, #PHR1307), bestatin hydrochloride (Sigma-Aldrich, #B8385) and Z-Pro-prolinal (Sigma-Aldrich, #SML0205) are added just before usage. The stock solution of the substrate (4 mM) is prepared by diluting the lyophilized substrate with DMSO and is stored at -20°C.

*rACE2-N'His standard production:*

Q18-V739 fragment of human ACE2 was cloned into mammalian expression vector containing a signal peptide (SP) and a molecular tag composed of eight histidine moieties (His-tag) connected to the C-terminus of ACE2 via GSSGSSGSS linker. The vector was used to transfect HEK293 cells. It was determined experimentally that C-end His-tag is not suitable for efficient protein purification possibly due to the cleavage by proteases such as ADAM17 expressed by HEK293 cells. To address this issue, a His-tag was introduced between the SP and the N-terminus of ACE2 fragment. This allowed for the efficient expression and purification of the protein by His SpinTrap columns according to manufacturer's instructions (Cytiva, 95056-290). The eluted protein was transferred to phosphate-buffered saline (PBS) via buffer exchange using Amicon Ultra-4 centrifugal filter units with 50 kDa cutoff (Millipore, UFC805008). The concentration of the protein was estimated using the ELISA specific for His-tag which is performed according to the following procedure: different dilutions of the purified protein are prepared in PBS, distributed into half-area high-binding 96-well plate (Corning, CLS3690) and incubated for at least 8h at +4°C. The coated plate is washed 3 times with PBST buffer and incubated with 1 μg/ml Anti-His-tag antibody (Abcam, #ab18184) for an hour at room temperature. The stained plate is washed 3 times with PBST and incubated with 2 μg/ml Goat Anti-Mouse IgG Fc antibodies conjugated to alkaline phosphatase (Southern Biotech, cat #SBA-1033-04) for an hour at room temperature. The stained plate is washed 3 times with PBST and incubated with 4-nitrophenyl-phosphate disodium salt dissolved to 0.25 g/L in 0.05M sodium carbonate-bicarbonate buffer for 30 minutes. The absorbance at 405 nm is measured by Cytation5 device, the calculation performed using a commercially available His-tagged protein of a similar size as a standard. The correct size was confirmed by SDS-PAGE combined with Anti-His-tag Western Blot performed as followed: an aliquot of the purified ACE2 was loaded in a well of 4-15% Mini-PROTEAN(R) TGX Stain-Free(tm) Protein Gel (Bio-Rad, #4568083), size-separated and transferred to the Trans-Blot Turbo Midi Nitrocellulose membrane (Bio-Rad, #1704159) according to the manufacturer instruction. The membrane was blocked with 5% skimmed milk dissolved in a buffer containing 20 mM Tris, 150 mM NaCl and 0.1% Tween-20 (TBST buffer) for an hour at room temperature. The blocked membrane was stained with 1 μg/ml Anti-His antibodies (Abcam, #ab18184) for at least 8h at +4°C. The stained membrane was washed three times in TBST and stained with IRDye 800CW Donkey anti-mouse antibodies (Li-Cor Biosciences, #925-32212) for two hours at room temperature. The membrane was washed three times in TBST and scanned using the Li-Cor Odyssey infrared scanner. The enzymatic activity of the purified protein was standardized using the commercially available human recombinant ACE2 (Abcam, #ab151852).

*b) Amount of a-sACE2 in sera of healthy individuals as determined by enzymatic activity assay for a-sACE2 by measuring the amount of fluorescence*

Sera from 66 male and 78 female healthy donors were analyzed exactly as described in example 2a. Female donors had a mean age 46 years (min 23; max 83 years) and a mean BMI of 24.6 (min 17.4; max 37.9) (**Figs. 3B-E**). Male donors had a mean age of 48 years (min 20; max 86 years) and a mean BMI of 25.9 (min 20.7; max 36.9). For the described cohort a-sACE2 levels were measured showing a mean at 1.96 ng/ml for female (min 0.87; max 5.86 ng/ml) and a mean of 2.67 ng/ml for male (min 1.1; max 8.6 ng/ml). Plots in Fig 3B-E show BMI and age of male and female donors plotted against a-sACE2.

***Example 4***

*a) The cleaving activity of a-sACE2 is specific for its substrate; Determination of optimal $Zn^{2+}$ concentration for a-sACE2 enzymatic activity*

Different amounts of recombinant ACE2 were added to a healthy donor plasma sample (HD-1) in presence of different concentrations of $ZnCl_2$ and DX600, a specific inhibitor for ACE2 (**Fig. 4A**). Enzymatic activity of plasma samples was determined as described in Example 3a with the following changes: 15 μl donor plasma was distributed in the wells of high-binding half-area 96-well plate along with recombinant ACE2 (Bon-Opus #BP042) used as the standard, in dilutions ranging from 1 to 50 ng/ml prepared in PBS-BSA 1%. DX600 was added to the 15μl plasma sample to a final concentration of 1 or 10 μM. Samples were incubated with DX600 for an hour at 37°C. 85 μl of the substrate buffer containing 8 μM substrate and 5, 100 or 5000 μM of $ZnCl_2$ was mixed with the samples. The first 340/420 nm fluorescence and 450 nm absorbance reading was performed immediately after the substrate addition, the second reading was performed after 29 hours.

The data provide evidence that cleavage of Mca-Ala-Pro-Lys(Dnp) in plasma is mediated by ACE2, as the addition of the ACE2 specific inhibitor DX600 to plasma abrogates cleavage and fluorescence release.

*b) In healthy individuals a-sACE2 levels fluctuate only poorly over a period of one day*

The a-sACE2 concentrations were measured via the procedure described above in example 3a with the following changes: as the ACE2 concentration in these samples was expected to be low, the second measurement was performed at 21 hours, the third - at 45h, the fourth - at 50h. 45h time point was used for the final calculation. Recombinant ACE2 (Bon-Opus #BP042) was used as the standard. Two healthy donors (HD-A, -B) donated blood over one day at distinct time points (**Fig**. **4B**). Measurement was performed in technical duplicates.

The data indicate that a-sACE2 levels are stable over one day and do not drastically fluctuate, for instance upon nutrient uptake.

*c) In healthy individuals a-sACE2 levels fluctuate over a period of 1.5 years*

Analysis of plasma samples collected from the same healthy individual (HD-C) at different time points over a period of 1.5 years shows some degree of biological variation **(Fig. 4C).** The experiment was performed as described in example 4b.

***Example 5:***

*a) Commercial ELISA cannot detect sACE2 at the same high level as the affinity assay according to the present invention and sACE2 correlates less well with the enzymatic activity assays according to the present invention than with the affinity assay according to the present invention.*

A commercial sACE2 ELISA (SEB886Hu kit from the CloudClone) was applied following manufacturer instructions to determine the total sACE2 concentration in sera of 4 COVID-19 patients (COVID-1, -2, -3, -4) and 2 healthy donors (HD-B, -C) **(Fig. 5A).**

*b) Affinity assay according to the present invention: The amount of sACE2 in the blood may be considerably higher as determined via assays relying on detection of enzymatic activity and conventional ELISA*

sACE2 was pulled down from 500μl of distinct serum samples used in 5a. For concentrating sACE2, recombinant RBD was expressed by using the nucleotide sequence of SEQ ID NO:7, which contains the nucleotide sequence for the RBD of the Wuhan variant, codon-optimized for expression in human cells, and contains the genetic code for a TwinStrep tag. The recombinant RBD has the amino acid sequence SEQ ID NO:6 comprising the amino acid sequence SEQ ID NO:3 (RBD of Wuhan variant) and a TwinStrep tag (WSHPQFEKGGGSGGGSGG-SAWSHPQFEK) - a peptide sequence allowing the recombinant protein to bind to StrepTactin **(Fig. 5B).** 10 μg RBD was mixed with 100 μl of 5% magnetic beads suspension covalently coated with StrepTactin (Strep-Tactin®XT Cat# 2-4090-002 or -010 from IBA GmbH) after equilibration according to manufacturer protocol. The mixture was incubated at 37°C for 30 minutes on the orbital shaker set at 1400 rpm. The unbound RBD was removed by pelleting and resuspending the magnetic beads 5 times in 1 ml phosphate-buffered saline (PBS). The RBD-bound beads were resuspended in 500 μl serum and incubated at +4°C overnight with constant mixing. The beads were pelleted and washed 3 times with 1 ml PBS with 0.05% Tween-20. Pelleted beads are resuspended in 20-40 μl buffer containing 94 mM trisaminomethane adjusted to pH 6.8 with hydrochloride, 12.5% glycerol, 2.3% SDS and 0.005% bromphenol blue and incubated at 95°C for 30 minutes. Beads were pelleted, and the eluate was harvested. Different amounts of the eluate corresponding to indicated volumes of the serum were loaded into 4 wells of a 4-15% gradient polyacrylamide gel together with the Precision Plus Protein Dual Color Standard (Bio-Rad, #1610374). Proteins were transferred to a nitrocellulose membrane, blocked with 30 ml of 5% skimmed cow milk diluted in trisaminomethane-buffered saline with 0.1% Tween-20 (TBST) for an hour at room temperature on the orbital shaker. The membrane was stained in 5 ml goat anti-human ACE2 antibodies (R&D Systems, #AF933) diluted to 0.2 μg/ml at +4°C overnight with constant mixing on the roller. The stained membrane was washed 5 times with 30 ml of TBST and stained with donkey anti-goat IgG antibodies at 0.2 μg/ml (LI-COR Biosciences GmbH, #925-32214) conjugated to the Infrared fluorescent dye 700DX for an hour at room temperature with constant mixing on the roller. The membrane was washed 3 times with 30 ml TBST and scanned with an infrared membrane scanner (Li-Cor Odyssey IR scanner). The sACE2 concentration was determined by densitometry analysis with GelAnalyzer 19.1 (http://www.gelanalyzer.com/?i=1) using 60-170 kDa portion of the sample lanes and comparing the intensity to loading controls: 150, 50, 15, and 5 ng of recombinant human rACE2-N'His produced in our laboratory (ACE2 Q18-V739 with an N terminas His tag (no linker). Markers flanking the samples indicate molecular weight in kDa.

*c) Comparison of ACE2 concentrations obtained by different assays*

The table in **Fig. 5C** compares the sACE2 concentration obtained by commercial ELISA shown in in **Fig. 5A,** by enzymatic activity measurement of a-sACE2 according to Example 3a) and by densitometry analysis according to the affinity assay of sACE2 Example 4b) of the plots shown in **Fig. 5B.**

*d) Determination of optimal SDS detergent concentration*

An alternative to sACE2 concentration measurement by densitometry analysis after western blotting is to determine the amount of sACE2 in the eluate by ELISA **(Fig. 5D).** However, SDS can interfere with ELISA detection. To determine the influence of SDS on sACE2 concentration measurement by ELISA, recombinant 4 ng/μl ACE2 was coated in a buffer containing indicated percentages of SDS in PBS 1% BSA. After washing 3x with 100 μl TBST/well, blocking

for 1h with 25 µl/well 1% BSA in PBS (w/v), the plates were washed again 3x 100 µl TBST/well and incubated either with a goat polyclonal sACE2 specific antibody raised against the full length sACE2 protein at 200 ng/ml (R&D Systems, #AF933) in PBST 5% milkpowder (w/v), alternatively a rabbit polyclonal sACE2 specific antibody raised against-N-terminus region of ACE2 (Bio-Rad Laboratories GmbH, #AHP888, 1 µg/ml or NSJ Bioreagents, # 49433, 2 µg/ml) can be used. Finally, plates were washed 3x with 100 µl/well PBST and incubated with AP coupled secondary antibodies (Dianova, #705-056-147, Jackson Immuno Research, #111-056-003) specific for goat and rabbit IgG, respectively. Bicarbonate buffer with 4-Nitrophenyl phosphate disodium salt hexahydrate substrate (SIGMA, Cat No S0942-50TAB; 1 tablet per 20ml buffer) was added (50 µl/well) and absorbance was measured at 405 nm after 30 min in a Cytation 5 device (BioTEk).

The data show that ACE2 can be properly determined at SDS <0.1%.

Indicated amounts of rACE2-N'His were coated to ELISA plates in presence of 0.1, 0.05, and 0.025 % of SDS **(Fig. 5E).** The ELISA was developed with anti-ACE2 and secondary antibodies as described in **Fig. 5D**). Down to 1-3 µg/ml ACE2 can be detected in presence of 0.1% or preferably lower percentages of SDS.

*e) Pearson correlations of ACE2 concentrations*

Pearson correlations of ACE2 concentration measurements by enzymatic activity measurement of a-sACE2 according to Example 3a, densitometry analysis of sACE2 according to the affinity assay of Example 5b and a commercial ELISA for 4 COVID-19 patients (COVID-1, -2, -3, -4) and 2 healthy donors (HD-B, -C) of Example 5a. Pearson correlation was performed with a 95% confidence interval, Pearson r values and p values are indicated **(Fig. 5F).**

The data show that the enzymatic assay for a-sACE2 according to Example 3a and the affinity assay for sACE2 according to Example 5b show a better correlation compared to the commercial ELISA and Assay B. Furthermore, although the commercial ELISA is meant to detect total sACE2 concentrations in the blood, less than 100 ng/ml were determined. Instead, Assay B detected as much as 2.2 µg/ml suggesting that it is suited to measure total sACE2 protein in the serum.

***Example 6:*** *Determination of optimal concentrations of non-covalent bonds-dissociating agents*

The ELISA plates were coated 4 µg/ml SARS-CoV-2 RBD **(Figs. 6A** and **6B)** or the influenza vaccine Influvac Tetra 19/20 **(Figs. 6C** and **6D)** in PBS for a minimum of 8h at +4°C. After washing 3x with 100 µl/well PBST buffer plates were blocked for 1h with 25 µl/well 1% BSA in PBS (w/v), followed by 3x washing with 100 µl/well PBST. Afterwards, serum and antibodies were added to plates at indicated dilutions in PBS 1% BSA and incubated for 1h at room temperature. Sera (HD-C, COVID-5, 6), SARS-CoV-2 reactive antibodies (S309, S2H13), influenza reactive antibodies (FLU1, FLU2) and recombinant ACE2 fused to the human Fc of IgG1 (ACE2-Fcg1) were used at dilutions or concentrations to achieve 50% of maximal IgG binding (ED50 for sera; EC50 for recombinant antibodies/proteins) specific for either SARS-CoV-2 RBD or Influvac Tetra 19/20. Recombinant antibodies were produced as described in example 1a. The SARS-CoV-2 negative healthy donor control serum (HD-C) in the RBD ELISA was used at the same dilution as COVID-5. After washing 3x with 100 µl/well PBST buffer, indicated amounts of NaSCN and Urea were added in PBS and incubated for 20min at room temperature. After washing 3x with 100 µl/well PBST buffer, the wells were incubated with anti-human IgG coupled to AP at 1:500 dilution in PBS 1% BSA (Cat#2040-04 from SouthernBiotech) for 1h at room temperature. The ELISA was developed and analysed as described in example 1a.

The data show that at EC50 the interaction of ACE2 with RBD is stable in the range of 0-2 M Urea and 0-1 M NaSCN. This range of detergents at maximum mildly affects binding of RBD and influvac specific IgG, and may therefore dissolve low avidity interactions that can mask the recognition of ACE2 in serum after pull down in the affinity assays according to Example 5b Therefore, these non-covalent bonds-dissociating agents (NaSCN; Urea) may be used as an alternative to SDS denaturation, to enable ACE2 to stay bound to the beads and to be detected by ACE2 specific antibodies.

***Example 7:***

*a) Low auto-ACE2-antibody avidity and concentration in COVID-19 patients*

It is possible that complexes of the viral spike with sACE2 drive autoimmunity during infection **(Fig. 7A**). Therefore, sACE2 reactive antibodies in patient sera were measured. 4 µg/ml recombinant rACE2-N-His were coated to ELISA plates in PBS for a minimum of 8h at +4°C. After washing 3x with 100 µl/well PBST buffer plates were blocked for 1h with 25 µl/well 1% BSA in PBS (w/v), followed by 3x washing with 100 µl/well PBST. Afterwards, different dilutions of patient sera (25 µl/well) in PBS 1% BSA were added and incubated for 1h at room temperature. After washing 3x with 100 µl/well PBST buffer, a detection reagent, AP-coupled goat anti-human IgG (Cat#2040-04 from SouthernBiotech) in PBS 1% BSA was added (25 μl/well) for 1h. The ELISA was developed and analysed as described in example 1a. SARS-CoV-2 RBD fused to the Fc portion of human IgG1 (RBD-Fcg1) at distinct concentrations served as a reference to calculate a proxy for the amount of ACE2 autoantibodies in serum that corresponds to the RBD-Fcg1 Ace2 interaction.

Overall anti-ACE2 IgG binding titers were poor, which likely reflects low auto-antibody avidity and concentration. However, ACE2 auto-antibody titers significantly correlated with COVID-19 severity.

*b) Polyclonal anti-ACE2 autoantibody interferes with enzymatic activity of a-sACE2*

By binding to the protein, autoantibodies could interfere with the enzymatic activity of a-sACE2 through inhibition of substrate binding or induction of protein conformational changes **(Fig. 7B).** Therefore, it was tested if the addition of distinct concentrations of a polyclonal ACE2 specific antibody (R&D Systems, #AF933) changed assay (A) outcomes.

If the ACE2 autoantibody was present at concentrations higher than 5 μg/ml, the enzymatic activity measurement performed according to the enzymatic activity assay of example 3a) is decreased. Importantly, ACE2 autoantibodies have the potential to interfere with enzymatic activity measurement and bias the results of assay (A). Therefore, if an individual shows high titers of ACE2 specific autoantibodies, assay (B) should be preferred over assay (A) to determine soluble ACE2 levels.

***Example 8:*** *In patients with high sACE2 levels, the RBD specific IgG titer and the potential of antibodies to block ACE2 binding to RBD is significantly reduced.*

*a) Serum levels of a-sACE2 and RBD specific IgG responses in COVID-19 patients*

To address if a-sACE2 levels in COVID-19 patients correlate with poor antibody responses to the RBD, a-sACE according to the example 3a were measured in controls and COVID-19 patient sera with a score of WHO3-6+ in accordance with the WHO clinical progression scale 12-50 days post-symptom onset. Furthermore, an RBD specific ELISA was determined as described in example 1c and the serum dilution with 50% of maximum IgG binding to RBD was determined for each sample (ED50). The data show **(Fig. 8A** and Fig. **8B** [enlarged section of Fig. 8A]) that individuals with high a-sACE2 concentrations have poor anti-RBD specific IgG responses, while some individuals with low a-sACE2 levels can develop very strong anti-RBD IgG antibody responses.

*b) Sera with a-sACE2 levels above 20 ng/ml show significantly decreased blocking potential of ACE2 binding to RBD and poor RBD specific antibody responses*

To further address if increased a-sACE2 levels in COVID-19 patients correlate with decreased a-sACE2 blocking antibody titers **(Fig. 8C),** serum dilution that block 50% of a-sACE2 binding (BD50) were calculated after applying the assay according to example 1a.

In **Fig. 8C** three groups of COVID-19 patients are shown distinguished by their level of a-sACE2 which was measured according to example 3a. The group on the left displayed a-sACE2 serum concentrations of <10 ng/ml, the group shown in the middle displayed a-sACE2 serum concentrations of 10-20 ng/ml, and the group on the right displayed a-sACE2 serum concentrations of >20 ng/ml. COVID-19 patients of the cohort described in example 3a with a score of WHO6+ between 12-50 days post symptom onset were included. Interestingly, a decreased potential of sera was observed to interfere with binding of the RBD to sACE2 if a-sACE2 concentration was higher than 20ng/ml.

The data provided in example 8 provide evidence that increased concentrations of a-sACE2 correlate with poor antibody responses to the RBD and, consequently, a decreased potential to block binding of the viral spike to the ACE2 host receptor. Thus, masking of the spike RBM by sACE2 is likely to interfere with neutralizing antibody responses.

***Example 9:*** *In two patients with high sACE2 serum levels, RBD specific IgG titers and the potential of antibodies to block ACE2 binding to RBD are inversely correlated with a-sACE2 early during the disease.*

Serum samples of 4 COVID-19 donors with high (donor 113 and 85, **Fig 9 A-B**) and low (donor 98 and 87, **Fig9 C-D**) a-sACE2 concentrations were compared for their a-sACE2 levels (measured as described in example 3a), the dilutions to achieve 50% of maximal IgG binding (ED50) specific for SARS-CoV-2 RBD (determined as described in example 1a and example 6) and the serum dilution that blocked 50% of ACE2 binding (BD50) to the RBD (BD50 was calculated after performing the assay as described in example 1a).

The data show that in two patients with high a-sACE2 levels, but not in two patients with low a-sACE2, the rise of a-sACE2 post symptom onset inversely correlates with RBD-specific IgG titers and the potential to block ACE2 binding to RBD.

## Claims

1. *In-vitro*-Assay (A) for determining an amount of a-sACE2 in a sample, comprising the steps of:

   a) preparing a mixture comprising the sample and a substrate of a-sACE2; and
   b) determining the amount of a-sACE2 comprising measuring an amount of a reacted and/or unreacted substrate and/or an amount of fluorescence in the mixture,

wherein the mixture further comprises a divalent cation and/or
wherein step b) is conducted in total 1 to 10 times within 45 h on the mixture obtained in step a).

**2.** Assay (A) according to claim 1, wherein the divalent cation is $Zn^{2+}$, wherein preferably the mixture comprises $Zn^{2+}$ at a final concentration of $Zn^{2+}$ from 0.001 mM to less than 3.5 mM, preferably from 0.01 mM to 2 mM, more preferably from 0.1 mM to 1 mM.

**3.** Assay (A) according to any one of the preceding claims, wherein step b) is conducted in total 1 to 8 times, more preferably 2 to 6 times and most preferably 3 to 4 times within 30h, more preferably within 25h, most preferably within 21h after step a),
preferably, wherein step b) is conducted at least once at each time point $t_1$=0h to <0.5h; $t_2$=0.5h to <2h; $t_3$=2h to <10h; and/or $t_4$=10h to 30h after step a).

**4.** Assay (A) according to any one of the preceding claims, wherein no solid carrier is used and/or the mixture is free or substantially free of heparin and/or free of a chelating agent.

**5.** *In-vitro*-Assay (B) for determining an amount of sACE2 in a sample, comprising the steps of:

a) purifying the sACE2, wherein the purifying comprises performing of an affinity purification, to obtain a purified sample;
b) denaturing the purified sample obtained in step a) or dissociating of non-covalent bonds in the purified sample obtained in step a) to obtain a denatured sample or non-covalent bonds dissociated sample; and
c) determining the amount of the sACE2 in the denatured sample or non-covalent bonds dissociated sample obtained in step b).

**6.** Assay (B) according to claim 5, wherein the affinity purification is a method based on protein interaction partners of the type analyte-ligand, wherein the ligand may be any molecule to which sACE2 exhibits an affinity and the analyte is sACE2, more preferably, the affinity purification is a pull-down assay of the bait and prey type, even more preferably the pull-down assay is of the bait and prey type, wherein the bait is any molecule to which sACE2 exhibits an affinity, preferably a SARS-CoV-2 spike protein, preferably a RBD (receptor binding domain) of the SARS-CoV-2 spike protein, and the prey is sACE2.

**7.** Assay (B) according to any one of claims 5 or 6, wherein step b) is a step b) of dissociating of non-covalent bonds in the purified sample obtained in step a) to obtain a non-covalent bonds-dissociated sample in step b), preferably, wherein the dissociation of non-covalent bonds is by adding a non-covalent bonds-dissociating agent like sodium thiocyanate, glycine, diethylamine, guanidine or urea, more preferably
by adding the non-covalent bonds-dissociating to a final concentration in the non-covalent bonds-dissociated sample of 3 M or less, more preferably 2.5 M or less, even more preferably 1.5 M or less, still even more preferably 1 M or less and most preferably 0.8 M or less.

**8.** *In-vitro*-Method for prognosing the course of a SARS-CoV-2-infection and/or the response to a SARS-CoV-2 immunization and/or SARS-CoV-2 natural infection of a SARS-CoV-2 non-infected or a SARS-CoV-2 infected subject by using a-sACE2 and/or sACE2 as a marker comprising the step of:

a) determining an amount of a-sACE2 and/or sACE2 in a sample of the subject.

**9.** Method according to claim 8, wherein the amount of sACE2 is the amount of sACE2 capable of binding the SARS-CoV-2 spike protein, more preferably the amount of sACE2 capable of binding the RBD of the SARS-CoV-2 spike protein.

**10.** Method according to claim 8 or 9, wherein in step a) the amount of a-sACE2 and/or sACE2 is determined by performing a method comprising:

electrochemoluminescence, acridinium ester based chemoluminescence, paramagnetic particles as solid phase like magnetic beads coated actively, i.e. covalent, or passively, i.e. non-covalent, with a ligand or bait of a-sACE2 and/or sACE2 by using the Strep-tactinXT/Twin strep or Biotin/Streptavidin system;

mass spectroscopy (MS), especially liquid chromatography tandem mass spectrometry (LC-MS-MS), using an enzymatic digestion step like tryptic digestion of a sample containing sACE2 or a-sACE2 in combination with separation and quantification of sACE specific fragments or of a sACE specific fragment by mass spectroscopy and comparison to a calibrator or calibrators of known sACE2 content;
enzymatic activity measurement of a-sACE2 e.g. after affinity purification, ion exchange chromatography, semiquantitative or quantitative measurement of the amount of sACE2-mRNA in the sample, e.g. by polymerase chain reaction (PCR)-based methods such as quantitative PCR like RT-PCR, or cycle threshold, ELISA-based systems, Reverse Phase HPLC, wherein the ELISA-based system comprise the measurement of a-sACE2 and/or sACE2 in a blood sample, like directly coating of the blood sample on an ELISA plate, washing with suitable buffer and detection of enzymatic activity by a measurable fragment released during cleavage, using e.g. a dye, fluorescence and/or mass spectrometry, and finally comparison with a sample running at the cutoff or in a known position to the cutoff; and/or
the *in-vitro* assay (A) according to claims 1 to 4 and/or the *in-vitro* assay (B) according to claims 5 to 7,

preferably the amount of sACE2 is determined by performing the *in-vitro* assay (B) according to claims 5 to 7.

**11.** Method according to any one of claims 8 to 10, further comprising the step of:
b) comparing the amount of a-sACE2 and/or sACE2 determined in step a) with an a-sACE2 reference amount and/or a sACE2 reference amount, respectively,
preferably, wherein
an amount of a-sACE2 and/or sACE2 higher than the a-sACE2 reference amount and/or the sACE2 reference amount is indicative for the SARS-CoV-2 non-infected subject or SARS-CoV-2 infected subject to develop a course of a SARS-CoV-2-infection with a score of WHO3 or higher in accordance with the World Health Organization clinical progression scale and/or being a non- or poor responder to a SARS-CoV-2 immunization and/or SARS-CoV-2 natural infection.

**12.** Method according to claim 11, wherein the amount of a-sACE2 and/or sACE2 determined in step b) is at least 1.2 fold higher than the a-sACE2 reference amount and/or a sACE2 reference amount, respectively.

**13.** Kit of parts (A) for performing the assay (A) according to claims 1 to 4, comprising:

- a substrate of a-sACE2, preferably a fluorescent substrate of a-sACE2;
- optionally a source of a divalent cation; and
- optionally a manual.

**14.** Kit of parts (B) for performing the assay (B) according to claims 5 to 7, comprising:

- a ligand, preferably a bait, according to claim 6, optionally immobilized on a matrix;
- optionally a denaturing agent, preferably a non-covalent bonds-dissociating agent; and
- optionally a manual.

**15.** Kit of parts (C) for performing the *in-vitro* method according to claims 8 to 12, comprising:

- the Kit of parts (A) according to claims 1 to 4 and/or the Kit of parts (B) according to claims 5 to 7; and
- optionally a manual.

**16.** a-sACE2 and/or sACE2, preferably sACE2, as a marker for prognosing the course of a SARS-CoV-2-infection and/or the response to a SARS-CoV-2 immunization and/or SARS-CoV-2 natural infection of a SARS-CoV-2 non-infected or a SARS-CoV-2 infected subject.

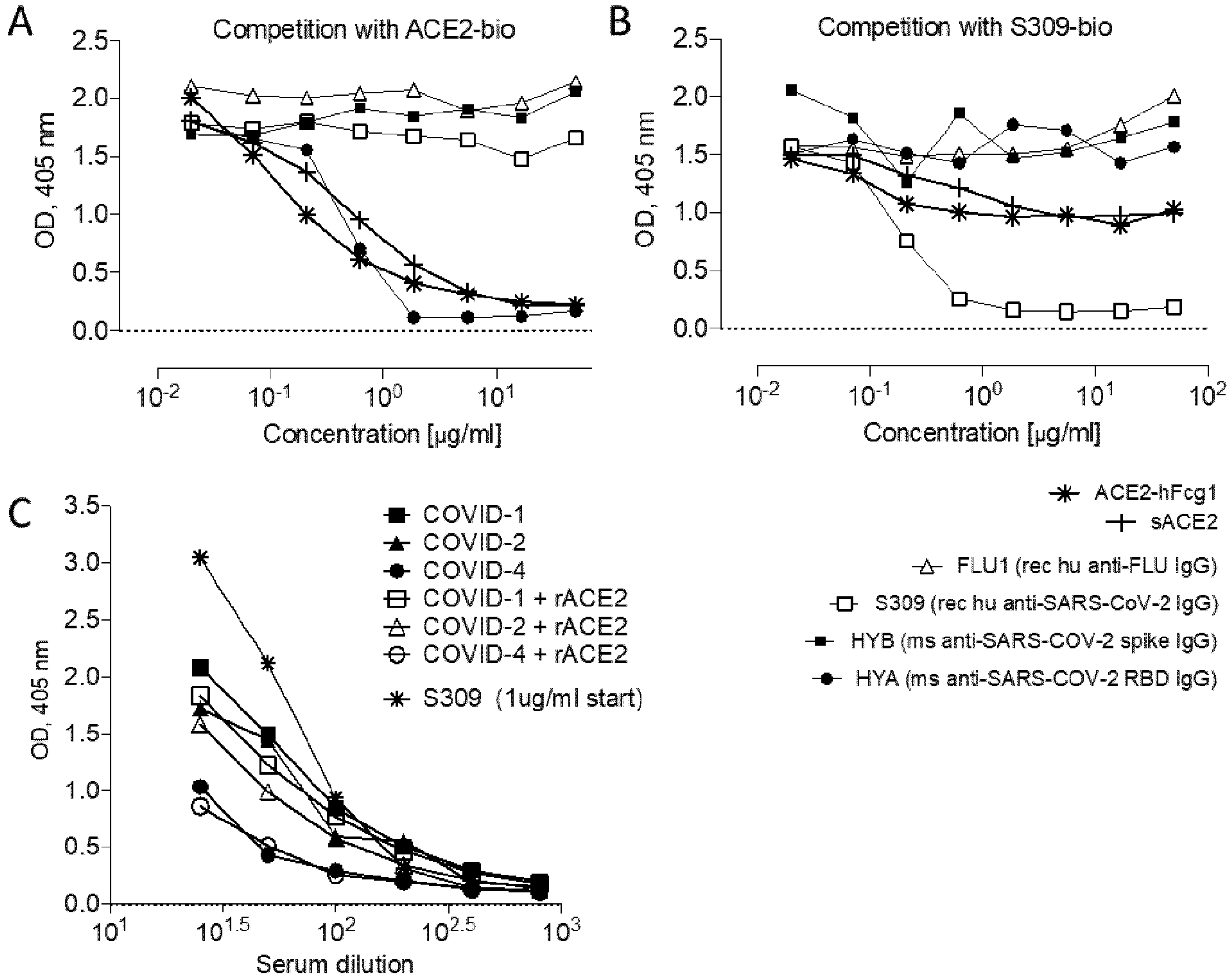
A
Competition with ACE2-bio
OD, 405 nm
Concentration [µg/ml]
B
Competition with S309-bio
OD, 405 nm
Concentration [µg/ml]
C
OD, 405 nm
Serum dilution
COVID-1
COVID-2
COVID-4
COVID-1 + rACE2
COVID-2 + rACE2
COVID-4 + rACE2
S309 (1ug/ml start)
ACE2-hFcg1
sACE2
FLU1 (rec hu anti-FLU IgG)
S309 (rec hu anti-SARS-CoV-2 IgG)
HYB (ms anti-SARS-COV-2 spike IgG)
HYA (ms anti-SARS-COV-2 RBD IgG)

Fig. 1A-C

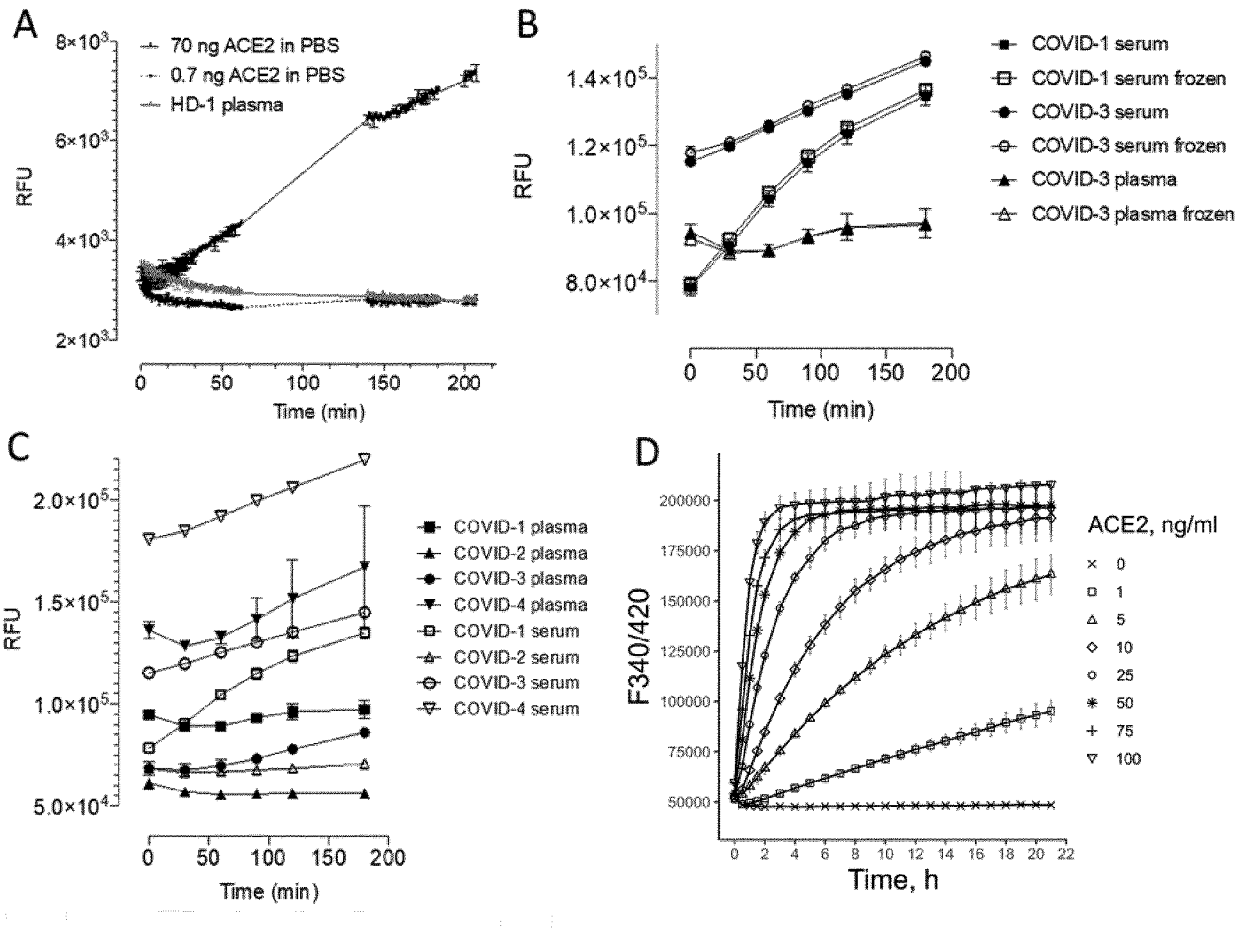
A
8×10³
6×10³
4×10³
2×10³
RFU
70 ng ACE2 in PBS
0.7 ng ACE2 in PBS
HD-1 plasma
0
50
100
150
200
Time (min)
B
1.4×10⁵
1.2×10⁵
1.0×10⁵
8.0×10⁴
RFU
COVID-1 serum
COVID-1 serum frozen
COVID-3 serum
COVID-3 serum frozen
COVID-3 plasma
COVID-3 plasma frozen
0
50
100
150
200
Time (min)
C
2.0×10⁵
1.5×10⁵
1.0×10⁵
5.0×10⁴
RFU
COVID-1 plasma
COVID-2 plasma
COVID-3 plasma
COVID-4 plasma
COVID-1 serum
COVID-2 serum
COVID-3 serum
COVID-4 serum
0
50
100
150
200
Time (min)
D
200000
175000
150000
125000
100000
75000
50000
F340/420
ACE2, ng/ml
0
1
5
10
25
50
75
100
0
2
4
6
8
10
12
14
16
18
20
22
Time, h

Fig. 2A-D

E

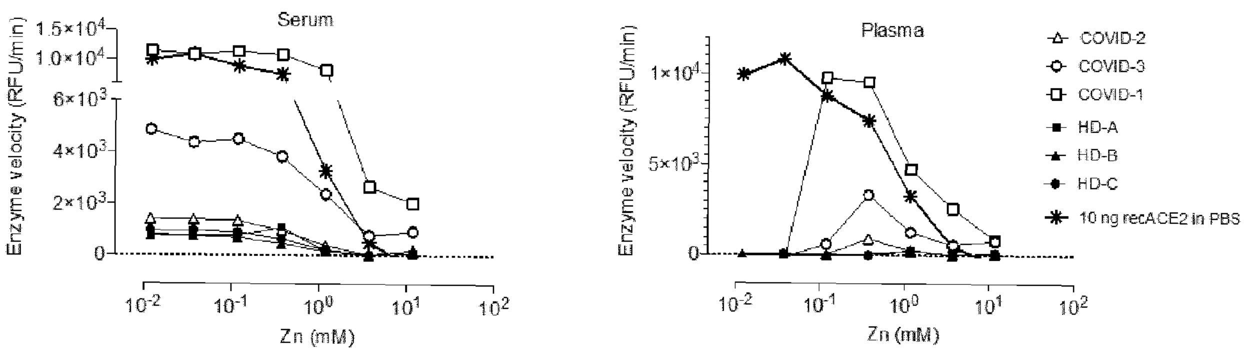
Serum
Enzyme velocity (RFU/min)
1.5×10⁴
1.0×10⁴
6×10³
4×10³
2×10³
0
10-2
10-1
10⁰
10¹
10²
Zn (mM)
Plasma
1×10⁴
5×10³
COVID-2
COVID-3
COVID-1
HD-A
HD-B
HD-C
10 ng recACE2 in PBS

Fig. 2E

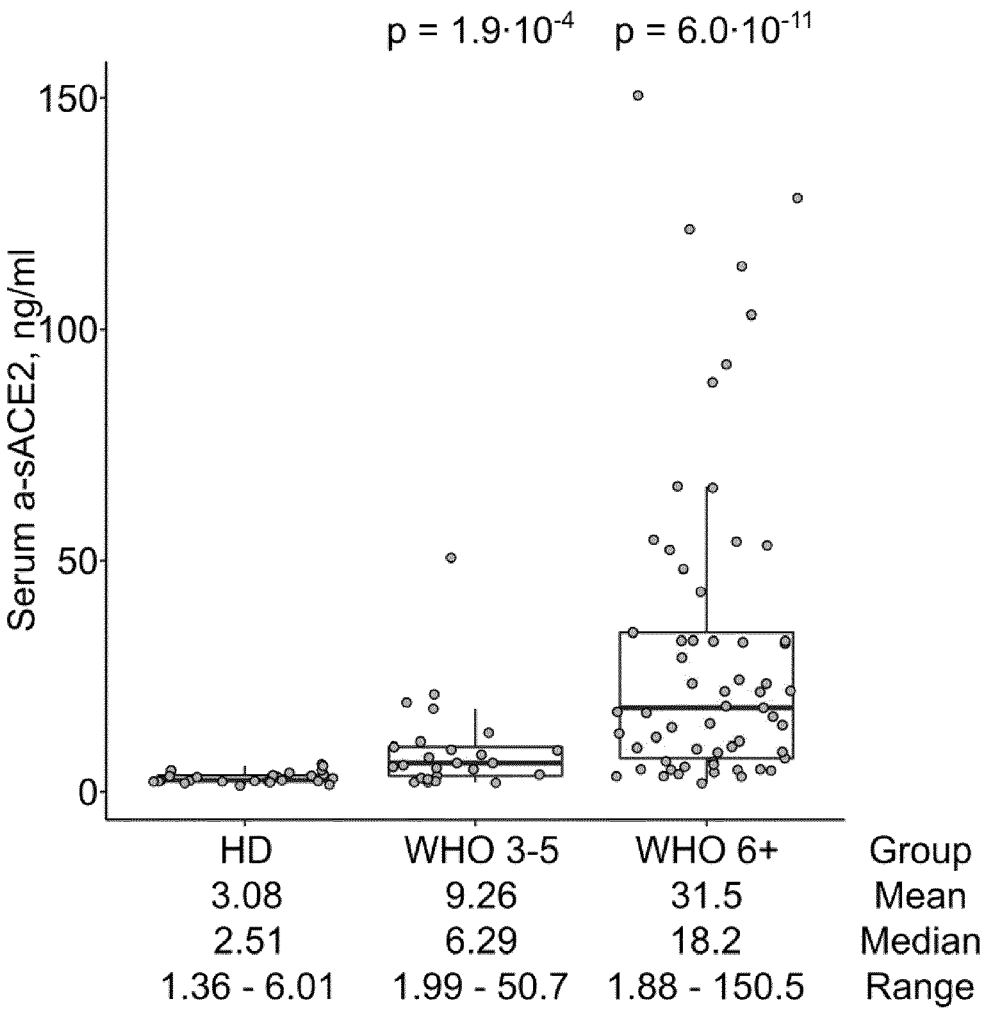
p = 1.9·10-4
p = 6.0·10-11
Serum a-sACE2, ng/ml
150
100
50
0
HD
WHO 3-5
WHO 6+
Group
3.08
9.26
31.5
Mean
2.51
6.29
18.2
Median
1.36 - 6.01
1.99 - 50.7
1.88 - 150.5
Range

Fig. 3A

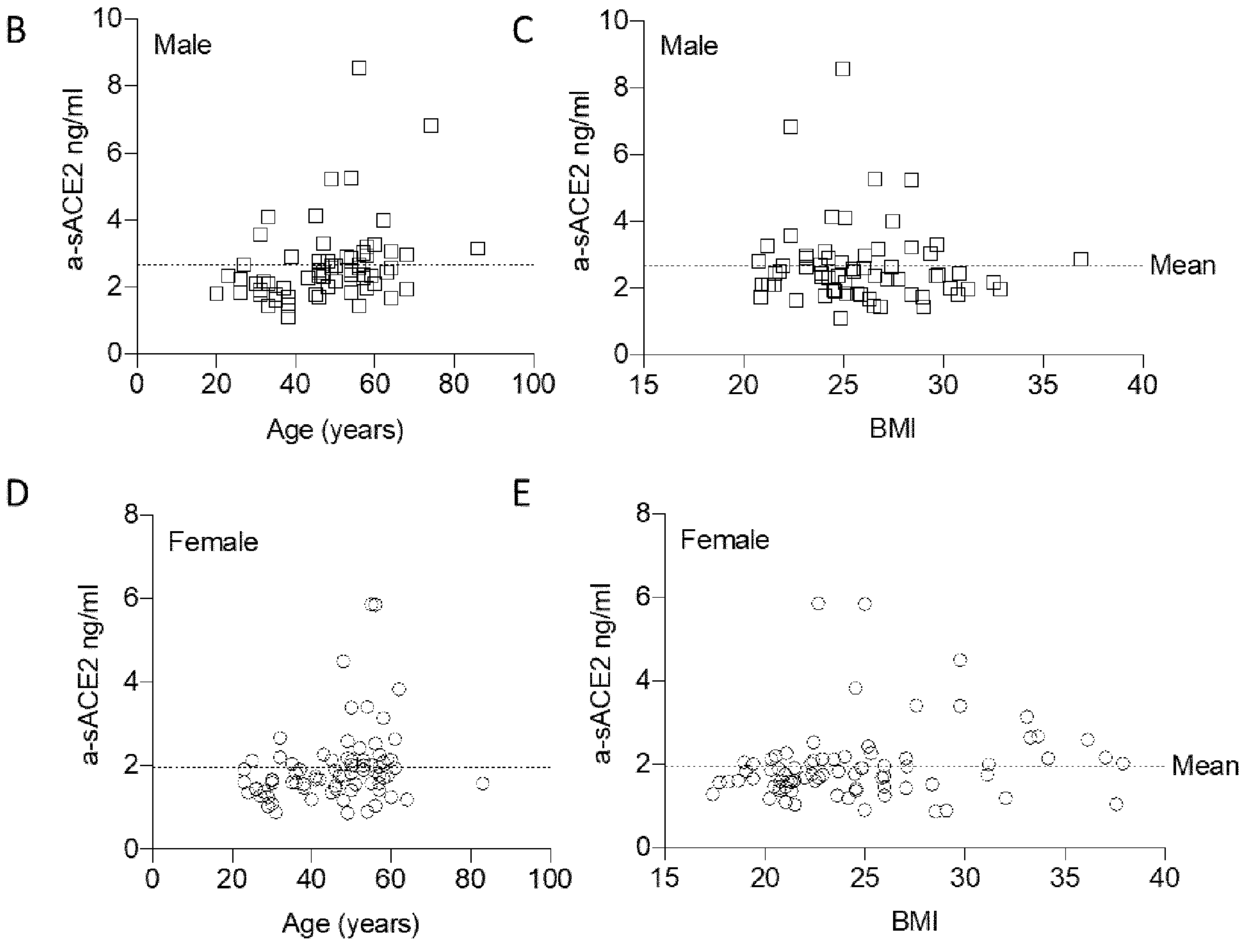
B
Male
a-sACE2 ng/ml
Age (years)
C
Male
Mean
a-sACE2 ng/ml
BMI
D
Female
a-sACE2 ng/ml
Age (years)
E
Female
Mean
a-sACE2 ng/ml
BMI

Fig. 3B-E

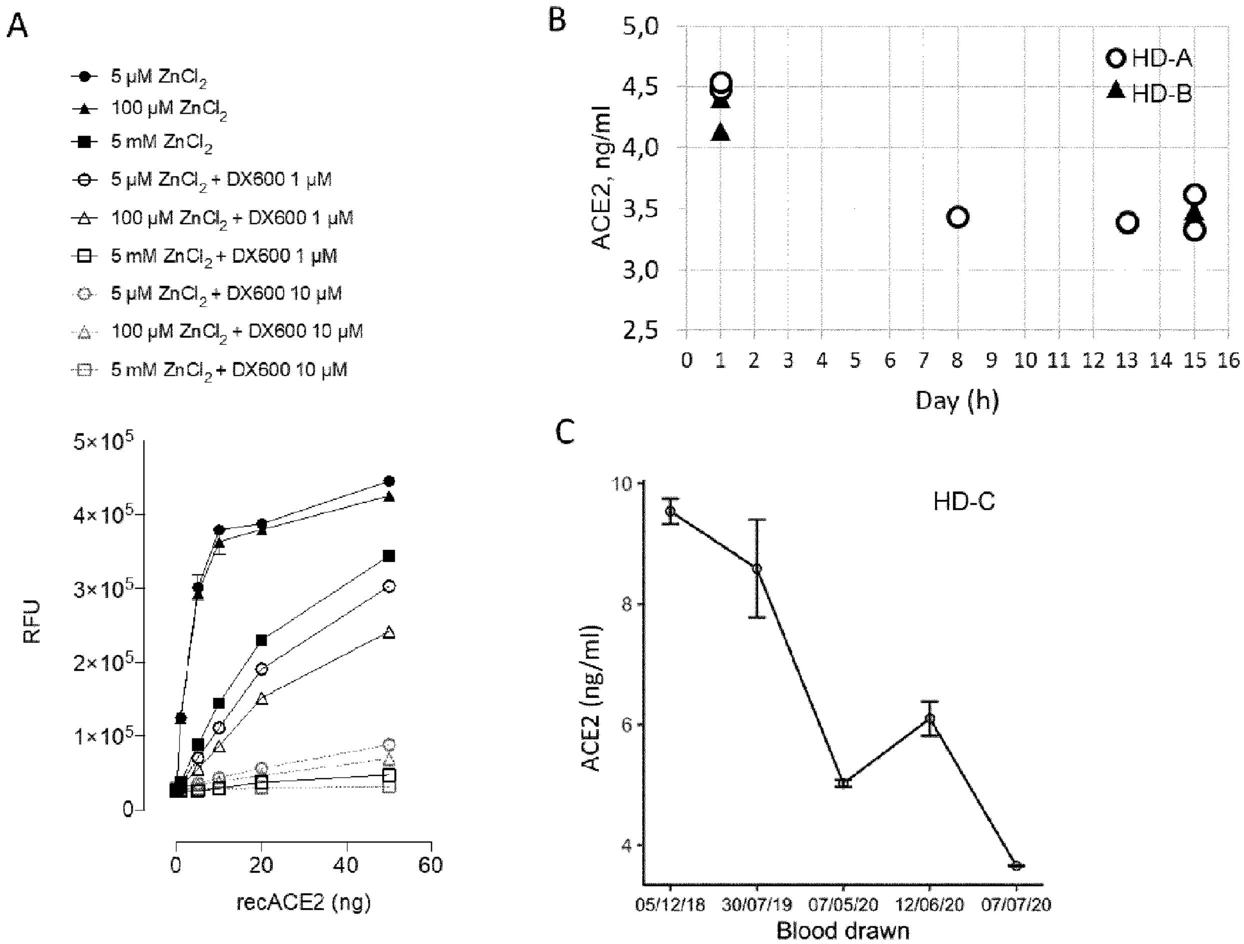
A
5 µM $ZnCl_2$
100 µM $ZnCl_2$
5 mM $ZnCl_2$
5 µM $ZnCl_2$ + DX600 1 µM
100 µM $ZnCl_2$ + DX600 1 µM
5 mM $ZnCl_2$ + DX600 1 µM
5 µM $ZnCl_2$ + DX600 10 µM
100 µM $ZnCl_2$ + DX600 10 µM
5 mM $ZnCl_2$ + DX600 10 µM
RFU
recACE2 (ng)
B
ACE2, ng/ml
Day (h)
HD-A
HD-B
C
HD-C
ACE2 (ng/ml)
05/12/18
30/07/19
07/05/20
12/06/20
07/07/20
Blood drawn

Fig. 4A-C

A

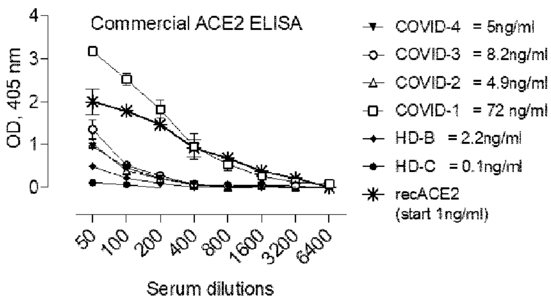


B

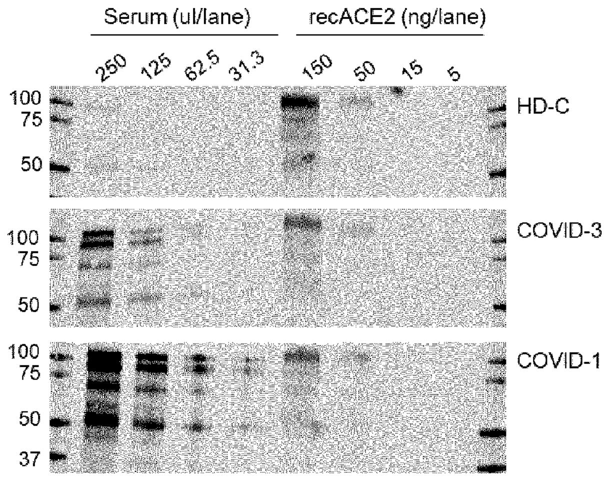


C

| Donor | Commercial ELISA | a-sACE2 Assay (A) | total sACE2 Assay (B) |
|---|---|---|---|
| HD-C | 0.1 ng/ml | 1.3 ng/ml | 43 ng/ml |
| COVID-1 | 72 ng/ml | 37.2ng/ml | 2266 ng/ml |
| COVID-3 | 8.2 ng/ml | 16 ng/ml | 824 ng/ml |

Fig. 5A-C

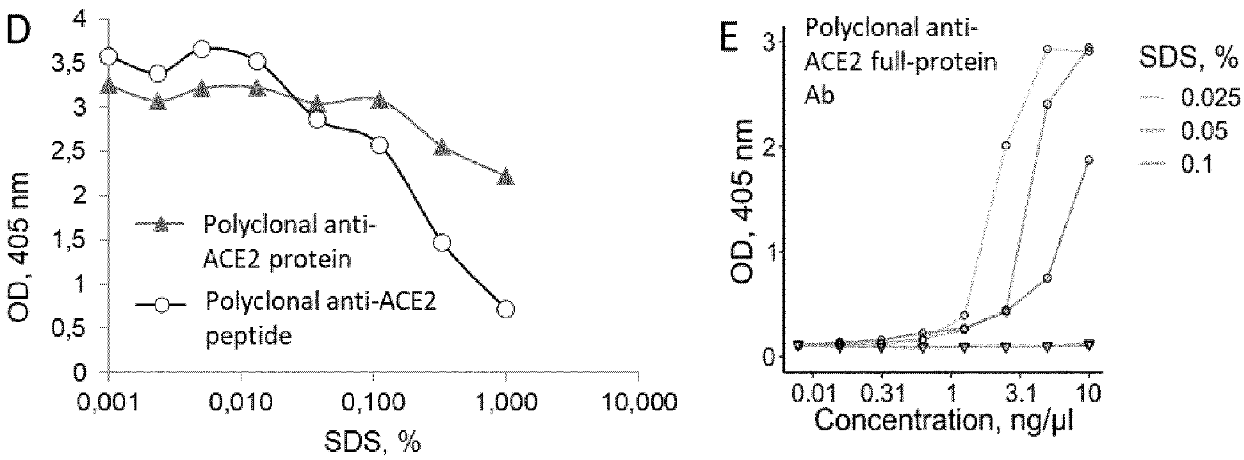
D
OD, 405 nm
4
3,5
3
2,5
2
1,5
1
0,5
0
Polyclonal anti-ACE2 protein
Polyclonal anti-ACE2 peptide
0,001
0,010
0,100
1,000
10,000
SDS, %
E
Polyclonal anti-ACE2 full-protein Ab
OD, 405 nm
3
2
1
0
0.01
0.31
1
3.1
10
Concentration, ng/µl
SDS, %
0.025
0.05
0.1

Fig. 5D-E

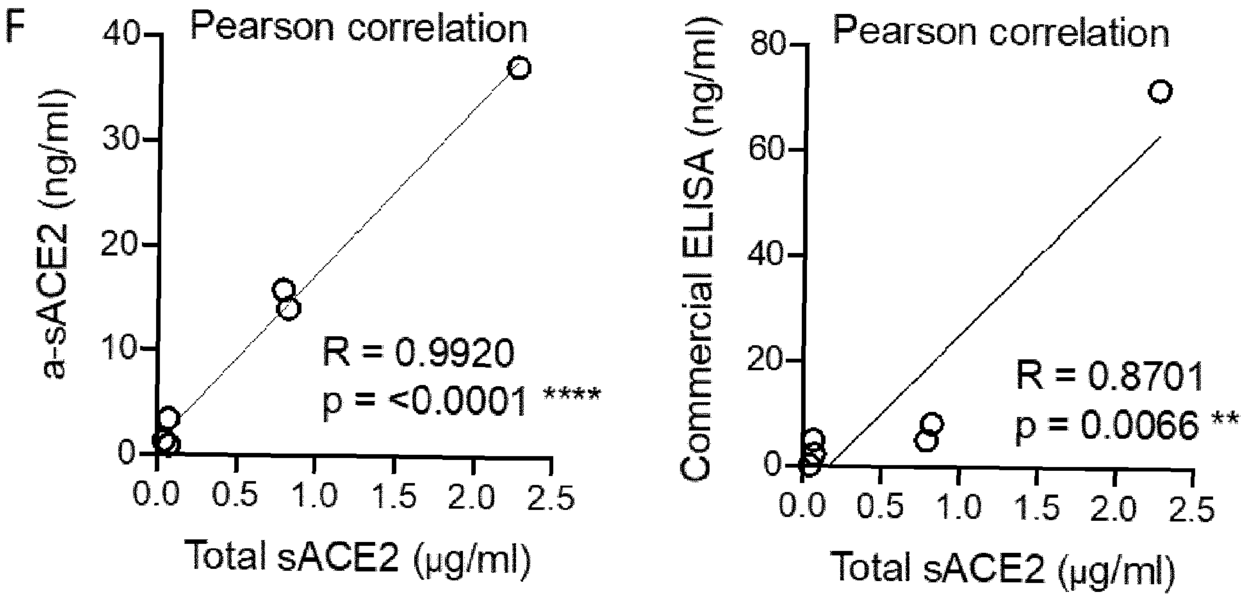
F
Pearson correlation
a-sACE2 (ng/ml)
40
30
20
10
0
0.0 0.5 1.0 1.5 2.0 2.5
Total sACE2 (µg/ml)
R = 0.9920
p = <0.0001 ****
Pearson correlation
Commercial ELISA (ng/ml)
80
60
40
20
0
0.0 0.5 1.0 1.5 2.0 2.5
Total sACE2 (µg/ml)
R = 0.8701
p = 0.0066 **

Fig. 5F

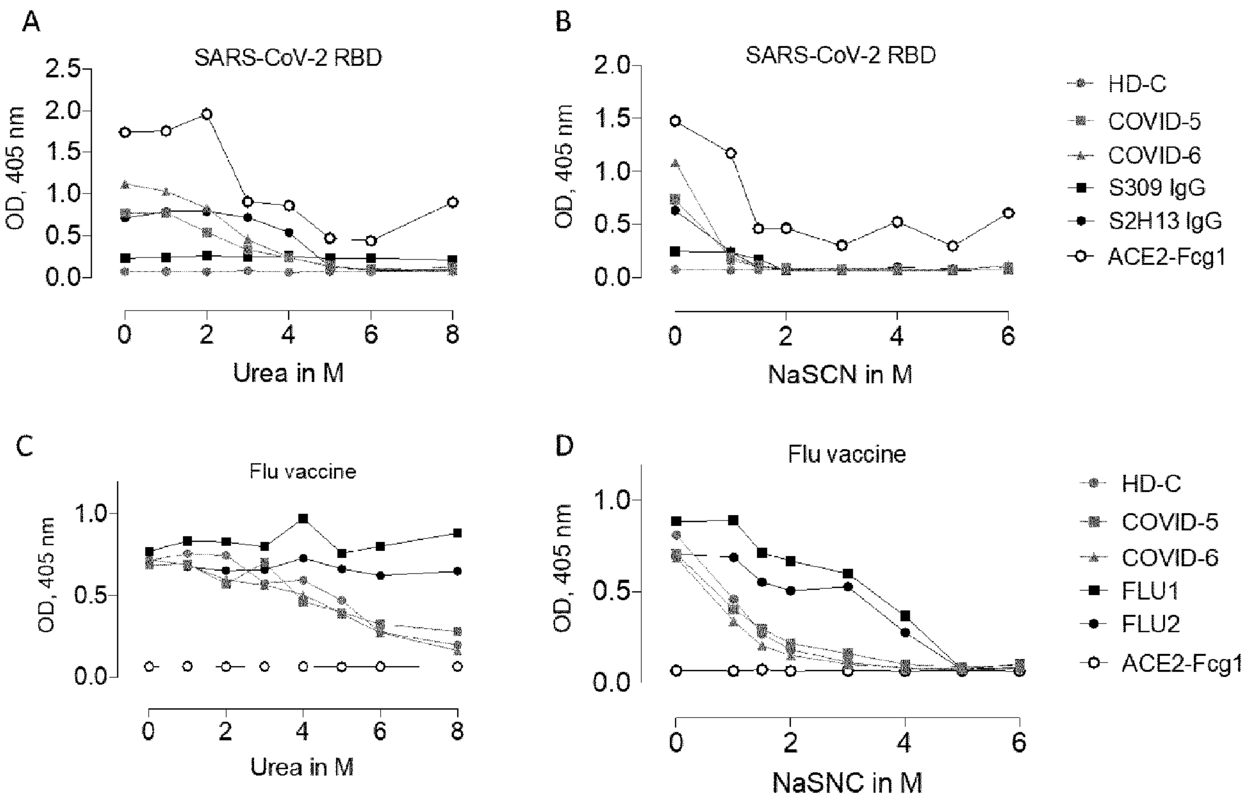
A
SARS-CoV-2 RBD
OD, 405 nm
2.5
2.0
1.5
1.0
0.5
0.0
0
2
4
6
8
Urea in M
B
SARS-CoV-2 RBD
OD, 405 nm
2.0
1.5
1.0
0.5
0.0
0
2
4
6
NaSCN in M
HD-C
COVID-5
COVID-6
S309 IgG
S2H13 IgG
ACE2-Fcg1
C
Flu vaccine
OD, 405 nm
1.0
0.5
0.0
0
2
4
6
8
Urea in M
D
Flu vaccine
OD, 405 nm
1.0
0.5
0.0
0
2
4
6
NaSNC in M
HD-C
COVID-5
COVID-6
FLU1
FLU2
ACE2-Fcg1

Fig. 6A-D

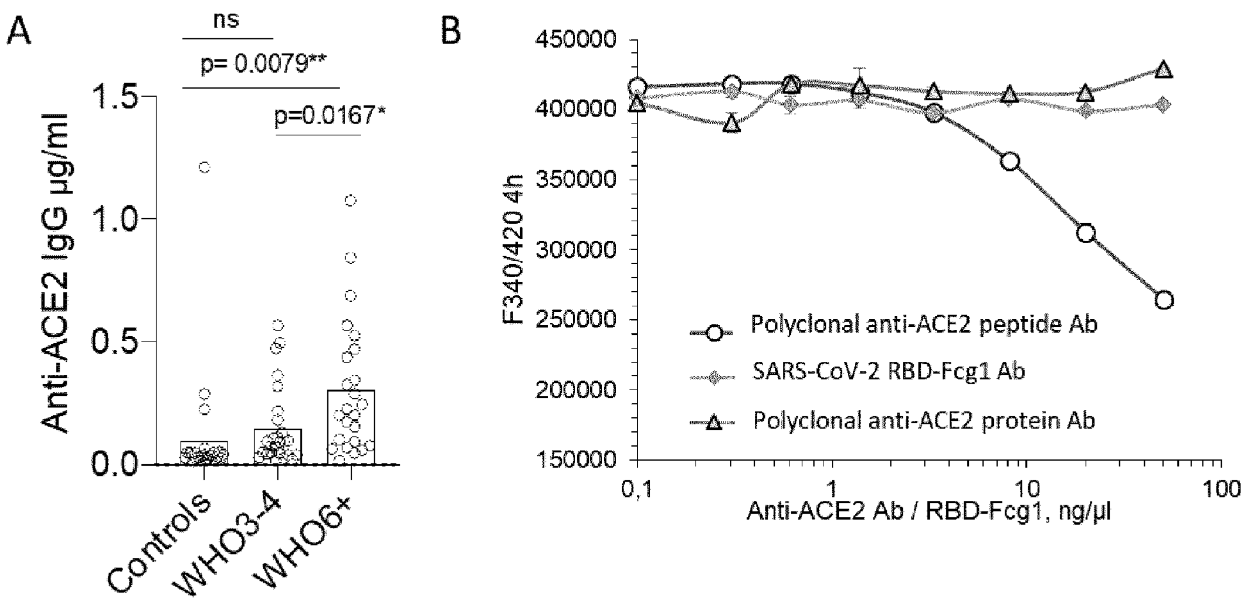
A
ns
p= 0.0079**
p=0.0167*
Anti-ACE2 IgG µg/ml
1.5
1.0
0.5
0.0
Controls
WHO3-4
WHO6+
B
450000
400000
350000
300000
250000
200000
150000
F340/420 4h
Polyclonal anti-ACE2 peptide Ab
SARS-CoV-2 RBD-Fcg1 Ab
Polyclonal anti-ACE2 protein Ab
0,1
1
10
100
Anti-ACE2 Ab / RBD-Fcg1, ng/µl

Fig. 7A-B

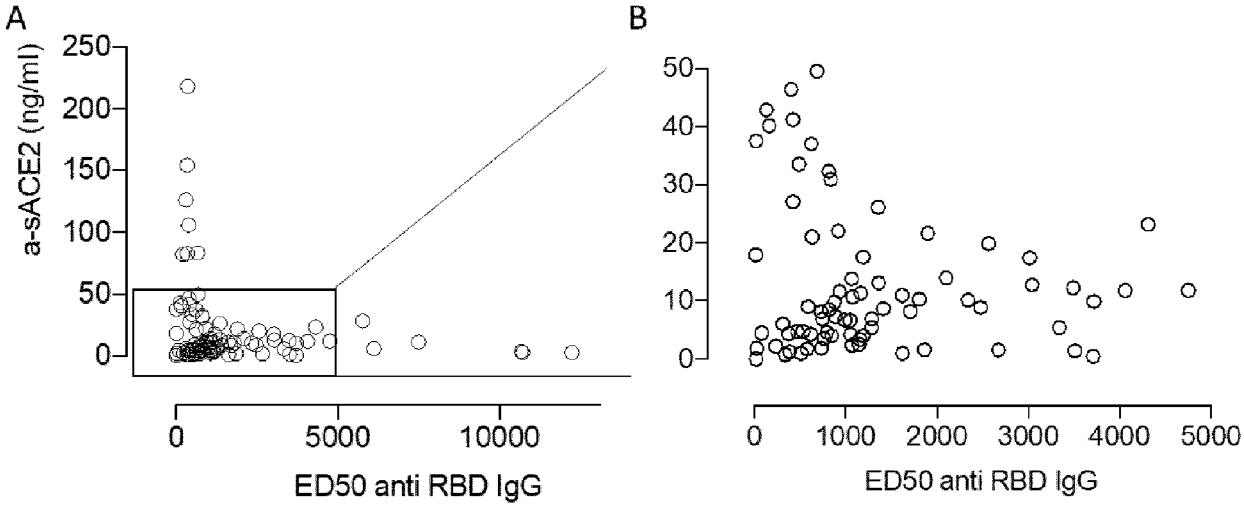
A
B
a-sACE2 (ng/ml)
250
200
150
100
50
0
0
5000
10000
ED50 anti RBD IgG
50
40
30
20
10
0
0
1000
2000
3000
4000
5000
ED50 anti RBD IgG

Fig. 8A-B

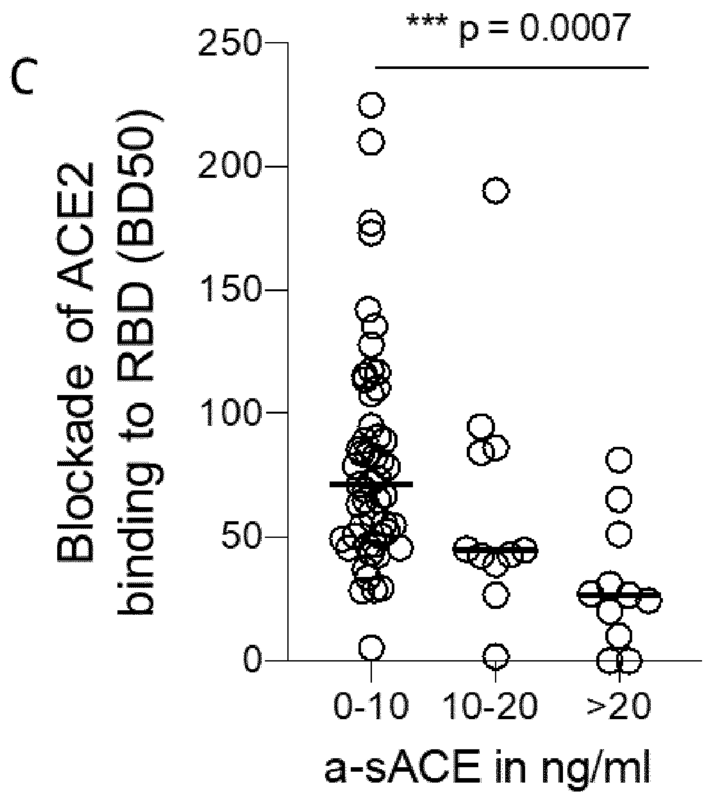
C
*** p = 0.0007
250
200
150
100
50
0
Blockade of ACE2 binding to RBD (BD50)
0-10
10-20
>20
a-sACE in ng/ml

Fig. 8C

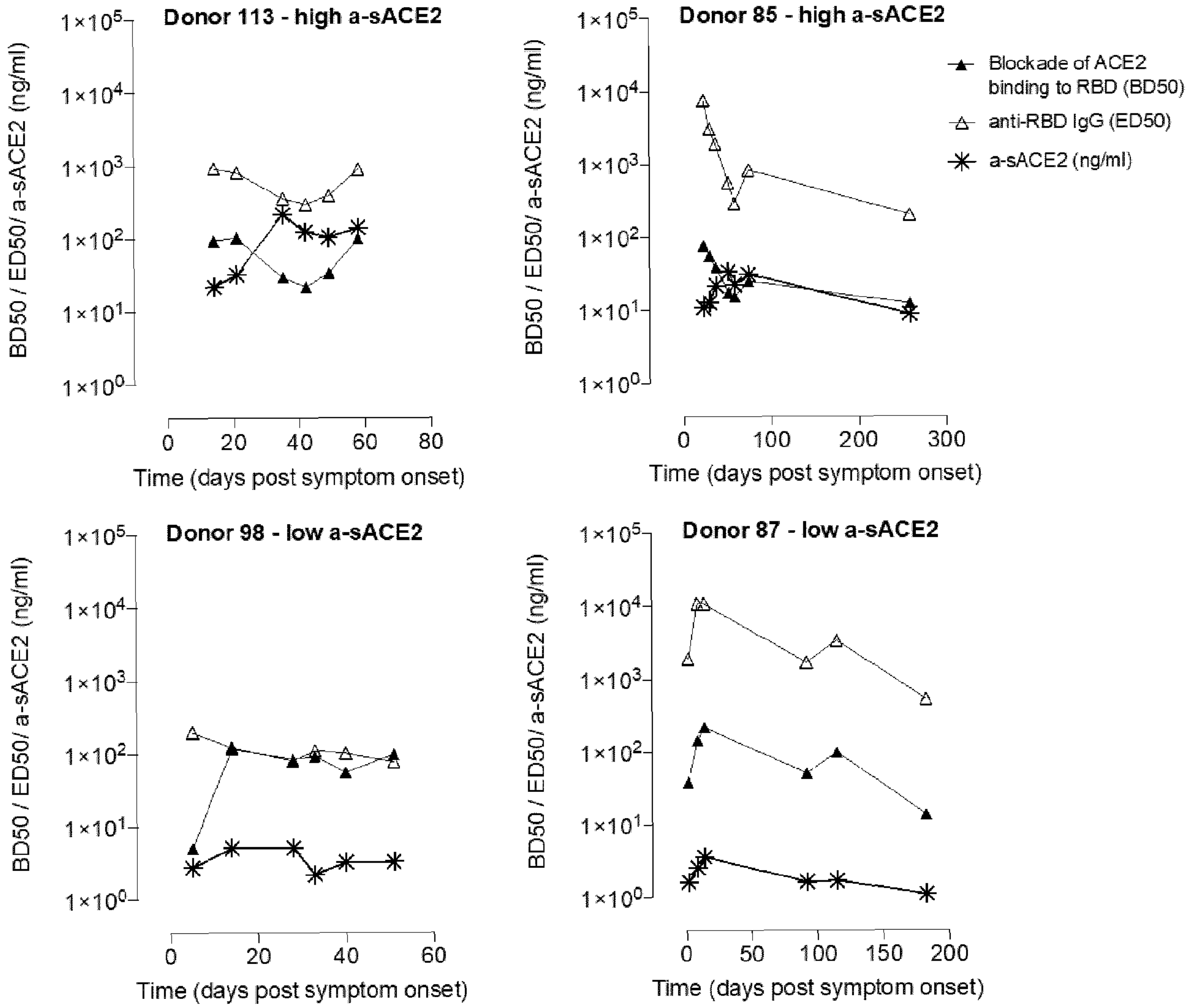
Donor 113 - high a-sACE2
Donor 85 - high a-sACE2
Donor 98 - low a-sACE2
Donor 87 - low a-sACE2
BD50 / ED50/ a-sACE2 (ng/ml)
Time (days post symptom onset)
Blockade of ACE2 binding to RBD (BD50)
anti-RBD IgG (ED50)
a-sACE2 (ng/ml)

Fig. 9A-D

Europäisches Patentamt
European Patent Office
Office européen des brevets

# EUROPEAN SEARCH REPORT

Application Number
EP 21 15 5584

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | RICE GILLIAN I. ET AL: "Circulating Activities of Angiotensin-Converting Enzyme, Its Homolog, Angiotensin-Converting Enzyme 2, and Neprilysin in a Family Study", HYPERTENSION, vol. 48, no. 5, 1 November 2006 (2006-11-01), pages 914-920, XP055820382, US ISSN: 0194-911X, DOI: 10.1161/01.HYP.0000244543.91937.79 * p. 915, par.: "Assays" * & Rice Gillian I ET AL: "Supplementary Information_Circulating Activities of Angiotensin_Converting Enzyme (ACE), its Homolog ACE2 and Neprilysin in a Family Study", , 1 November 2006 (2006-11-01), XP055820424, Retrieved from the Internet: URL:https://www.ahajournals.org/doi/suppl/10.1161/01.HYP.0000244543.91937.79 [retrieved on 2021-07-01] * par.: "Assays" * ----- -/-- | 1-4,13, 15 | INV. C12Q1/37 G01N33/566 G01N33/68 |

| TECHNICAL FIELDS SEARCHED (IPC) |
|---|
| C12Q G01N |

3

The present search report has been drawn up for all claims

| Place of search | Date of completion of the search | Examiner |
|---|---|---|
| Munich | 21 September 2021 | Schalich, Juliane |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 5

Europäisches Patentamt
European Patent Office
Office européen des brevets

# EUROPEAN SEARCH REPORT

Application Number
EP 21 15 5584

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Xiao Fengxia ET AL: "Measurement of Angiotensin Converting Enzyme 2 Activity in Biological Fluid (ACE2) : Methods and Protocols"<br>In: "Hypertension : Methods and Protocols",<br>1 January 2017 (2017-01-01), Humana Press, XP055820414,<br>ISSN: 1064-3745<br>vol. 1527, pages 101-115, DOI: 10.1007/978-1-4939-6625-7_8,<br>Retrieved from the Internet:<br>URL:https://www.ncbi.nlm.nih.gov/pmc/articles/PMC7121061/pdf/978-1-4939-6625-7_Chapter_8.pdf><br>* p. 102-103, par.: 2 Materials;<br>p. 102-107, par. 3 Methods;<br>p. 110-111 *<br>----- | 1-4,13, 15 | |
| X | ÚRI KATALIN ET AL: "New Perspectives in the Renin-Angiotensin-Aldosterone System (RAAS) IV: Circulating ACE2 as a Biomarker of Systolic Dysfunction in Human Hypertension and Heart Failure",<br>PLOS ONE,<br>vol. 9, no. 4, 1 April 2014 (2014-04-01), pages e87845-] e87845, XP055820690,<br>DOI: 10.1371/journal.pone.0087845<br>Retrieved from the Internet:<br>URL:https://www.ncbi.nlm.nih.gov/pmc/articles/PMC3972189/pdf/pone.0087845.pdf><br>* p. 3, par.: "Measurement of serum ACE2 activity" *<br>-----<br>-/-- | 1-4,13, 15 | TECHNICAL FIELDS SEARCHED (IPC) |

The present search report has been drawn up for all claims

| Place of search | Date of completion of the search | Examiner |
|---|---|---|
| Munich | 21 September 2021 | Schalich, Juliane |

3

EPO FORM 1503 03.82 (P04C01)

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

page 2 of 5

Europäisches Patentamt
European Patent Office
Office européen des brevets

# EUROPEAN SEARCH REPORT

Application Number
EP 21 15 5584

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | KATALIN URI ET AL: "Circulating ACE2 activity correlates with cardiovascular disease development", JOURNAL OF THE RENIN-ANGIOTENSIN-ALDOSTERONE SYSTEM., 12 December 2016 (2016-12-12), pages 1-11, XP055820680, Retrieved from the Internet: URL:https://journals.sagepub.com/doi/pdf/10.1177/1470320316668435> * p. 3, par.: Measurement of serum ACE2 activity * ----- | 1-4,13, 15 | |
| X | NAGY BÉLA ET AL: "A dramatic rise in serum ACE2 activity in a critically ill COVID-19 patient", INTERNATIONAL JOURNAL OF INFECTIOUS DISEASES, INTERNATIONAL SOCIETY FOR INFECTIOUS DISEASES, HAMILTON, CA, vol. 103, 26 November 2020 (2020-11-26), pages 412-414, XP086488977, ISSN: 1201-9712, DOI: 10.1016/J.IJID.2020.11.184 [retrieved on 2020-11-26] * p. 413, co. 2; abstract; p. 414 * ----- | 1-4,8, 13,15,16 | TECHNICAL FIELDS SEARCHED (IPC) |
| X | Anonymous: "Mca-APK(Dnp) - BML-P163 - Enzo Life Sciences", , 25 January 2017 (2017-01-25), pages 1-2, XP055820671, Retrieved from the Internet: URL:https://www.enzolifesciences.com/BML-P163/mca-apk-dnp/ [retrieved on 2021-07-02] * the whole document * ----- -/-- | 13,15 | |

The present search report has been drawn up for all claims

| Place of search | Date of completion of the search | Examiner |
|---|---|---|
| Munich | 21 September 2021 | Schalich, Juliane |

3

EPO FORM 1503 03.82 (P04C01)

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

page 3 of 5

Europäisches Patentamt
European Patent Office
Office européen des brevets

# EUROPEAN SEARCH REPORT

Application Number
EP 21 15 5584

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | LI WENHUI ET AL: "Angiotensin-converting enzyme 2 is a functional receptor for the SARS coronavirus", NATURE, MACMILLAN JOURNALS LTD., ETC, LONDON, vol. 426, no. 6965, 1 November 2003 (2003-11-01), pages 450-454, XP037065772, ISSN: 0028-0836, DOI: 10.1038/NATURE02145 * p. 453, co. 2, par.: Immunoprecipitation and identification of ACE2 * ----- | 5-7,14, 15 | |
| X | CN 112 017 782 A (BEIJING SONGGUOTIANMU HEALTH MAN CO LTD) 1 December 2020 (2020-12-01) * abstract; Fig. 1; Description * ----- | 8 | |
| X | Lier D.P.T. Van ET AL: "Increased blood angiotensin converting enzyme 2 activity in critically ill COVID-19 patients", ERJ open research, 28 January 2021 (2021-01-28), page 848, XP055842962, England DOI: 10.1183/23120541.00848-2020 Retrieved from the Internet: URL:https://openres.ersjournals.com/content/erjor/early/2021/01/21/23120541.00848-2020.full.pdf [retrieved on 2021-09-21] * par.: "Methods"; fig. 1; par.: "Discussion" * ----- | 8-12,16 | TECHNICAL FIELDS SEARCHED (IPC) |

-/--

The present search report has been drawn up for all claims

3

| Place of search | Date of completion of the search | Examiner |
|---|---|---|
| Munich | 21 September 2021 | Schalich, Juliane |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 4 of 5

Europäisches Patentamt
European Patent Office
Office européen des brevets

# EUROPEAN SEARCH REPORT

Application Number
EP 21 15 5584

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| A | BENTON DONALD J ET AL: "Receptor binding and priming of the spike protein of SARS-CoV-2 for membrane fusion", NATURE, vol. 588, no. 7837, 20 December 2020 (2020-12-20), pages 327-330, XP037313512, ISSN: 0028-0836, DOI: 10.1038/S41586-020-2772-0 * the whole document * ----- | 1-16 | |
| | | | TECHNICAL FIELDS SEARCHED (IPC) |

3

The present search report has been drawn up for all claims

| Place of search | Date of completion of the search | Examiner |
|---|---|---|
| Munich | 21 September 2021 | Schalich, Juliane |

EPO FORM 1503 03.82 (P04C01)

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

Europäisches Patentamt
European Patent Office
Office européen des brevets

Application Number
EP 21 15 5584

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☒ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

☐ The present supplementary European search report has been drawn up for those parts of the European patent application which relate to the invention first mentioned in the claims (Rule 164 (1) EPC).

Europäisches Patentamt
European Patent Office
Office européen des brevets

# LACK OF UNITY OF INVENTION
# SHEET B

Application Number
EP 21 15 5584

The Search Division considers that the present European patent application does not comply with the requirements of unity of invention and relates to several inventions or groups of inventions, namely:

1. claims: 1-4, 13(completely); 15(partially)

   Assays for indirect quantification of the amounts of the enzyme sACE2 via its enzymatic activity.

   ---

2. claims: 5-7, 14(completely); 15(partially)

   Assays for direct quantification of sACE2 protein.

   ---

3. claims: 8-12, 16

   Methods for identifying subjects with a higher risk of a severe course of COVID-19

   ---

## ANNEX TO THE EUROPEAN SEARCH REPORT ON EUROPEAN PATENT APPLICATION NO. EP 21 15 5584

This annex lists the patent family members relating to the patent documents cited in the above-mentioned European search report.
The members are as contained in the European Patent Office EDP file on
The European Patent Office is in no way liable for these particulars which are merely given for the purpose of information.

21-09-2021

| Patent document cited in search report | Publication date | Patent family member(s) | Publication date |
|---|---|---|---|
| CN 112017782 A | 01-12-2020 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **ELASED, K.M. ; CUNHA, T.S. ; GURLEY, S.B. ; COFFMAN, T.M. ; MORRIS, M.** New mass spectrometric assay for angiotensin-converting enzyme 2 activity. *Hypertension,* 2006, vol. 47, 1010-1017
- **PICCOLI, L. ; PARK, Y.-J. ; TORTORICI, M.A. ; CZUDNOCHOWSKI, N. ; WALLS, A.C. ; BELTRAMELLO, M. ; SILACCI-FREGNI, C. ; PINTO, D. ; ROSEN, L.E. ; BOWEN, J.E.** Mapping Neutralizing and Immunodominant Sites on the SARS-CoV-2 Spike Receptor-Binding Domain by Structure-Guided High-Resolution Serology. *Cell,* 2020, vol. 183, 1024-1042
- **PINTO, D. ; PARK, Y.-J. ; BELTRAMELLO, M. ; WALLS, A.C. ; TORTORICI, M.A. ; BIANCHI, S. ; JACONI, S. ; CULAP, K. ; ZATTA, F. ; DE MARCO, A.** Cross-neutralization of SARS-CoV-2 by a human monoclonal SARS-CoV antibody. *Nature,* 2020, vol. 583, 290-295